# EUROPEAN PATENT SPECIFICATION

(11) **EP 3 587 596 B1**
(45) Date of publication and mention of the grant of the patent: **27.09.2023**
(21) Application number: 18757726.7
(22) Date of filing: 23.02.2018
(51) Int. Cl.: C12Q 1/6883, C12Q 1/689

(54) **METHOD FOR DIAGNOSING CHRONIC OBSTRUCTIVE AIRWAY DISEASE THROUGH BACTERIAL METAGENOME ANALYSIS**
VERFAHREN ZUR DIAGNOSE VON CHRONISCHER OBSTRUKTIVER ATEMWEGSERKRANKUNG DURCH BAKTERIELLE METAGENOMANALYSE
PROCÉDÉ DE DIAGNOSTIC D'UNE MALADIE RESPIRATOIRE OBSTRUCTIVE CHRONIQUE PAR ANALYSE DU MÉTAGÉNOME BACTÉRIEN

(30) Priority: 24.02.2017 KR 20170025084; 12.05.2017 KR 20170059081
(43) Date of publication of application: 01.01.2020
(73) Proprietor: MD Healthcare Inc., Seoul 03923 (KR)
(72) Inventor: KIM, Yoon-Keun, Namyangju-si Gyeonggi-do 12146 (KR)
(74) Representative: Dehns
(86) International application number: PCT/KR2018/002290
(87) International publication number: WO 2018/155967

(56) References cited:
- EP-A1- 2 875 348
- WO-A1-2009/100029
- WO-A1-2011/127219
- WO-A1-2016/133324
- WO-A1-2017/009693
- WO-A1-2018/008895
- WO-A1-2018/030732
- KR-A- 20110 025 603
- KR-A- 20160 035 230
- KR-A- 20160 073 157
- TSUKASA KADOTA ET AL: "Extracellular Vesicles in Chronic Obstructive Pulmonary Disease", INTERNATIONAL JOURNAL OF MOLECULAR SCIENCES, vol. 17, no. 11, 27 October 2016 (2016-10-27), page 1801, XP055556096, DOI: 10.3390/ijms17111801
- HYUN JUNG KIM ET AL: "The microbiome of the lung and its extracellular vesicles in nonsmokers, healthy smokers and COPD patients", EXPERIMENTAL & MOLECULAR MEDICINE, vol. 49, no. 4, 14 April 2017 (2017-04-14) , pages e316-e316, XP055732390, DOI: 10.1038/emm.2017.7
- PRAGMAN, A. A. et al.: "The Lung Microbiome in Moderate and Severe Chronic Obstructive Pulmonary Disease", PLoS One, vol. 7, no. 10, 11 October 2012 (2012-10-11), page e47305, XP055538254,

## Description

### [Technical Field]

The present invention relates to a method of diagnosing chronic obstructive pulmonary disease or asthma through bacterial metagenomic analysis, and more particularly, by analyzing an increase or decrease in content of extracellular vesicles derived from specific bacteria through bacterial metagenomic analysis using a subject-derived sample.

### [Background Art]

Chronic obstructive airway diseases are lung diseases characterized by respiratory difficulty due to chronic airway obstruction and may be broadly classified into asthma, which is characterized by reversible airway obstruction, and chronic obstructive pulmonary disease (COPD), which is characterized by irreversible airway obstruction. According to data of the National Statistical Office in 2012, the number of annual deaths due to respiratory diseases has been steadily increasing since 2006, and among these, death due to COPD is known to be the only cause of death which is on the rise, among the 10 leading causes of death, and the 2013 WHO report has shown that COPD-induced death is the fourth leading cause of death worldwide.

COPD is a disease in which chronic inflammation of the lungs causes the occurrence of chronic bronchitis, chronic bronchiolitis, and emphyema, resulting in irreversible airway obstruction. With regard to the causative factors of COPD, it is known that smoking or chemicals such as air pollutants and biological factors derived from viruses, bacteria, or the like are important. COPD is a disease that is pathologically characterized by neutrophilic inflammation, and this is immunologically characterized by Thl7 cell-induced hypersensitivity. Meanwhile, asthma is a disease characterized by airway hypersensitivity to non-specific stimuli and chronic inflammatory responses, and is characterized by Th2 cell-induced hypersensitivity due to allergens such as protein antigens derived from house dust mites and the like and eosinophilic inflammation occurring due to this.

It is known that the number of microorganisms symbiotically living in the human body is 100 trillion which is 10 times the number of human cells, and the number of genes of microorganisms exceeds 100 times the number of human genes. A microbiota or microbiome is a microbial community that includes bacteria, archaea, and eukaryotes present in a given habitat. The intestinal microbiota is known to play a vital role in human's physiological phenomena and significantly affect human health and diseases through interactions with human cells. Bacteria coexisting in human bodies secrete nanometer-sized vesicles to exchange information about genes, proteins, and the like with other cells. The mucous membranes form a physical barrier membrane that does not allow particles with the size of 200 nm or more to pass therethrough, and thus bacteria symbiotically living in the mucous membranes are unable to pass therethrough, but bacteria-derived extracellular vesicles have a size of approximately 100 nm or less and thus relatively freely pass through the mucous membranes and are absorbed into the human body.

Metagenomics, also called environmental genomics, may be analytics for metagenomic data obtained from samples collected from the environment (Korean Patent Publication No. 2011-0073049). Recently, the bacterial composition of human microbiota has been listed using a method based on 16s ribosomal RNA (16s rRNA) base sequences, and 16s rDNA base sequences, which are genes of 16s ribosomal RNA, are analyzed using a next generation sequencing (NGS) platform. However, in the onset of chronic obstructive airway diseases, identification of causative factors of chronic obstructive airway diseases such as asthma, and COPD and the like through metagenomic analysis of bacteria-derived vesicles isolated from a human-derived substance, such as blood and the like, and a method of diagnosing a risk of developing chronic obstructive airway diseases have never been reported.

EP2875348 discloses a method for diagnosis of asthma comprising isolation of microvesicles£ from a patient sample and measuring the increase or decrease of biomarkers compared to a| reference value.

WO2009/100029 discloses a diagnostic method comprising isolation of microvesicles from a patient sample and detecting the presence or absence of biomarkers, wherein the method may beg used to diagnose COPD.

### [Disclosure]

### [Technical Problem]

To diagnose a chronic obstructive airway diseases based on causative factors of COPD and asthma, the inventors of the present invention extracted DNA from bacteria-derived extracellular vesicles in blood, which is a subject-derived sample, and performed metagenomic analysis on the extracted DNA, and, as a result, identified bacteria-derived extracellular vesicles capable of acting as causative factors of COPD and asthma, thus completing the present invention based on these findings.

Therefore, an object of the present invention is to provide a method of providing information for COPD and asthma diagnosis through metagenomic analysis of bacteria-derived extracellular vesicles.

### [Technical Solution]

To achieve the above-described object of the present invention, there is provided a method of diagnosing COPD or asthma according to the appended claims, comprising the following processes:
(a) extracting DNA from extracellular vesicles isolated from a subject sample;
(b) performing polymerase chain reaction (PCR) on the extracted DNA using a pair of primers having SEQ ID NO: 1 and SEQ ID NO: 2; and
(c) comparing an increase or decrease in content of bacteria-derived extracellular vesicles between a normal individual-derived sample and a chronic obstructive pulmonary disease (COPD) patient-derived sample through sequencing of a product of the PCR, or
comparing an increase or decrease in content of bacteria-derived extracellular vesicles between a normal individual-derived sample and an asthma patient-derived sample through sequencing of a product of the PCR.

The subject sample is blood.

In another embodiment of the present invention, in process (c) above, COPD may be diagnosed by comparing an increase or decrease in content of extracellular vesicles derived from bacteria belonging to the phylum *Tenericutes* of the subject sample with that of a normal individual-derived sample.

In another embodiment of the present invention, in process (c) above, COPD may be diagnosed by comparing an increase or decrease in content of extracellular vesicles derived from one or more bacteria selected from the group consisting of the class *Mollicutes* and the class *Solibacteres* of the subject sample with that of a normal individual-derived sample.

In another embodiment of the present invention, in process (c) above, COPD may be diagnosed by comparing an increase or decrease in content of extracellular vesicles derived from one or more bacteria selected from the group consisting of the order *Stramenopiles,* the order *Rubrobacterales,* the order *Turicibacterales,* and the order *Rhodocyclales* of the subject sample with that of a normal individual-derived sample.

In another embodiment of the present invention, in process (c) above, COPD may be diagnosed by comparing an increase or decrease in content of extracellular vesicles derived from one or more bacteria selected from the group consisting of the family *Rubrobacteraceae,* the family *Turicibacteraceae,* and the family *Rhodocyclaceae* of the subject sample with that of a normal individual-derived sample.

In another embodiment of the present invention, in process (c) above, COPD may be diagnosed by comparing an increase or decrease in content of extracellular vesicles derived from one or more bacteria selected from the group consisting of the genus *Proteus,* the genus *Rubrobacter,* the genus *Turicibacter,* the genus *Faecalibacterium,* the genus *Coprococcus,* the genus *Enhydrobacter,* the genus *Citrobacter,* and the genus *Brevundimonas* of the subject sample with that of a normal individual-derived sample.

In another embodiment of the present invention, in process (c) above, asthma may be diagnosed by comparing an increase or decrease in content of extracellular vesicles derived from one or more bacteria selected from the group consisting of the phylum *Chloroflexi,* the phylum *Armat*ž*monadetes,* the phylum *Fusobacteria,* the phylum *Cyanobacteria*, the phylum *Thermi,* and the phylum *Verrucomicrobia* of the subject sample with that of a normal individual-derived sample.

In another embodiment of the present invention, in process (c) above, asthma may be diagnosed by comparing an increase or decrease in content of extracellular vesicles derived from one or more bacteria selected from the group consisting of the class *Fimbriimonadia,* the class *Cytophagia,* the class *Chloroplast,* the class *Fusobacteriia,* the class *Deinococci,* the class *Alphaproteobacteria,* the class *Flavobacteriia,* and the class *Bacilli* of the subject sample with that of a normal individual-derived sample.

In another embodiment of the present invention, in process (c) above, asthma may be diagnosed by comparing an increase or decrease in content of extracellular vesicles derived from one or more bacteria selected from the group consisting of the order *Bacillales,* the order *Rhodocyclales,* the order *Cytophagales,* the order *Actinomycetales,* the order *Streptophyta,* the order *Fusobacteriales,* the order *Aeromonadales,* the order *Neisseriales,* the order *Rhizobiales,* the order *Pseudomonadales,* the order *Deinococcales,* the order *Xanthomonadales,* the order *Sphingomonadales,* the order *Verrucomicrobiales,* the order *Flavobacteriales,* and the order *Caulobacterales* of the subject sample with that of a normal individual-derived sample.

In another embodiment of the present invention, in process (c) above, asthma may be diagnosed by comparing an increase or decrease in content of extracellular vesicles derived from one or more bacteria selected from the group consisting of the family *Nocardiaceae,* the family *Brevibacteriaceae,* the family *Staphylococcaceae,* the family *Planococcaceae,* the family *Tissierellaceae,* the family *Propionibacteriaceae,* the family *Dermacoccaceae,* the family *Rhizobiaceae,* the family *Bacillaceae,* the family *Corynebacteriaceae,* the family the family *Pseudomonadaceae,* the family *Neisseriaceae,* the family *Methylobacteriaceae,* the family *Xanthomonadaceae,* the family *Micrococcaceae,* the family *Moraxellaceae,* the family *Sphingomonadaceae,* the family *Actinomycetaceae,* the family *Lactobacillaceae,* the family *Caulobacteraceae,* the family *Weeksellaceae,* the family *Aerococcaceae,* the family *Porphyromonadaceae,* the family *Veillonellaceae,* the family *Enterobacteriaceae,* the family *Barnesiellaceae,* the family *Rikenellaceae,* the family *Bacteroidaceae,* and the family *Bifidobacteriaceae* of the subject sample with that of a normal individual-derived sample.

In another embodiment of the present invention, in process (c) above, asthma may be diagnosed by comparing an increase or decrease in content of extracellular vesicles derived from one or more bacteria selected from the group consisting of the genus *Rhodococcus,* the genus *Proteus,* the genus *Staphylococcus,* the genus *Micrococcus,* the genus *Acinetobacter,* the genus *Corynebacterium,* the genus *Pseudomonas,* the genus *Sphingomonas,* the genus *Lactobacillus,* the genus *Akkermansia,* the genus *Rothia,* the genus *Faecalibacterium,* the genus *Roseburia,* the genus *Klebsiella,* the genus *Sutterella,* the genus *Paraprevotella,* the genus *Parabacteroides,* the genus *Butyricimonas,* the genus *Lachnobacterium,* the genus *Veillonella,* the genus *Bacteroides,* the genus *Lachnospira,* the genus *Bifidobacterium,* the genus *Bilophila,* and the genus *Enterobacter* of the subject sample with that of a normal individual-derived sample.

The blood may be whole blood, serum, plasma, or blood mononuclear cells.

### [Advantageous Effects]

Extracellular vesicles secreted from bacteria present in the environment are absorbed into the human body, and thus may directly affect inflammatory responses, and it is difficult to diagnose chronic obstructive airway diseases such as asthma, COPD, and the like early before symptoms occur, and thus efficient treatment therefor is difficult. Thus, a risk of developing chronic obstructive airway diseases can be predicted through metagenomic analysis of bacteria-derived extracellular vesicles by using a human body-derived sample, and thus the onset of chronic obstructive airway diseases can be delayed or prevented through appropriate management by early diagnosis and prediction of a risk group for chronic obstructive airway diseases, and, even after chronic obstructive airway diseases occur, early diagnosis therefor can be implemented, thereby lowering the incidence rate of chronic obstructive airway diseases and increasing therapeutic effects. In addition, patients diagnosed with asthma or COPD are able to avoid exposure to causative factors predicted by metagenomic analysis, whereby the progression of diseases can be ameliorated, or recurrence thereof can be prevented.

### [Description of Drawings]

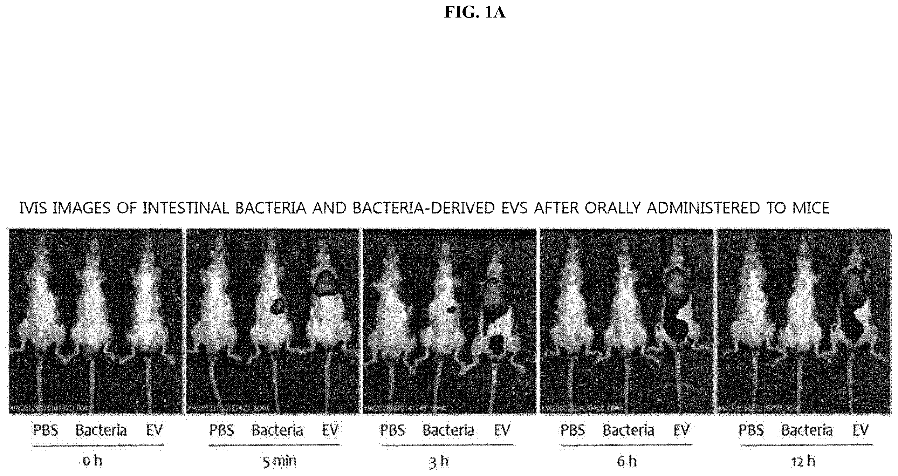

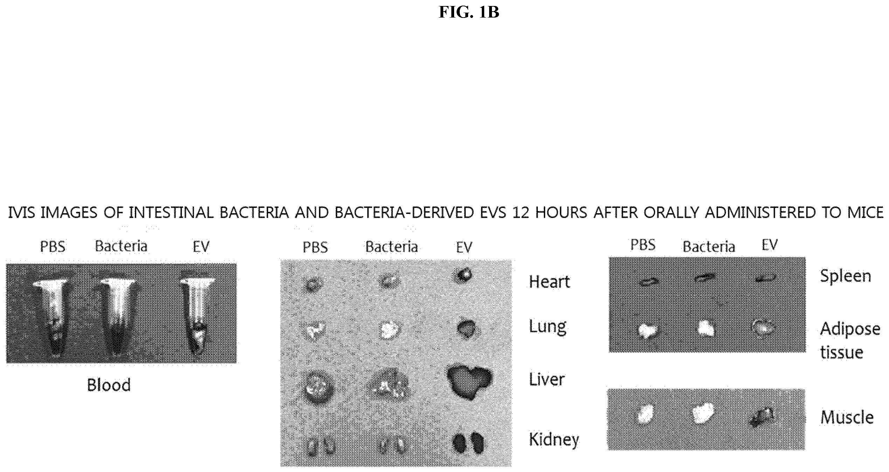

FIGS. 1A and 1B are views for evaluating the distribution pattern of extracellular vesicles (EVs) derived from bacteria *in vivo.* FIG. 1A illustrates images showing the distribution pattern of intestinal bacteria and EVs derived from bacteria per time (0 h, 5 min, 3 h, 6 h, and 12 h) after being orally administered to mice. FIG. 1B illustrates images showing the distribution pattern of gut bacteria and EVs derived from bacteria after being orally administered to mice and, after 12 hours, blood and various organs (heart, lung, liver, kidney, spleen, adipose tissue, and muscle) of the mice were extracted.
FIG. 2 illustrates distribution results of bacteria-derived EVs exhibiting significant diagnostic performance at a phylum level, after metagenomic analysis of bacteria-derived EVs isolated from COPD patient-derived blood and normal individual-derived blood.
FIG. 3 illustrates distribution results of bacteria-derived EVs exhibiting significant diagnostic performance at a class level, after metagenomic analysis of bacteria-derived EVs isolated from COPD patient-derived blood and normal individual-derived blood.
FIG. 4 illustrates distribution results of bacteria-derived EVs exhibiting significant diagnostic performance at an order level, after metagenomic analysis of bacteria-derived EVs isolated from COPD patient-derived blood and normal individual-derived blood.
FIG. 5 illustrates distribution results of bacteria-derived EVs exhibiting significant diagnostic performance at a family level, after metagenomic analysis of bacteria-derived EVs isolated from COPD patient-derived blood and normal individual-derived blood.
FIG. 6 illustrates distribution results of bacteria-derived EVs exhibiting significant diagnostic performance at a genus level, after metagenomic analysis of bacteria-derived EVs isolated from COPD patient-derived blood and normal individual-derived blood.
FIG. 7 illustrates distribution results of bacteria-derived EVs exhibiting significant diagnostic performance at a phylum level, after metagenomic analysis of bacteria-derived EVs isolated from asthma patient-derived blood and normal individual-derived blood.
FIG. 8 illustrates distribution results of bacteria-derived EVs exhibiting significant diagnostic performance at a class level, after metagenomic analysis of bacteria-derived EVs isolated from asthma patient-derived blood and normal individual-derived blood.
FIG. 9 illustrates distribution results of bacteria-derived EVs exhibiting significant diagnostic performance at an order level, after metagenomic analysis of bacteria-derived EVs isolated from asthma patient-derived blood and normal individual-derived blood.
FIG. 10 illustrates distribution results of bacteria-derived EVs exhibiting significant diagnostic performance at a family level, after metagenomic analysis of bacteria-derived EVs isolated from asthma patient-derived blood and normal individual-derived blood.
FIG. 11 illustrates distribution results of bacteria-derived EVs exhibiting significant diagnostic performance at a genus level, after metagenomic analysis of bacteria-derived EVs isolated from asthma patient-derived blood and normal individual-derived blood.
FIG. 12 illustrates distribution results of bacteria-derived EVs exhibiting significant diagnostic performance at a phylum level, after metagenomic analysis of bacteria-derived EVs isolated from COPD patient-derived blood and asthma patient-derived blood.
FIG. 13 illustrates distribution results of bacteria-derived EVs exhibiting significant diagnostic performance at a class level, after metagenomic analysis of bacteria-derived EVs isolated from COPD patient-derived blood and asthma patient-derived blood.
FIG. 14 illustrates distribution results of bacteria-derived EVs exhibiting significant diagnostic performance at an order level, after metagenomic analysis of bacteria-derived EVs isolated from COPD patient-derived blood and asthma patient-derived blood.
FIG. 15 illustrates distribution results of bacteria-derived EVs exhibiting significant diagnostic performance at a family level, after metagenomic analysis of bacteria-derived EVs isolated from COPD patient-derived blood and asthma patient-derived blood.
FIG. 16 illustrates distribution results of bacteria-derived EVs exhibiting significant diagnostic performance at a genus level, after metagenomic analysis of bacteria-derived EVs isolated from COPD patient-derived blood and asthma patient-derived blood.

### [Best Mode]

The present invention is defined by the scope of the appended claims and relates to a method of diagnosing COPD or asthma through bacterial metagenomic analysis. The inventors of the present invention extracted genes from bacteria-derived extracellular vesicles present in subject-derived samples, performed metagenomic analysis thereon, and identified bacteria-derived extracellular vesicles capable of acting as a causative factor of COPD and asthma.

The term "chronic obstructive airway disease" as used herein is a concept comprising asthma and COPD.

The term "COPD" as used herein is a concept comprising chronic bronchitis, chronic bronchiolitis, and emphyema, but the present invention is not limited thereto.

The term "chronic obstructive airway disease diagnosis" as used herein refers to determining whether a patient has a risk for chronic obstructive airway disease, whether the risk for chronic obstructive airway disease is relatively high, or whether chronic obstructive airway disease has already occurred. The method may be used to delay the onset of chronic obstructive airway disease through special and appropriate care for a specific patient, which is a patient having a high risk for chronic obstructive airway disease or prevent the onset of chronic obstructive airway disease. In addition, the method may be clinically used to determine treatment by selecting the most appropriate treatment method through early diagnosis of chronic obstructive airway disease.

The term "metagenome" as used herein refers to the total of genomes including all viruses, bacteria, fungi, and the like in isolated regions such as soil, the intestines of animals, and the like, and is mainly used as a concept of genomes that explains identification of many microorganisms at once using a sequencer to analyze non-cultured microorganisms. In particular, a metagenome does not refer to a genome of one species, but refers to a mixture of genomes, including genomes of all species of an environmental unit. This term originates from the view that, when defining one species in a process in which biology is advanced into omics, various species as well as existing one species functionally interact with each other to form a complete species. Technically, it is the subject of techniques that analyzes all DNAs and RNAs regardless of species using rapid sequencing to identify all species in one environment and verify interactions and metabolism. In the present invention, bacterial metagenomic analysis is performed using bacteria-derived extracellular vesicles isolated from, for example, serum.

In the present invention, the subject sample is blood, and the blood may be preferably whole blood, serum, plasma, or blood mononuclear cells, but the present invention is not limited thereto.

Metagenomic analysis was performed on bacteria-derived extracellular vesicles in normal individual-derived blood, asthma patient-derived blood, and COPD patient-derived blood, and bacteria-derived extracellular vesicles capable of acting as a cause of the onset of COPD and asthma were actually identified by analysis at phylum, class, order, family, and genus levels.

More particularly, as a result of performing metagenomic analysis on bacteria-derived extracellular vesicles at a phylum level, the content of extracellular vesicles derived from bacteria belonging to the phylum *Tenericutes* was significantly different between COPD patients and normal individuals (see Example 4).

More particularly, as a result of performing metagenomic analysis on bacteria-derived extracellular vesicles at a class level, the content of extracellular vesicles derived from bacteria belonging to the class *Mollicutes* and the class *Solibacteres* was significantly different between COPD patients and normal individuals (see Example 4).

More particularly, as a result of performing metagenomic analysis on bacteria-derived extracellular vesicles at an order level, the content of extracellular vesicles derived from bacteria belonging to the order *Stramenopiles,* the order *Rubrobacterales,* the order *Turicibacterales,* the order *Rhodocyclales,* the order RF39, and the order *Solibacterales* was significantly different between COPD patients and normal individuals (see Example 4).

More particularly, as a result of performing metagenomic analysis on bacteria-derived extracellular vesicles at a family level, the content of extracellular vesicles derived from bacteria belonging to the family *Rubrobacteraceae,* the family *Turicibacteraceae,* the family *Rhodocyclaceae,* the family *Nocardiaceae,* the family *Clostridiaceae,* the family S24-7, the family *Staphylococcaceae,* and the family *Gordon*ž*aceae* was significantly different between COPD patients and normal individuals (see Example 4).

More particularly, as a result of performing metagenomic analysis on bacteria-derived extracellular vesicles at a genus level, the content of extracellular vesicles derived from bacteria belonging to the genus *Hydrogenophilus,* the genus *Proteus,* the genus *Geobacillus,* the genus *Chromohalobacter,* the genus *Rubrobacter,* the genus *Megamonas,* the genus *Turicibacter,* the genus *Rhodococcus,* the genus *Phascolarctobacterium,* the genus SMB53, the genus *Desulfovibrio,* the genus *Jeotgalicoccus,* the genus *Cloacibacterium,* the genus *Klebsiella,* the genus *Escherichia,* the genus *Cupriavidus,* the genus *Adlercreutzia,* the genus *Clostridium,* the genus *Faecalibacterium,* the genus *Stenotrophomonas,* the genus *Staphylococcus,* the genus *Gordonia,* the genus *Micrococcus,* the genus *Coprococcus,* the genus *Novosphingobium,* the genus *Enhydrobacter,* the genus *Citrobacter,* and the genus *Brevundimonas* was significantly different between COPD patients and normal individuals (see Example 4).

More particularly, as a result of performing metagenomic analysis on bacteria-derived extracellular vesicles at a phylum level, the content of extracellular vesicles derived from bacteria belonging to the phylum *Chloroflexi,* the phylum *Armatimonadetes,* the phylum *Fusobacteria,* the phylum *Cyanobacteria,* the phylum *Planctomycetes,* the phylum *Thermi,* the phylum *Verrucomicrobia,* the phylum *Acidobacteria,* and the phylum TM7 was significantly different between asthma patients and normal individuals (see Example 5).

More particularly, as a result of performing metagenomic analysis on bacteria-derived extracellular vesicles at a class level, the content of extracellular vesicles derived from bacteria belonging to the class *Rubrobacteria,* the class *Fimbriimonadia,* the class *Cytophagia,* the class *Chloroplast,* the class *Fusobacteriia,* the class *Saprospirae,* the class *Sphingobacteriia,* the class *Deinococci,* the class *Verrucomicrobiae,* the class TM7-3, the class *Alphaproteobacteria,* the class *Flavobacteriia,* the class *Bacilli,* and the class 4C0d-2 was significantly different between asthma patients and normal individuals (see Example 5).

More particularly, as a result of performing metagenomic analysis on bacteria-derived extracellular vesicles at an order level, the content of extracellular vesicles derived from bacteria belonging to the order *Rubrobacterales,* the order *Stramenopiles,* the order *Bacillales,* the order *Rhodocyclales,* the order *Fimbriimonadales,* the order *Cytophagales,* the order *Rickettsiales,* the order *Alteromonadales,* the order *Actinomycetales,* the order *Streptophyta,* the order *Fusobacteriales,* the order CW040, the order *Saprospirales,* the order *Aeromonadales,* the order *Neisseriales,* the order *Rhizobiales,* the order *Pseudomonadales,* the order *Deinococcales,* the order *Xanthomonadales,* the order *Sphingomonadales,* the order *Sphingobacteriales,* the order *Verrucomicrobiales,* the order *Flavobacteriales,* the order *Caulobacterales,* the order *Enterobacteriales,* the order *Bifidobacteriales,* and the order YS2 was significantly different between asthma patients and normal individuals (see Example 5).

More particularly, as a result of performing metagenomic analysis on bacteria-derived extracellular vesicles at a family level, the content of extracellular vesicles derived from bacteria belonging to the family *Rubrobacteraceae,* the family *Exiguobacteraceae,* the family *Nocardiaceae,* the family F16, the family *Pseudonocardiaceae,* the family *Dermabacteraceae,* the family *Brevibacteriaceae,* the family *Microbacteriaceae,* the family *Staphylococcaceae,* the family *Cytophagaceae,* the family *Planococcaceae,* the family *Tissierellaceae,* the family *Rhodocyclaceae,* the family *Propionibacteriaceae,* the family *Fimbriimonadaceae,* the family *Campylobacteraceae,* the family *Dermacoccaceae,* the family *Burkholderiaceae,* the family *Rhizobiaceae,* the family *Bacillaceae,* the family *Corynebacteriaceae,* the family *mitochondria,* the family *Fusobacteriaceae,* the family *Leptotrichiaceae,* the family *Pseudomonadaceae,* the family *Bradyrhizobiaceae,* the family *Aeromonadaceae,* the family *Neisseriaceae,* the family *Methylobacteriaceae,* the family *Carnobacteriaceae,* the family *Xanthomonadaceae,* the family *Geodermatophilaceae,* the family *Mycobacteriaceae,* the family *Gordon*ž*aceae,* the family *Micrococcaceae,* the family *Hyphomicrobiaceae,* the family *Moraxellaceae,* the family *Sphingomonadaceae,* the family *Actinomycetaceae,* the family *Deinococcaceae,* the family *Intrasporangiaceae,* the family *Flavobacteriaceae,* the family *Lactobacillaceae,* the family *Verrucomicrobiaceae,* the family *Nocardioidaceae,* the family *Sphingobacteriaceae,* the family *Rhodospirillaceae,* the family *Caulobacteraceae,* the family *Weeksellaceae,* the family *Dietziaceae,* the family *Aerococcaceae,* the family *Porphyromonadaceae,* the family *Veillonellaceae,* the family *Enterobacteriaceae,* the family *Barnesiellaceae,* the family *Rikenellaceae,* the family *Bacteroidaceae,* and the family *Bifidobacteriaceae* was significantly different between asthma patients and normal individuals (see Example 5).

More particularly, as a result of performing metagenomic analysis on bacteria-derived extracellular vesicles at a genus level, the content of extracellular vesicles derived from bacteria belonging to the genus *Geobacillus,* the genus *Rubrobacter,* the genus *Exiguobacterium,* the genus *Ralstonia,* the genus *Sporosarcina,* the genus *Hydrogenophilus,* the genus *Rhodococcus,* the genus *Proteus,* the genus *Leptotrichia,* the genus *Brevibacterium,* the genus *Brachybacterium,* the genus *Staphylococcus,* the genus *Peptoniphilus,* the genus *Lautropia,* the genus *Finegoldia,* the genus *Anaerococcus,* the genus *Sphingobacterium,* the genus *Propionibacterium,* the genus *Micrococcus,* the genus *Fimbriimonas,* the genus *Dermacoccus,* the genus *Campylobacter,* the genus *Agrobacterium,* the genus *Neisseria,* the genus *Acinetobacter,* the genus *Thermus,* the genus *Corynebacterium,* the genus *Fusobacterium,* the genus *Pseudomonas,* the genus *Jeotgalicoccus,* the genus *Dietzia,* the genus *Rubellimicrobium,* the genus *Flavobacterium,* the genus *Megamonas,* the genus *Porphyromonas,* the genus *Granulicatella,* the genus *Novosphingobium,* the genus *Sphingomonas,* the genus *Mycobacterium,* the genus *Methylobacterium,* the genus *Gordonia,* the genus *Burkholderia,* the genus *Kocuria,* the genus *Lactobacillus,* the genus *Deinococcus,* the genus *Kaistobacter,* the genus *Akkermansia,* the genus *Actinomyces,* the genus *Brevundimonas,* the genus *Virgibacillus,* the genus *Bacillus,* the genus *Eubacterium,* the genus *Rothia,* the genus *Chryseobacterium,* the genus *Faecalibacterium,* the genus *Roseburia,* the genus *Klebsiella,* the genus *Sutterella,* the genus *Paraprevotella,* the genus *Parabacteroides,* the genus *Butyricimonas,* the genus *Lachnobacterium,* the genus *Veillonella,* the genus *Bacteroides,* the genus *Lachnospira,* the genus *Bifidobacterium,* the genus *Bilophila,* and the genus *Enterobacter* was significantly different between asthma patients and normal individuals (see Example 5).

More particularly, as a result of performing metagenomic analysis on bacteria-derived extracellular vesicles at a phylum level, the content of extracellular vesicles derived from bacteria belonging to the phylum *Bacteroidetes,* the phylum *Tenericutes,* the phylum *Thermi,* the phylum TM7, the phylum *Cyanobacteria,* the phylum *Verrucomicrobia,* the phylum *Fusobacteria,* the phylum *Acidobacteria,* the phylum *Planctomycetes,* the phylum *Armatimonadetes,* and the phylum *Chloroflexi* was significantly different between asthma patients and COPD patients (see Example 6).

More particularly, as a result of performing metagenomic analysis on bacteria-derived extracellular vesicles at a class level, the content of extracellular vesicles derived from bacteria belonging to the class *Bacteroidia,* the class 4C0d-2, the class *Mollicutes,* the class *Bacilli,* the class *Deinococci,* the class TM7-3, the class *Flavobacteriia,* the class *Alphaproteobacteria,* the class *Verrucomicrobiae,* the class *Fusobacteriia,* the class *Saprospirae,* the class *Sphingobacteriia,* the class *Chloroplast,* the class *Cytophagia,* the class *Fimbriimonadia,* the class *Thermomicrobia,* and the class *Solibacteres* was significantly different between asthma patients and COPD patients (see Example 6).

More particularly, as a result of performing metagenomic analysis on bacteria-derived extracellular vesicles at an order level, the content of extracellular vesicles derived from bacteria belonging to the order YS2, the order *Bifidobacteriales,* the order *Turicibacterales,* the order *Bacteroidales,* the order RF39, the order *Enterobacteriales,* the order *Rhodobacterales,* the order *Neisseriales,* the order *Gemellales,* the order *Deinococcales,* the order *Flavobacteriales,* the order *Xanthomonadales,* the order *Verrucomicrobiales,* the order *Sphingomonadales,* the order *Caulobacterales,* the order *Fusobacteriales,* the order *Saprospirales,* the order *Pseudomonadales,* the order *Sphingobacteriales,* the order *Rhizobiales,* the order *Actinomycetales,* the order CW040, the order *Streptophyta,* the order *Rickettsiales,* the order *Alteromonadales,* the order *Cytophagales,* the order *Aeromonadales,* the order *Fimbriimonadales,* the order JG30-KF-CM45, the order *Bacillales,* and the order *Solibacterales* was significantly different between asthma patients and COPD patients (see Example 6).

More particularly, as a result of performing metagenomic analysis on bacteria-derived extracellular vesicles at a family level, the content of extracellular vesicles derived from bacteria belonging to the family *Helicobacteraceae,* the family *Bacteroidaceae,* the family *Bifidobacteriaceae,* the family *Turicibacteraceae,* the family *Rikenellaceae,* the family *Odoribacteraceae,* the family *Clostridiaceae,* the family *Barnesiellaceae,* the family *Veillonellaceae,* the family *Porphyromonadaceae,* the family *Enterobacteriaceae,* the family *Christensenellaceae,* the family *Lactobacillaceae,* the family *Rhodobacteraceae,* the family *Nocardiaceae,* the family *Neisseriaceae,* the family *Gemellaceae,* the family *Carnobacteriaceae,* the family *Aerococcaceae,* the family *Weeksellaceae,* the family *Deinococcaceae,* the family *Leptotrichiaceae,* the family *Mycobacteriaceae,* the family *Dietziaceae,* the family *Xanthomonadaceae,* the family *Pseudomonadaceae,* the family *Verrucomicrobiaceae,* the family *Methylobacteriaceae,* the family *Flavobacteriaceae,* the family *Actinomycetaceae,* the family *Burkholderiaceae,* the family *Nocardioidaceae,* the family *Caulobacteraceae,* the family *Sphingomonadaceae,* the family *Corynebacteriaceae,* the family *Tissierellaceae,* the family *Chitinophagaceae,* the family *mitochondria,* the family *Sphingobacteriaceae,* the family *Fusobacteriaceae,* the family *Moraxellaceae,* the family *Micrococcaceae,* the family *Geodermatophilaceae,* the family *Dermacoccaceae,* the family *Intrasporangiaceae,* the family *Dermabacteraceae,* the family *Propionibacteriaceae,* the family *Rhodospirillaceae,* the family *Bradyrhizobiaceae,* the family *Campylobacteraceae,* the family *Brevibacteriaceae,* the family *Microbacteriaceae,* the family *Cellulomonadaceae,* the family *Gordon*ž*aceae,* the family *Bacillaceae,* the family *Planococcaceae,* the family *Rhizobiaceae,* the family *Aeromonadaceae,* the family *Fimbriimonadaceae,* the family *Cytophagaceae,* the family F16, the family *Staphylococcaceae,* the family *Exiguobacteraceae,* and the family *Alteromonadaceae* was significantly different between asthma patients and COPD patients (see Example 6).

More particularly, as a result of performing metagenomic analysis on bacteria-derived extracellular vesicles at a genus level, the content of extracellular vesicles derived from bacteria belonging to the genus *Enterobacter,* the genus *Trabulsiella,* the genus *Phascolarctobacterium,* the genus *Klebsiella,* the genus *Bifidobacterium,* the genus *Bacteroides,* the genus *Turicibacter,* the genus *Sutterella,* the genus *Butyricimonas,* the genus *Parabacteroides,* the genus *Ruminococcus,* the genus *Veillonella,* the genus *Pediococcus,* the genus *Desulfovibrio,* the genus SMB53, the genus *Roseburia,* the genus *Odoribacter,* the genus *Dialister,* the genus *Escherichia,* the genus *Sphingobium,* the genus *Rothia,* the genus *Paracoccus,* the genus *Lactobacillus,* the genus *Rhodococcus,* the genus *Eubacterium,* the genus *Granulicatella,* the genus *Kaistobacter,* the genus *Capnocytophaga,* the genus *Deinococcus,* the genus *Mycobacterium,* the genus *Microbispora,* the genus *Methylobacterium,* the genus *Chryseobacterium,* the genus *Actinomyces,* the genus *Porphyromonas,* the genus *Kocuria,* the genus *Akkermansia,* the genus *Pseudomonas,* the genus *Coprococcus,* the genus *Peptoniphilus,* the genus *Neisseria,* the genus *Corynebacterium,* the genus *Anaerococcus,* the genus *Acinetobacter,* the genus *Rubellimicrobium,* the genus *Sphingobacterium,* the genus *Sphingomonas,* the genus *Pedobacter,* the genus *Finegoldia,* the genus *Fusobacterium,* the genus *Lautropia,* the genus *Moraxella,* the genus *Enhydrobacter,* the genus *Dermacoccus,* the genus *Thermus,* the genus *Citrobacter,* the genus *Bacillus,* the genus *Stenotrophomonas,* the genus *Hymenobacter,* the genus *Brachybacterium,* the genus *Propionibacterium,* the genus *Leptotrichia,* the genus *Dietzia,* the genus *Brevibacterium,* the genus *Flavobacterium,* the genus *Gordonia,* the genus *Agrobacterium,* the genus *Fimbriimonas,* the genus *Novosphingobium,* the genus *Lysinibacillus,* the genus *Brevundimonas,* the genus *Achromobacter,* the genus *Micrococcus,* the genus *Staphylococcus,* the genus *Ralstonia,* the genus *Exiguobacterium,* and the genus *Alkanindiges* was significantly different between asthma patients and COPD patients (see Example 6).

From the above-described example results, it was confirmed that bacteria-derived extracellular vesicles exhibiting significant changes in content in normal individual-derived blood, asthma patient-derived blood, and COPD patient-derived blood were identified by performing metagenomic analysis on bacteria-derived extracellular vesicles isolated from blood, and COPD and asthma not only could be diagnosed, but could also be differentially diagnosed by analyzing an increase or decrease in the content of bacteria-derived extracellular vesicles at each level through metagenomic analysis.

Hereinafter, the present invention will be described with reference to exemplary examples to aid in understanding of the present invention. However, these examples are provided only for illustrative purposes and are not intended to limit the scope of the present invention.

### [Mode of the Invention]

### [Examples]

### Example 1. Analysis of In Vivo Absorption, Distribution, and Excretion Patterns of Intestinal Bacteria and Bacteria-Derived Extracellular Vesicles

To evaluate whether intestinal bacteria and bacteria-derived extracellular vesicles are systematically absorbed through the gastrointestinal tract, an experiment was conducted using the following method. More particularly, 50 µg of each of intestinal bacteria and the bacteria-derived extracellular vesicles (EVs), labeled with fluorescence, were orally administered to the gastrointestinal tracts of mice, and fluorescence was measured at 0 h, and after 5 min, 3 h, 6 h, and 12 h. As a result of observing the entire images of mice, as illustrated in FIG. 1A, the bacteria were not systematically absorbed when administered, while the bacteria-derived EVs were systematically absorbed at 5 min after administration, and, at 3 h after administration, fluorescence was strongly observed in the bladder, from which it was confirmed that the EVs were excreted via the urinary system, and were present in the bodies up to 12 h after administration.

After intestinal bacteria and intestinal bacteria-derived extracellular vesicles were systematically absorbed, to evaluate a pattern of invasion of intestinal bacteria and the bacteria-derived EVs into various organs in the human body after being systematically absorbed, 50 µg of each of the bacteria and bacteria-derived EVs, labeled with fluorescence, were administered using the same method as that used above, and then, at 12 h after administration, blood, the heart, the lungs, the liver, the kidneys, the spleen, adipose tissue, and muscle were extracted from each mouse. As a result of observing fluorescence in the extracted tissues, as illustrated in FIG. 1B, it was confirmed that the intestinal bacteria were not absorbed into each organ, while the bacteria-derived EVs were distributed in the blood, heart, lungs, liver, kidneys, spleen, adipose tissue, and muscle.

### Example 2. Vesicle Isolation and DNA Extraction from Blood

To isolate extracellular vesicles and extract DNA, from blood, first, stool and urine was added to a 10 ml tube and centrifuged at 3,500 x g and 4 □ for 10 min to precipitate a suspension, and only a supernatant was collected, which was then placed in a new 10 ml tube. The collected supernatant was filtered using a 0.22 µm filter to remove bacteria and impurities, and then placed in centripreigugal filters (50 kD) and centrifuged at 1500 x g and 4 □ for 15 min to discard materials with a smaller size than 50 kD, and then concentrated to 10 ml. Once again, bacteria and impurities were removed therefrom using a 0.22 µm filter, and then the resulting concentrate was subjected to ultra-high speed centrifugation at 150,000 x g and 4 □ for 3 hours by using a Type 90ti rotor to remove a supernatant, and the agglomerated pellet was dissolved with phosphate-buffered saline (PBS), thereby obtaining vesicles.

100 µl of the extracellular vesicles isolated from the stool and urine according to the above-described method was boiled at 100 □ to allow the internal DNA to come out of the lipid and then cooled on ice. Next, the resulting vesicles were centrifuged at 10,000 x g and 4 □ for 30 minutes to remove the remaining suspension, only the supernatant was collected, and then the amount of DNA extracted was quantified using a NanoDrop sprectrophotometer. In addition, to verify whether bacteria-derived DNA was present in the extracted DNA, PCR was performed using 16s rDNA primers shown in Table 1 below.

**[Table 1]**

| Primer | | Sequence | SEQ ID NO. |
|---|---|---|---|
| 16S rDNA | 16S V3 F | 5'-TCGTCGGCAGCGTC AGATGTGTATAAGAG ACAGCCTACGGGNGG CWGCAG-3' | 1 |
| | 16S_V4_R | 5'-GTCTCGTGGGCTCG GAGATGTGTATAAGA GACAGGACTACHVGG GTATCTAATCC-3' | 2 |

### Example 3. Metagenomic Analysis Using DNA Extracted from Vesicle in Blood

DNA was extracted using the same method as that used in Example 2, and then PCR was performed thereon using 16S rDNA primers shown in Table 1 to amplify DNA, followed by sequencing (Illumina MiSeq sequencer). The results were output as standard flowgram format (SFF) files, and the SFF files were converted into sequence files (.fasta) and nucleotide quality score files using GS FLX software (v2.9), and then credit rating for reads was identified, and portions with a window (20 bps) average base call accuracy of less than 99% (Phred score <20) were removed. After removing the low-quality portions, only reads having a length of 300 bps or more were used (Sickle version 1.33), and, for operational taxonomy unit (OTU) analysis, clustering was performed using UCLUST and USEARCH according to sequence similarity. In particular, clustering was performed based on sequence similarity values of 94% for genus, 90% for family, 85% for order, 80% for class, and 75% for phylum, and phylum, class, order, family, and genus levels of each OTU were classified, and bacteria with a sequence similarity of 97% or more were analyzed (QIIME) using 16S DNA sequence databases (108,453 sequences) of BLASTN and GreenGenes.

### Example 4. COPD Diagnostic Model Based on Metagenomic Analysis of Bacteria-Derived EVs Isolated from Blood of Normal Individuals and COPD Patients

EVs were isolated from blood samples of 205 COPD patients and 231 normal individuals, the two groups matched in age and gender, and then metagenomic sequencing was performed thereon using the method of Example 3. For the development of a diagnostic model, first, a strain exhibiting a p value of less than 0.05 between two groups in a t-test and a difference of two-fold or more between two groups was selected, and then an area under curve (AUC), sensitivity, and specificity, which are diagnostic performance indexes, were calculated by logistic regression analysis.

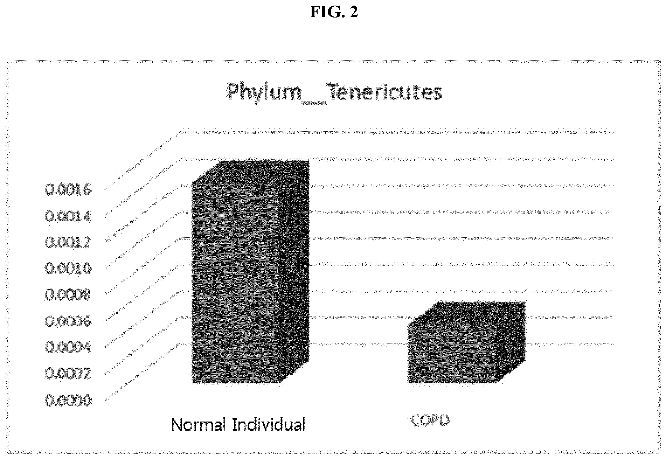

As a result of analyzing bacteria-derived extracellular vesicles in blood at a phylum level, a diagnostic model developed using bacteria belonging to the phylum *Tenericutes* exhibited significant diagnostic performance for COPD (see Table 2 and FIG. 2).

**[Table 2]**

| | normal individual | | COPD | | t-test | | Training Set | | | Test Set | | |
|---|---|---|---|---|---|---|---|---|---|---|---|---|
| Taxon | Mean | SD | Mean | SD | p-value | Ratio | AUC | sensit ivity | specif icity | AUC | sensit ivity | specif icity |
| p Teneri cutes | 0.001 5 | 0.004 5 | 0.000 4 | 0.001 2 | 0.000 0 | 0.30 | 0.80 | 0.88 | 0.45 | 0.76 | 0.82 | 0.47 |

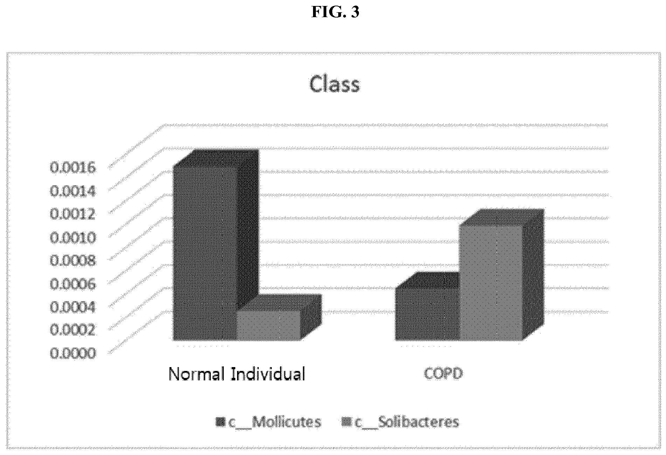

As a result of analyzing bacteria-derived extracellular vesicles in blood at a class level, a diagnostic model developed using, as a biomarker, one or more bacteria from the class *Mollicutes,* and the class *Solibacteres* exhibited significant diagnostic performance for COPD (see Table 3 and FIG. 3).

**[Table 3]**

| | normal individual | | COPD | | t-test | | Training Set | | | Test Set | | |
|---|---|---|---|---|---|---|---|---|---|---|---|---|
| Taxon | Mean | SD | Mean | SD | p-value | Rati o | AUC | sensit ivity | specif icity | AUC | sensit ivity | specif icity |
| c _ Mollic utes | 0.001 5 | 0.004 5 | 0.000 4 | 0.001 2 | 0.000 0 | 0.30 | 0.80 | 0.89 | 0.41 | 0.76 | 0.83 | 0.44 |
| c Soliba | 0.000 | 0.001 | 0.001 | 0.002 | 0.000 | 3.87 | 0.78 | 0.90 | 0.19 | 0.77 | 0.87 | 0.33 |
| cteres | 3 | 8 | 0 | 8 | 8 | | | | | | | |

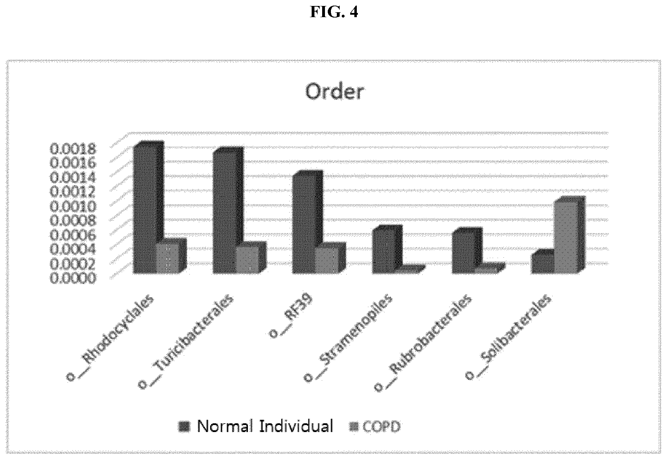

As a result of analyzing bacteria-derived extracellular vesicles in blood at an order level, a diagnostic model developed using, as a biomarker, one or more bacteria from the order *Stramenopiles,* the order *Rubrobacterales,* the order *Turicibacterales,* the order *Rhodocyclales,* the order RF39, and the order *Solibacterales* exhibited significant diagnostic performance for COPD (see Table 4 and FIG. 4).

**[Table 4]**

| | normal individual | | COPD | | t-test | | Training Set | | | Test Set | | |
|---|---|---|---|---|---|---|---|---|---|---|---|---|
| Taxon | Mean | SD | Mean | SD | p-value | Ratio | AUC | sensit ivity | specif icity | AUC | sensit ivity | speci ficity |
| o_Stra menopi les | 0.0006 | 0.003 4 | 0.0000 | 0.000 6 | 0.0010 | 0.07 | 0.79 | 0.88 | 0.33 | 0.79 | 0.87 | 0.38 |
| o_Rub robacte rales | 0.0006 | 0.002 5 | 0.0001 | 0.000 5 | 0.0001 | 0.13 | 0.77 | 0.87 | 0.23 | 0.74 | 0.85 | 0.29 |
| o Tur icibacte rales | 0.0017 | 0.003 3 | 0.0004 | 0.001 4 | 0.0000 | 0.22 | 0.83 | 0.88 | 0.53 | 0.77 | 0.83 | 0.49 |
| o_Rho docycla | 0.0017 | 0.007 5 | 0.0004 | 0.001 2 | 0.0003 | 0.23 | 0.79 | 0.88 | 0.40 | 0.77 | 0.87 | 0.41 |
| les | | | | | | | | | | | | |
| o RF 39 | 0.0013 | 0.004 4 | 0.0004 | 0.001 1 | 0.0000 | 0.26 | 0.79 | 0.89 | 0.43 | 0.76 | 0.85 | 0.44 |
| o_Soli bacteral es | 0.0003 | 0.001 8 | 0.0010 | 0.002 8 | 0.0008 | 3.87 | 0.78 | 0.90 | 0.19 | 0.77 | 0.87 | 0.33 |

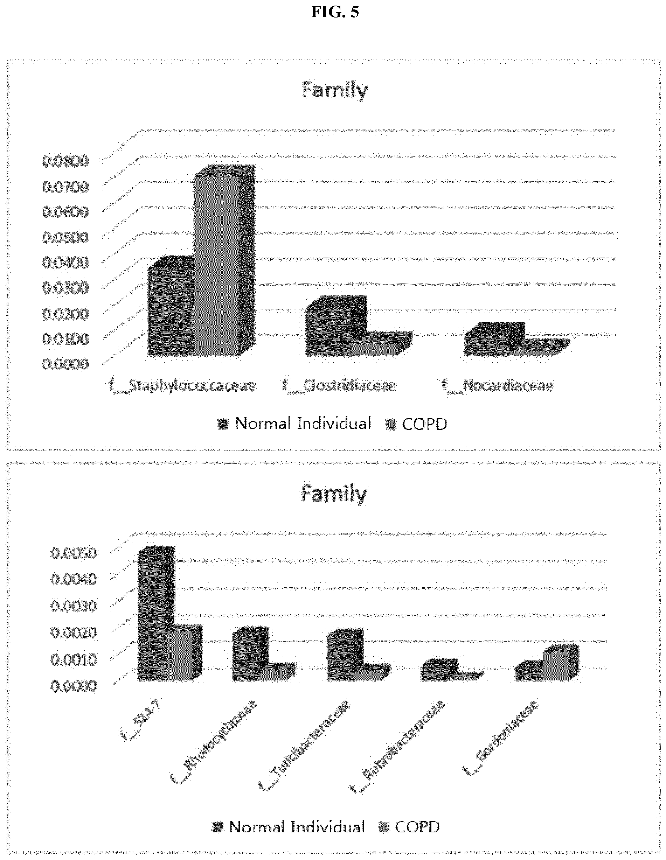

As a result of analyzing bacteria-derived extracellular vesicles in blood at a family level, a diagnostic model developed using, as a biomarker, one or more bacteria from the family *Rubrobacteraceae,* the family *Turicibacteraceae,* the family *Rhodocyclaceae,* the family *Nocardiaceae,* the family *Clostridiaceae,* the family S24-7, the family *Staphylococcaceae,* and the family *Gordoniaceae* exhibited significant diagnostic performance for COPD (see Table 5 and FIG. 5).

**[Table 5]**

| | normal individual | | COPD | | t-test | | Training Set | | | Test Set | | |
|---|---|---|---|---|---|---|---|---|---|---|---|---|
| Taxon | Mean | SD | Mean | SD | p-value | Ratio | AUC | sensit ivity | specif icity | AUC | sensit ivity | speci ficity |
| f_Rubrobac teraceae | 0.000 6 | 0.002 5 | 0.000 1 | 0.000 5 | 0.000 1 | 0.13 | 0.77 | 0.87 | 0.23 | 0.74 | 0.85 | 0.29 |
| f _Turicibact eraceae | 0.001 7 | 0.003 3 | 0.000 4 | 0.001 4 | 0.000 0 | 0.22 | 0.83 | 0.88 | 0.53 | 0.77 | 0.83 | 0.49 |
| f_Rhodocyc laceae | 0.001 7 | 0.007 5 | 0.000 4 | 0.001 2 | 0.000 3 | 0.23 | 0.79 | 0.88 | 0.40 | 0.77 | 0.87 | 0.42 |
| f_Nocardiac eae | 0.008 2 | 0.035 6 | 0.002 0 | 0.002 8 | 0.000 3 | 0.25 | 0.77 | 0.88 | 0.24 | 0.77 | 0.87 | 0.32 |
| f_Clostridia ceae | 0.018 9 | 0.043 9 | 0.004 8 | 0.006 2 | 0.000 0 | 0.25 | 0.82 | 0.87 | 0.47 | 0.80 | 0.85 | 0.58 |
| f_S24-7 | 0.004 8 | 0.012 5 | 0.001 8 | 0.003 7 | 0.000 0 | 0.38 | 0.78 | 0.87 | 0.29 | 0.72 | 0.87 | 0.29 |
| f_Staphylocl occaceae | 0.034 4 | 0.059 1 | 0.070 0 | 0.071 1 | 0.000 0 | 2.04 | 0.82 | 0.92 | 0.33 | 0.83 | 0.90 | 0.42 |
| f_Gordonia ceae | 0.000 5 | 0.002 3 | 0.001 1 | 0.002 6 | 0.004 3 | 2.17 | 0.77 | 0.88 | 0.21 | 0.76 | 0.84 | 0.29 |

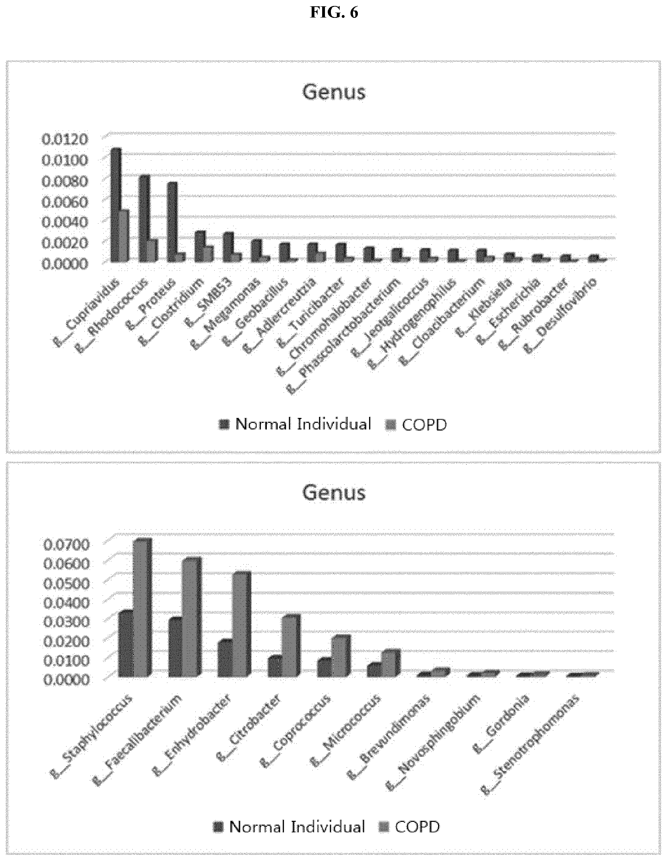

As a result of analyzing bacteria-derived extracellular vesicles in blood at a genus level, a diagnostic model developed using, as a biomarker, one or more bacteria from the genus *Hydrogenophilus,* the genus *Proteus,* the genus *Geobacillus,* the genus *Chromohalobacter,* the genus *Rubrobacter,* the genus *Megamonas,* the genus *Turicibacter,* the genus *Rhodococcus,* the genus *Phascolarctobacterium,* the genus SMB53, the genus *Desulfovibrio,* the genus *Jeotgalicoccus,* the genus *Cloacibacterium,* the genus *Klebsiella,* the genus *Escherichia,* the genus *Cupriavidus,* the genus *Adlercreutzia,* the genus *Clostridium,* the genus *Faecalibacterium,* the genus *Stenotrophomonas,* the genus *Staphylococcus,* the genus *Gordonia,* the genus *Micrococcus,* the genus *Coprococcus,* the genus *Novosphingobium,* the genus *Enhydrobacter,* the genus *Citrobacter,* and the genus *Brevundimonas* exhibited significant diagnostic performance for COPD (see Table 6 and FIG. 6).

**[Table 6]**

| | normal individual | | COPD | | t-test | | Training Set | | | Test Set | | |
|---|---|---|---|---|---|---|---|---|---|---|---|---|
| Taxon | Mean | SD | Mean | SD | p-value | Rati 3 | AUC | sensit ivity | speci ficity | AUC | sensit ivity | speci ficity |
| g_Hydrogeno philus | 0.001 1 | 0.007 0 | 0.000 1 | 0.000 4 | 0.002 1 | 0.09 | 0.78 | 0.88 | 0.38 | 0.79 | 0.87 | 0.40 |
| g_Proteus | 0.007 5 | 0.022 5 | 0.000 7 | 0.002 4 | 0.000 0 | 0.10 | 0.82 | 0.89 | 0.47 | 0.83 | 0.87 | 0.49 |
| g_Geobacillu s | 0.001 7 | 0.006 1 | 0.000 2 | 0.000 9 | 0.000 0 | 0.11 | 0.77 | 0.87 | 0.23 | 0.75 | 0.85 | 0.32 |
| g_Chromohal obacter | 0.001 3 | 0.007 3 | 0.000 2 | 0.001 2 | 0.001 0 | 0.12 | 0.78 | 0.87 | 0.32 | 0.74 | 0.84 | 0.35 |
| g_ Rubrobacte r | 0.000 6 | 0.002 5 | 0.000 1 | 0.000 5 | 0.000 1 | 0.13 | 0.77 | 0.87 | 0.23 | 0.74 | 0.85 | 0.29 |
| g_Megamona s | 0.002 0 | 0.009 4 | 0.000 4 | 0.001 4 | 0.000 5 | 0.21 | 0.77 | 0.88 | 0.30 | 0.75 | 0.86 | 0.33 |
| g_Turicibacte r | 0.001 7 | 0.003 3 | 0.000 4 | 0.001 4 | 0.000 0 | 0.22 | 0.83 | 0.88 | 0.53 | 0.77 | 0.83 | 0.49 |
| g_Rhodococc us | 0.008 2 | 0.035 5 | 0.002 0 | 0.002 8 | 0.000 3 | 0.25 | 0.77 | 0.88 | 0.24 | 0.77 | 0.87 | 0.32 |
| g_Phascolarct obacterium | 0.001 2 | 0.002 6 | 0.000 3 | 0.001 0 | 0.000 0 | 0.26 | 0.81 | 0.88 | 0.47 | 0.74 | 0.84 | 0.44 |
| g_SMB53 | 0.002 7 | 0.007 6 | 0.000 7 | 0.001 6 | 0.000 0 | 0.27 | 0.80 | 0.88 | 0.38 | 0.77 | 0.86 | 0.33 |
| g Desulfovib rio | 0.000 5 | 0.002 0 | 0.000 1 | 0.000 6 | 0.000 2 | 0.28 | 0.77 | 0.87 | 0.23 | 0.76 | 0.87 | 0.31 |
| g_Jeotgalicoc cus | 0.001 1 | 0.004 2 | 0.000 4 | 0.001 6 | 0.000 5 | 0.31 | 0.78 | 0.87 | 0.31 | 0.74 | 0.84 | 0.35 |
| g_Cloacibacte rium | 0.001 1 | 0.003 7 | 0.000 4 | 0.001 4 | 0.000 8 | 0.39 | 0.77 | 0.88 | 0.22 | 0.76 | 0.87 | 0.29 |
| g_Klebsiella | 0.000 7 | 0.001 7 | 0.000 3 | 0.000 6 | 0.000 0 | 0.39 | 0.77 | 0.87 | 0.29 | 0.76 | 0.86 | 0.35 |
| g_Escherichia | 0.000 6 | 0.000 9 | 0.000 3 | 0.000 3 | 0.000 0 | 0.43 | 0.82 | 0.88 | 0.44 | 0.81 | 0.87 | 0.56 |
| g_Cupriavidu s | 0.010 7 | 0.023 7 | 0.004 9 | 0.005 2 | 0.000 0 | 0.45 | 0.78 | 0.87 | 0.36 | 0.75 | 0.82 | 0.35 |
| g_Adlercreutz ia | 0.0011 7 | 0.003 8 | 0.000 8 | 0.001 4 | 0.000 0 | 0.49 | 0.78 | 0.88 | 0.35 | 0.77 | 0.84 | 0.35 |
| g_Clostridiu m | 0.002 8 | 0.007 5 | 0.001 4 | 0.003 2 | 0.000 6 | 0.50 | 0.78 | 0.87 | 0.34 | 0.75 | 0.87 | 0.36 |
| g_Faecalibact erium | 0.029 2 | 0.029 0 | 0.059 7 | 0.036 1 | 0.000 0 | 2.04 | 0.86 | 0.91 | 0.53 | 0.87 | 0.91 | 0.53 |
| g_Stenotroph omonas | 0.000 3 | 0.001 3 | 0.000 7 | 0.001 6 | 0.005. 9 | 2.05 | 0.78 | 0.89 | 0.25 | 0.77 | 0.86 | 0.38 |
| g_Staphyloco ccus | 0.032 8 | 0.058 8 | 0.069 4 | 0.071 1 | 0.000 0 | 2.12 | 0.83 | 0.92 | 0.34 | 0.83 | 0.90 | 0.42 |
| g_Gordonia | 0.000 5 | 0.002 3 | 0.001 1 | 0.002 6 | 0.004 3 | 2.17 | 0.77 | 0.88 | 0.21 | 0.76 | 0.84 | 0.29 |
| g_Micrococc us | 0.005 7 | 0.010 0 | 0.012 5 | 0.012 1 | 0.000 0 | 2.20 | 0.82 | 0.91 | 0.37 | 0.80 | 0.85 | 0.43 |
| g_Coprococc us | 0.008 3 | 0.011 5 | 0.019 9 | 0.015 9 | 0.000 0 | 2.40 | 0.86 | 0.95 | 0.54 | 0.80 | 0.92 | 0.36 |
| g Novosphin gobium | 0.000 7 | 0.002 7 | 0.001 8 | 0.004 4 | 0.001 6 | 2.42 | 0.79 | 0.90 | 0.30 | 0.77 | 0.85 | 0.29 |
| g_Enhydroba cter | 0.017 7 | 0.034 4 | 0.052 5 | 0.047 8 | 0.000 0 | 2.97 | 0.88 | 0.91 | 0.50 | 0.84 | 0.89 | 0.53 |
| g_ Citrobacter | 0.009 5 | 0.014 6 | 0.030 5 | 0.021 4 | 0.000 0 | 3.21 | 0.91 | 0.94 | 0.63 | 0.84 | 0.90 | 0.44 |
| g_Brevundim onas | 0.000 9 | 0.003 6 | 0.003 1 | 0.005 0 | 0.000 0 | 3.38 | 0.80 | 0.93 | 0.31 | 0.80 | 0.91 | 0.36 |

### Example 5. Asthma Diagnostic Model Based on Metagenomic Analysis of Bacteria-Derived EVs Isolated from Blood of Normal Individuals and Asthma Patients

Extracellular vesicles were isolated from blood samples of 219 asthma patients and 236 normal individuals, the two groups matched in age and gender, and then metagenomic sequencing was performed thereon using the method of Example 3. For the development of a diagnostic model, first, a strain exhibiting a p value of less than 0.05 between two groups in a t-test and a difference of two-fold or more between two groups was selected, and then an area under curve (AUC), sensitivity, and specificity, which are diagnostic performance indexes, were calculated by logistic regression analysis.

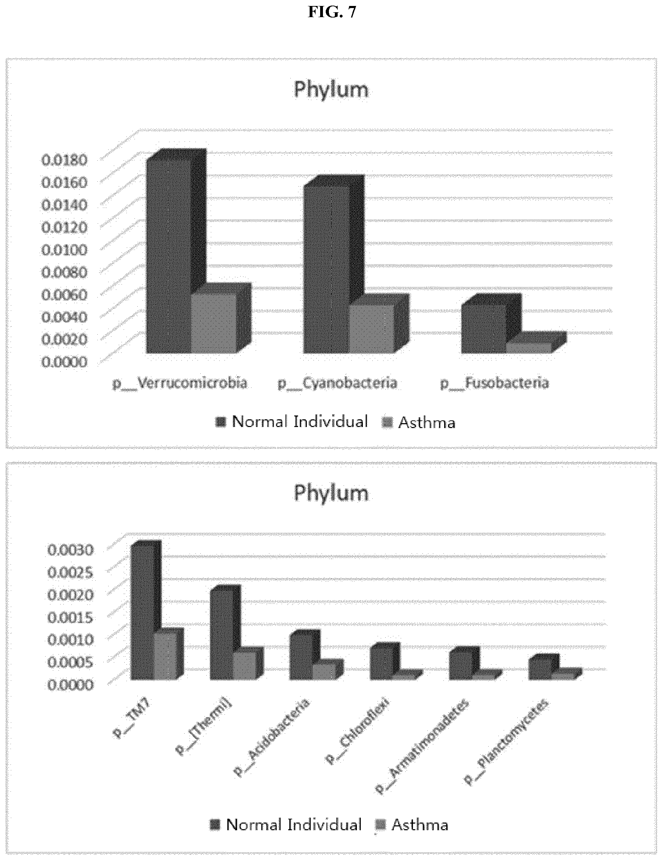

As a result of analyzing bacteria-derived extracellular vesicles in blood at a phylum level, a diagnostic model developed using, as a biomarker, one or more bacteria from the phylum *Chloroflexi,* the phylum *Armatimonadetes,* the phylum *Fusobacteria,* the phylum *Cyanobacteria,* the phylum *Planctomycetes,* the phylum *Thermi,* the phylum *Verrucomicrobia,* the phylum *Acidobacteria,* and the phylum TM7 exhibited significant diagnostic performance for asthma (see Table 7 and FIG. 7).

**[Table 7]**

| | normal individual | | asthma | | t-test | | Training Set | | | Test Set | | |
|---|---|---|---|---|---|---|---|---|---|---|---|---|
| Taxon | Mean | SD | Mean | SD | p-value | Rati o | AUC | sensit ivity | specif icity | AU C | sensit ivity | specif icity |
| p_Chloro flex 1 | 0.000 7 | 0.003 0 | 0.000 1 | 0.000 3 | 0.000 0 | 0.14 | 0.67 | 0.96 | 0.13 | 0.57 | 0.92 | 0.10 |
| p_Armatimo nadetes | 0.000 6 | 0.002 4 | 0.000 1 | 0.000 4 | 0.000 0 | 0.17 | 0.66 | 0.96 | 0.12 | 0.55 | 0.93 | 0.07 |
| p_Fusobacte ria | 0.004 3 | 0.009 5 | 0.000 9 | 0.002 9 | 0.000 0 | 0.21 | 0.73 | 0.90 | 0.26 | 0.68 | 0.88 | 0.24 |
| p_Cyanobact eria | 0.014 8 | 0.039 7 | 0.004 3 | 0.011 9 | 0.000 0 | 0.29 | 0.72 | 0.93 | 0.18 | 0.67 | 0.89 | 0.13 |
| p_Planctomy cetes | 0.000 4 | 0.001 8 | 0.000 1 | 0.000 8 | 0.002 3 | 0.30 | 0.64 | 0.97 | 0.11 | 0.58 | 0.95 | 0.06 |
| p_[Thermi] | 0.002 0 | 0.004 7 | 0.000 6 | 0.001 9 | 0.000 0 | 0.30 | 0.69 | 0.94 | 0.16 | 0.59 | 0.91 | 0.13 |
| p_Verrucomi crobia | 10.017 2 | 0.026 6 | 0.005 3 | 0.006 0 | 0.000 0 | 0.31 | 0.78 | 0.88 | 0.38 | 0.72 | 0.82 | 0.39 |
| p_Acidobact eria | 0.001 0 | 0.003 3 | 0.000 3 | 0.001 1 | 0.000 2 | 0.33 | 0.64 | 0.95 | 0.12 | 0.60 | 0.93 | 0.07 |
| p_TM7 | 0.003 0 | 0.005 5 | 0.001 0 | 0.002 9 | 0.000 0 | 0.35 | 0.70 | 0.94 | 0.16 | 0.60 | 0.96 | 0.08 |

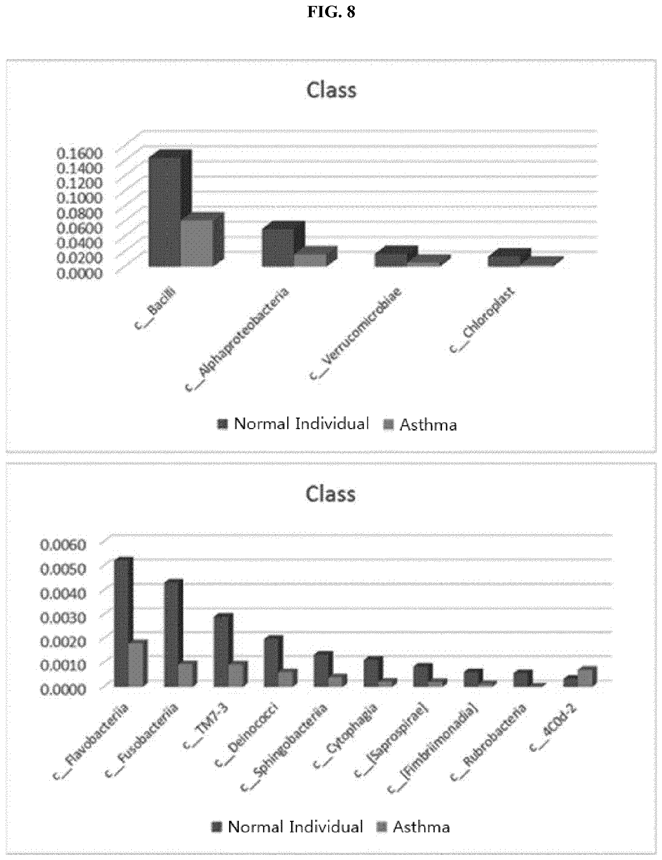

As a result of analyzing bacteria-derived extracellular vesicles in blood at a class level, a diagnostic model developed using, as a biomarker, one or more bacteria from the class *Rubrobacteria,* the class *Fimbriimonadia,* the class *Cytophagia,* the class *Chloroplast,* the class *Fusobacteriia,* the class *Saprospirae,* the class *Sphingobacteriia,* the class *Deinococci,* the class *Verrucomicrobiae,* the class TM7-3, the class *Alphaproteobacteria,* the class *Flavobacteriia,* the class *Bacilli,* and the class 4C0d-2 exhibited significant diagnostic performance for asthma (see Table 8 and FIG. 8).

**[Table 8]**

| | normal individual | | asthma | | t-test | | Training Set | | | Test Set | | |
|---|---|---|---|---|---|---|---|---|---|---|---|---|
| Taxon | Mean | SD | Mean | SD | p-value | Ratio | AUC | sensit ivity | speci ficity | AUC | sensit ivity | speci ficity |
| c_Rubrobact eria | 0.000 6 | 0.002 5 | 0.000 0 | 0.000 0 | 0.000 0 | 0.00 | 0.67 | 0.95 | 0.16 | 0.62 | 0.93 | 0.10 |
| c_[Fimbriim onadia] | 0.000 6 | 0.002 4 | 0.000 1 | 0.000 4 | 0.000 0 | 0.17 | 0.66 | 0.96 | 0.12 | 0.55 | 0.93 | 0.07 |
| c_Cytophagi a | 0.001 1 | 0.003 6 | 0.000 2 | 0.000 8 | 0.000 0 | 0.18 | 0.67 | 0.96 | 0.13 | 0.60 | 0.92 | 0.07 |
| c Chloropla st | 0.013 5 | 0.039 4 | 0.002 7 | 0.004 9 | 0.000 0 | 0.20 | 0.73 | 0.90 | 0.27 | 0.69 | 0.86 | 0.26 |
| c Fusobacte riia | 0.004 3 | 0.009 5 | 0.000 9 | 0.002 9 | 0.000 0 | 0.21 | 0.73 | 0.90 | 0.26 | 0.68 | 0.88 | 0.24 |
| c_[Saprospir ae] | 0.000 8 | 0.002 8 | 0.000 2 | 0.000 6 | 0.000 0 | 0.23 | 0.64 | 0.96 | 0.11 | 0.60 | 0.95 | 0.04 |
| c_Sphingoba cteriia | 0.001 3 | 0.004 2 | 0.000 4 | 1 | 0.000 0 | 0.29 | 0.65 | 0.96 | 0.10 | 0.58 | 0.92 | 0.06 |
| c_Deinococc i | 0.002 0 | 0.004 7 | 0.000 6 | 0.001 9 | 0.000 0 | 0.30 | 0.69 | 0.94 | 0.16 | 0.59 | 0.91 | 0.13 |
| c_Verrucom icrobiae | 0.016 9 | 0.026 5 | 0.005 2 | 0.006 0 | 0.000 0 | 0.31 | 0.78 | 0.88 | 0.39 | 0.72 | 0.82 | 0.38 |
| c TM7-3 | 0.002 9 | 0.005 5 | 0.000 9 | 0.002 7 | 0.000 0 | 0.32 | 0.70 | 0.94 | 0.17 | 0.61 | 0.95 | 0.08 |
| c Alphaprot eobacteria | 0.049 8 | 0.042 7 | 0.0161 3 | 0.032 3 | 0.000 0 | 0.33 | 0.81 | 0.88 | 0.50 | 0.77 | 0.85 | 0.32 |
| c Flavobact eriia | 0.005 2 | 0.007 6 | 0.001 8 | 0.006 6 | 0.000 0 | 0.34 | 0.73 | 0.93 | 0.20 | 0.69 | 0.95 | 0.14 |
| c Bacilli | 0.144 1 | 0.092 4 | 0.061 1 | 0.040 8 | 0.000 0 | 0.42 | 0.87 | 0.88 | 0.65 | 0.82 | 0.85 | 0.60 |
| c_4C0d-2 | 0.000 3 | 0.001 2 | 0.000 7 | 0.001 4 | 0.001 1 | 2.13 | 0.65 | 0.98 | 0.10 | 0.59 | 0.96 | 0.10 |

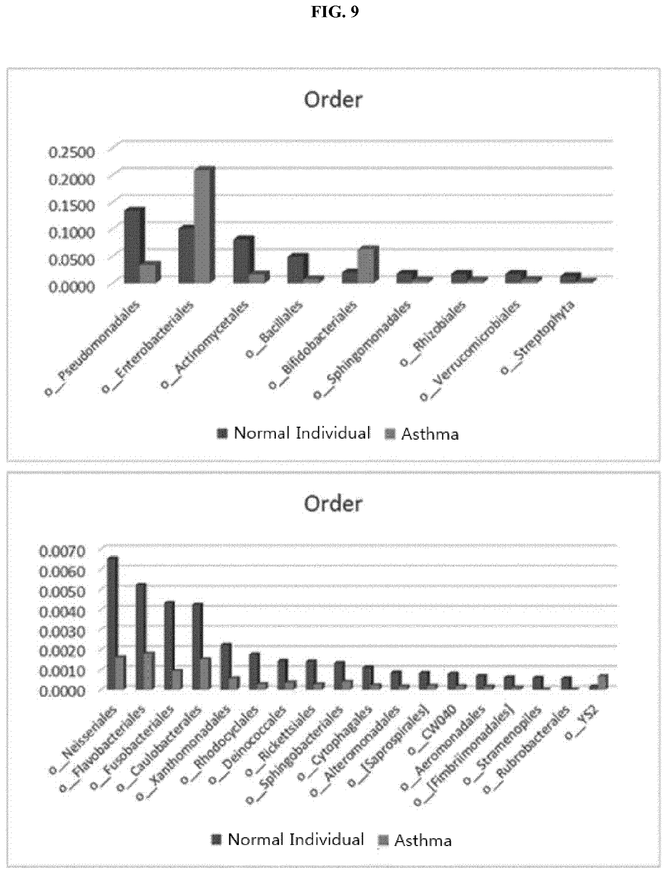

As a result of analyzing bacteria-derived extracellular vesicles in blood at an order level, a diagnostic model developed using, as a biomarker, one or more bacteria from the order *Rubrobacterales,* the order *Stramenopiles,* the order *Bacillales,* the order *Rhodocyclales,* the order *Fimbriimonadales,* the order *Cytophagales,* the order *Rickettsiales,* the order *Alteromonadales,* the order *Actinomycetales,* the order *Streptophyta,* the order *Fusobacteriales,* the order CW040, the order *Saprospirales,* the order *Aeromonadales,* the order *Neisseriales,* the order *Rhizobiales,* the order *Pseudomonadales,* the order *Deinococcales,* the order *Xanthomonadales,* the order *Sphingomonadales,* the order *Sphingobacteriales,* the order *Verrucomicrobiales,* the order *Flavobacteriales,* the order *Caulobacterales,* the order *Enterobacteriales,* the order *Bifidobacteriales,* and the order YS2 exhibited significant diagnostic performance for asthma (see Table 9 and FIG. 9).

**[Table 9]**

| | normal individual | | asthma | | t-test | | Training Set | | | Test Set | | |
|---|---|---|---|---|---|---|---|---|---|---|---|---|
| Taxon | Mean | SD | Mean | SD | p-value | Ratio | AUC | sensit ivity | speci ficity | AUC | sensit ivity | speci ficity |
| o_Rubrobac terales | 0.000 6 | 0.002 5 | 0.000 0 | 0.000 0 | 0.000 0 | 0.00 | 0.67 | 0.95 | 0.16 | 0.62 | 0.93 | 0.10 |
| o_Strameno piles | 0.000 6 | 0.003 4 | 0.000 0 | 0.000 1 | 0.000 3 | 0.01 | 0.64 | 0.99 | 0.06 | 0.57 | 0.98 | 0.03 |
| o_Bacillales | 0.048 4 | 0.071 1 | 0.007 1 | 0.008 5 | 0.000 0 | 0.15 | 0.88 | 0.82 | 0.73 | 0.79 | 0.77 | 0.57 |
| o_Rhodocyc lales | 7 | 0.007 5 | 0.000 3 | 0.000 9 | 0.000 0 | 0.16 | 0.68 | 0.97 | 0.15 | 0.63 | 0.92 | 0.10 |
| o_[Fimbrii monadales] | 0.000 6 | 0.002 4 | 0.000 1 | 0.000 4 | 0.000 0 | 0.17 | 0.66 | 0.96 | 0.12 | 0.55 | 0.93 | 0.07 |
| o_Cytophag ales | 0.001 1 | 0.003 6 | 0.000 2 | 0.000 8 | 0.000 0 | 0.18 | 0.67 | 0.96 | 0.13 | 0.60 | 0.92 | 0.07 |
| o_Rickettsia les | 0.001 4 | 0.005 3 | 0.000 3 | 0.001. 1 | 0.000 0 | 0.19 | 0.67 | 0.96 | 0.13 | 0.59 | 0.94 | 0.10 |
| ο_Alteromo nadales | 0.000 9 | 0.002 3 | 0.000 2 | 0.000 7 | 0.000 0 | 0.19 | 0.69 | 0.95 | 0.16 | 0.62 | 0.92 | 0.10 |
| o_Actinomy cetales | 0.080 5 | 0.080 2 | 0.016 0 | 0.021 8 | 0.000 0 | 0.20 | 0.88 | 0.84 | 0.71 | 0.80 | 0.81 | 0.53 |
| ο_Streptoph yta | 0.012 8 | 0.039 1 | 0.002 7 | 0.004 8 | 0.000 0 | 0.21 | 0.73 | 0.90 | 0.24 | 0.69 | 0.88 | 0.22 |
| o_Fusobacte riales | 0.004 3 | 0.009 5 | 0.000 9 | 0.002 9 | 0.000 0 | 0.21 | 0.73 | 0.90 | 0.26 | 0.68 | 0.88 | 0.24 |
| o_CW040 | 0.000 8 | 0.003 1 | 0.000 2 | 0.000 9 | 0.000 1 | 0.22 | 0.67 | 0.97 | 0.14 | 0.57 | 0.93 | 0.10 |
| ο_[Saprospi rales] | 0.000 8 | 0.002 8 | 0.000 2 | 0.000 6 | 0.000 0 | 0.23 | 0.64 | 0.96 | 0.11 | 0.60 | 0.95 | 0.04 |
| o_Aeromon adales | 0.000 7 | 0.002 8 | 0.000 2 | 0.000 7 | 0.000 3 | 0.24 | 0.66 | 0.96 | 0.13 | 0.60 | 0.95 | 0.06 |
| o_Neisserial es | 0.006 5 | 0.017 8 | 0.001 6 | 0.006 4 | 0.000 0 | 0.25 | 0.74 | 0.86 | 0.27 | 0.65 | 0.90 | 0.26 |
| ο_Rhizobial es | 0.017 1 | 0.018 6 | 0.004 3 | 0.005 7 | 0.000 0 | 0.25 | 0.78 | 0.84 | 0.48 | 0.75 | 0.87 | 0.33 |
| o_Pseudom onadales | 0.133 6 | 0.112 5 | 0.033 5 | 0.049 6 | 0.000 0 | 0.25 | 0.85 | 0.82 | 0.65 | 0.79 | 0.80 | 0.57 |
| o_Deinococ cales | 0.001 4 | 0.004 2 | 0.000 4 | 0.001 3 | 0.000 0 | 0.25 | 0.68 | 0.95 | 0.16 | 0.60 | 0.91 | 0.13 |
| o_Xanthom onadales | 0.002 2 | 0.004 1 | 0.000 6 | 0.001 2 | 0.000 0 | 0.26 | 0.72 | 0.93 | 0.25 | 0.66 | 0.92 | 0.21 |
| ο_Sphingo monadales | 0.017 3 | 0.019 9 | 0.004 9 | 0.008 2 | 0.000 0 | 0.29 | 0.78 | 0.87 | 0.44 | 0.71 | 0.88 | 0.25 |
| ο_Sphingob acteriales | 0.001 3 | 0.004 2 | 0.000 4 | 1 | 0.000 0 | 0.29 | 0.65 | 0.96 | 0.10 | 0.58 | 0.92 | 0.06 |
| o_icrobiales | 0.016 9 | 0.026 5 | 0.005 2 | 0.006 0 | 0.000 0 | 0.31 | 0.78 | 0.88 | 0.39 | 0.72 | 0.82 | 0.38 |
| o_Flavobact eriales | 0.005 2 | 0.007 6 | 0.001 8 | 0.006 6 | 0.000 0 | 0.34 | 0.73 | 0.93 | 0.20 | 0.69 | 0.95 | 0.14 |
| o_Caulobact erales | 0.004 2 | 0.007 2 | 0.001 5 | 0.003 0 | 0.000 0 | 0.36 | 0.72 | 0.90 | 0.24 | 0.59 | 0.92 | 0.18 |
| o_Enterobac teriales | 6 | 0.078 3 | 0.209 1 | 0.087 8 | 0.000 0 | 2.08 | 0.84 | 0.91 | 0.54 | 0.85 | 0.93 | 0.50 |
| o_Bifidobac teriales | 0.019 6 | 0.022 0 | 0.062 7 | 0.033 5 | 0.000 0 | 3.19 | 0.87 | 0.93 | 0.61 | 0.91 | 0.97 | 0.54 |
| o_YS2 | 0.000 2 | 0.000 6 | 0.000 7 | 0.001 4 | 0.000 0 | 4.30 | 0.69 | 0.96 | 0.16 | 0.65 | 0.97 | 0.17 |

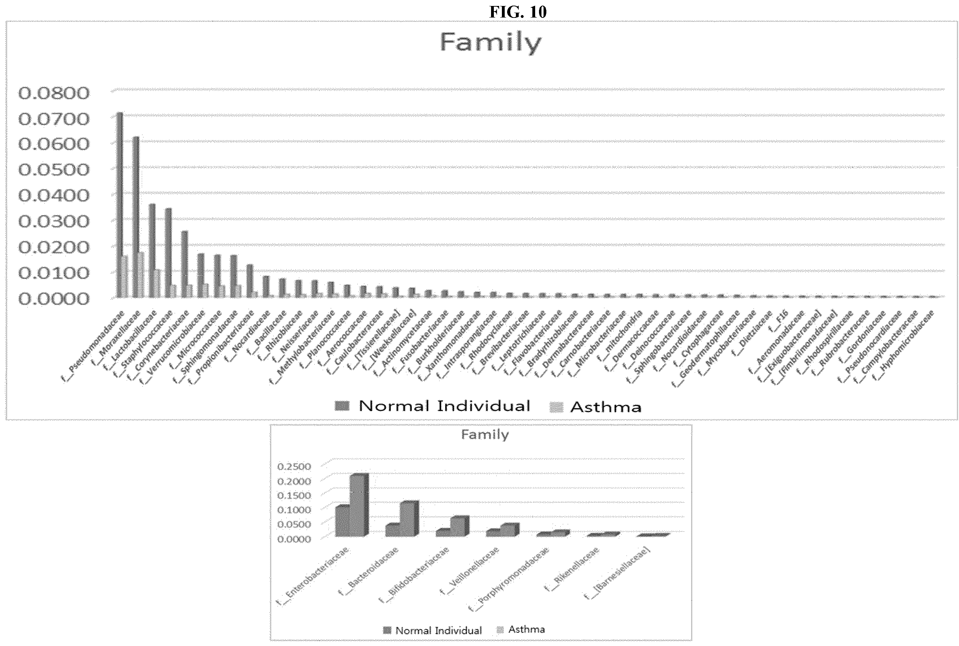

As a result of analyzing bacteria-derived extracellular vesicles in blood at a family level, a diagnostic model developed using, as a biomarker, one or more bacteria from the family *Rubrobacteraceae,* the family *Exiguobacteraceae,* the family *Nocardiaceae,* the family F16, the family *Pseudonocardiaceae,* the family *Dermabacteraceae,* the family *Brevibacteriaceae,* the family *Microbacteriaceae,* the family *Staphylococcaceae,* the family *Cytophagaceae,* the family *Planococcaceae,* the family *Tissierellaceae,* the family *Rhodocyclaceae,* the family *Propionibacteriaceae,* the family *Fimbriimonadaceae,* the family *Campylobacteraceae,* the family *Dermacoccaceae,* the family *Burkholderiaceae,* the family *Rhizobiaceae,* the family *Bacillaceae,* the family *Corynebacteriaceae,* the family *mitochondria,* the family *Fusobacteriaceae,* the family *Leptotrichiaceae,* the family *Pseudomonadaceae,* the family *Bradyrhizobiaceae,* the family *Aeromonadaceae,* the family *Neisseriaceae,* the family *Methylobacteriaceae,* the family *Carnobacteriaceae,* the family *Xanthomonadaceae,* the family *Geodermatophilaceae,* the family *Mycobacteriaceae,* the family *Gordon*ž*aceae,* the family *Micrococcaceae,* the family *Hyphomicrobiaceae,* the family *Moraxellaceae,* the family *Sphingomonadaceae,* the family *Actinomycetaceae,* the family *Deinococcaceae,* the family *Intrasporangiaceae,* the family *Flavobacteriaceae,* the family *Lactobacillaceae,* the family *Verrucomicrobiaceae,* the family *Nocardioidaceae,* the family *Sphingobacteriaceae,* the family *Rhodospirillaceae,* the family *Caulobacteraceae,* the family *Weeksellaceae,* the family *Dietziaceae,* the family *Aerococcaceae,* the family *Porphyromonadaceae,* the family *Veillonellaceae,* the family *Enterobacteriaceae,* the family *Barnesiellaceae,* the family *Rikenellaceae,* the family *Bacteroidaceae,* and the family *Bifidobacteriaceae* exhibited significant diagnostic performance for asthma (see Table 10 and FIG. 10).

**[Table 10]**

| | normal individual | | asthma | | t-test | | Training Set | | | Test Set | | |
|---|---|---|---|---|---|---|---|---|---|---|---|---|
| Taxon | Mean | SD | Mean | SD | p-value | Ratio | AUC | sensit ivity | speci ficity | AUC | sensit ivity | speci ficity |
| f_ Rubrobacte raceae | 0.000 6 | 0.002 5 | 0.000 0 | 0.000 0 | 0.000 0 | 0.00 | 0.67 | 0.95 | 0.16 | 0.62 | 0.93 | 0.10 |
| f_[Exiguobac teraceae] | 0.000 7 | 0.003 4 | 0.000 0 | 0.000 2 | 0.000 1 | 0.04 | 0.67 | 0.96 | 0.14 | 0.59 | 0.93 | 0.07 |
| f_Nocardiace ae | 0.008 2 | 0.035 6 | 0.000 8 | 0.001 9 | 0.000 0 | 0.09 | 0.73 | 0.91 | 0.27 | 0.63 | 0.88 | 0.21 |
| f_F16 | 0.000 7 | 0.003 1 | 0.000 1 | 0.000 5 | 0.000 0 | 0.09 | 0.67 | 0.96 | 0.14 | 0.60 | 0.92 | 0.10 |
| f_Pseudonoca rdiaceae | 0.000 5 | 0.002 8 | 0.000 1 | 0.000 2 | 0.001 3 | 0.11 | 0.67 | 0.95 | 0.16 | 0.57 | 0.92 | 0.08 |
| f_Dermabacte raceae | 0.001 3 | 0.004 7 | 0.000 2 | 0.000 5 | 0.000 0 | 0.12 | 0.70 | 0.92 | 0.20 | 0.62 | 0.92 | 0.10 |
| f_Brevibacter iaceae | 0.001 7 | 0.006 6 | 0.000 2 | 0.000 6 | 0.000 0 | 0.12 | 0.71 | 0.92 | 0.21 | 0.64 | 0.92 | 0.15 |
| f Microbacte riaceae | 0.001 3 | 0.007 9 | 0.000 2 | 0.000 8 | 0.003 4 | 0.13 | 0.71 | 0.94 | 0.19 | 0.67 | 0.94 | 0.14 |
| f_Staphyloco ccaceae | 0.034 4 | 1 | 0.004 7 | 0.006 8 | 0.000 0 | 0.14 | 0.85 | 0.82 | 0.61 | 0.75 | 0.80 | 0.43 |
| f_Cytophagac | 0.001 | 0.003 | 0.000 | 0.000 | 0.000 | 0.15 | 0.67 | 0.96 | 0.14 | 0.58 | 0.92 | 0.07 |
| eae | 1 | 6 | 2 | 5 | 0 | | | | | | | |
| f_Planococca ceae | 0.004 8 | 9 | 0.000 7 | 0.001 3 | 0.000 0 | 0.15 | 0.82 | 0.85 | 0.53 | 0.71 | 0.79 | 0.40 |
| f_[Tissierella ceae] | 0.003 8 | 0.008 5 | 0.000 6 | 0.001 6 | 0.000 0 | 0.15 | 0.74 | 0.93 | 0.29 | 0.70 | 0.90 | 0.22 |
| f_Rhodocycla ceae | 0.001 7 | 0.007 5 | 0.000 3 | 0.000 9 | 0.000 0 | 0.16 | 0.68 | 0.97 | 0.15 | 0.63 | 0.92 | 0.10 |
| f_Propionibac teriaceae | 7 | 0.015 4 | 0.002 1 | 0.003 1 | 0.000 0 | 0.17 | 0.83 | 0.84 | 0.56 | 0.77 | 0.83 | 0.43 |
| f_[Fimbriimo nadaceae] | 0.000 6 | 0.002 4 | 0.000 1 | 0.000 4 | 0.000 0 | 0.17 | 0.66 | 0.96 | 0.12 | 0.55 | 0.93 | 0.07 |
| f_Campyloba cteraceae | 0.000 5 | 0.001 6 | 0.000 1 | 0.000 4 | 0.000 0 | 0.17 | 0.66 | 0.95 | 0.14 | 0.60 | 0.92 | 0.08 |
| f_Dermacocc aceae | 0.001 2 | 0.003 6 | 0.000 2 | 0.000 5 | 0.000 0 | 0.18 | 0.68 | 0.94 | 0.18 | 0.58 | 0.90 | 0.10 |
| f_Burkholderi aceae | 3 | 0.006 6 | 0.000 4 | 0.001 0 | 0.000 0 | 0.18 | 0.71 | 0.94 | 0.23 | 0.63 | 0.93 | 0.10 |
| f_Rhizobiace ae | 0.006 6 | 0.009 9 | 0.001 2 | 0.002 5 | 0.000 0 | 0.18 | 0.75 | 0.86 | 0.31 | 0.71 | 0.88 | 0.25 |
| f_Bacillaceae | 0.007 2 | 0.010 6 | 0.001 3 | 0.002 0 | 0.000 0 | 0.18 | 0.79 | 0.86 | 0.45 | 0.72 | 0.86 | 0.33 |
| f_Corynebact eriaceae | 0.025 7 | 0.045 6 | 0.004 8 | 0.005 5 | 0.000 0 | 0.19 | 0.83 | 0.85 | 0.55 | 0.73 | 0.79 | 0.42 |
| f_mitochondr | 0.001 | 0.005 | 0.000 | 0.001 | 0.000 | 0.21 | 0.65 | 0.96 | 0.12 | 0.58 | 0.95 | 0.08 |
| ia | 3 | 2 | 3 | 1 | 1 | | | | | | | |
| f_Fusobacteri aceae | 0.002 7 | 2 | 0.000 6 | 0.002 6 | 0.000 0 | 0.21 | 0.72 | 0.92 | 0.23 | 0.66 | 0.90 | 0.15 |
| f_Leptotrichi aceae | 0.001 6 | 0.007 2 | 0.000 4 | 0.001 1 | 0.000 3 | 0.22 | 0.67 | 0.96 | 0.13 | 0.59 | 0.92 | 0.04 |
| f_Pseudomon adaceae | 0.071 4 | 0.071 7 | 0.016 0 | 0.015 4 | 0.000 0 | 0.22 | 0.83 | 0.82 | 0.64 | 0.75 | 0.75 | 0.56 |
| f_Bradyrhizo biaceae | 0.001 4 | 0.004 4 | 0.000 3 | 0.000 8 | 0.000 0 | 0.24 | 0.67 | 0.95 | 0.13 | 0.58 | 0.92 | 0.06 |
| f_Aeromonad aceae | 0.000 7 | 0.002 8 | 0.000 2 | 0.000 7 | 0.000 4 | 0.25 | 0.65 | 0.96 | 0.12 | 0.60 | 0.95 | 0.06 |
| f_Neisseriace ae | 0.006 5 | 0.017 8 | 0.001 6 | 0.006 4 | 0.000 0 | 0.25 | 0.74 | 0.86 | 0.27 | 0.65 | 0.90 | 0.26 |
| f_Methylobac teriaceae | 0.005 9 | 0.010 1 | 0.001 5 | 0.003 4 | 0.000 0 | 0.25 | 0.73 | 0.91 | 0.22 | 0.65 | 0.90 | 0.18 |
| f_Camobacte riaceae | 0.001 3 | 0.003 1 | 0.000 3 | 0.001 2 | 0.000 0 | 0.26 | 0.68 | 0.96 | 0.15 | 0.63 | 0.94 | 0.10 |
| f_Xanthomon adaceae | 0.002 1 | 0.004 1 | 0.000 5 | 0.001 2 | 0.000 0 | 0.26 | 0.71 | 0.93 | 0.24 | 0.66 | 0.92 | 0.22 |
| f_Geodermat ophilaceae | 0.001 0 | 0.003 3 | 0.000 3 | 0.001 2 | 0.000 1 | 0.27 | 0.67 | 0.97 | 0.12 | 0.58 | 0.94 | 0.08 |
| f_Mycobacter iaceae | 0.000 8 | 0.002 8 | 0.000 2 | 0.000 9 | 0.000 1 | 0.27 | 0.67 | 0.97 | 0.14 | 0.57 | 0.92 | 0.08 |
| f_Gordoniace | 0.000 | 0.002 | 0.000 | 0.000 | 0.001 | 0.27 | 0.66 | 0.96 | 0.14 | 0.55 | 0.92 | 0.08 |
| ae | 5 | 3 | 1 | 5 | 6 | | | | | | | |
| f_Micrococca ceae | 0.016 5 | 0.021 3 | 0.004 5 | 0.014 3 | 0.000 0 | 0.27 | 0.79 | 0.85 | 0.48 | 0.72 | 0.82 | 0.32 |
| f_Hyphomicr obiaceae | 0.000 5 | 0.001 7 | 0.000 1 | 0.000 6 | 0.000 3 | 0.27 | 0.65 | 0.96 | 0.14 | 0.57 | 0.94 | 0.07 |
| f_Moraxellac eae | 0.062 1 | 0 | 0.017 5 | 0.045 1 | 0.000 0 | 0.28 | 0.83 | 0.89 | 0.49 | 0.77 | 0.85 | 0.33 |
| f_Sphingomo nadaceae | 0.016 4 | 0.019 0 | 0.004 6 | 0.007 0 | 0.000 0 | 0.28 | 0.78 | 0.87 | 0.45 | 0.71 | 0.87 | 0.26 |
| f_Actinomyc etaceae | 0.002 7 | 7 | 0.000 8 | 0.003 8 | 0.000 0 | 0.29 | 0.76 | 0.88 | 0.35 | 0.70 | 0.88 | 0.29 |
| f_Deinococca ceae | 0.001 2 | 0.003 3 | 0.000 3 | 0.001 3 | 0.000 0 | 0.29 | 0.67 | 0.96 | 0.14 | 0.59 | 0.92 | 0.08 |
| f_Intrasporan giaceae | 0.002 0 | 6 | 0.000 6 | 0.001 3 | 0.000 0 | 0.30 | 0.70 | 0.93 | 0.18 | 0.64 | 0.93 | 0.11 |
| f_Flavobacter iaceae | 0.001 6 | 0.003 7 | 0.000 5 | 0.002 2 | 0.000 0 | 0.30 | 0.68 | 0.94 | 0.16 | 0.60 | 0.93 | 0.13 |
| f_Lactobacill aceae | 0.036 1 | 0.055 9 | 0.010 8 | 0.014 1 | 0.000 0 | 0.30 | 0.81 | 0.86 | 0.52 | 0.73 | 0.85 | 0.35 |
| f_Verrucomic robiaceae | 0.016 9 | 0.026 5 | 0.005 2 | 0.006 0 | 0.000 0 | 0.31 | 0.78 | 0.88 | 0.39 | 0.72 | 0.82 | 0.38 |
| f_Nocardioid aceae | 0.001 1 | 0.002 8 | 0.000 3 | 0.000 8 | 0.000 0 | 0.31 | 0.68 | 0.94 | 0.18 | 0.59 | 0.92 | 0.13 |
| f_Sphingobac | 0.001 | 0.003 | 0.000 | 0.001 | 0.000 | 0.31 | 0.65 | 0.96 | 0.10 | 0.59 | 0.93 | 0.06 |
| teriaceae | 1 | 5 | 3 | 0 | 0 | | | | | | | |
| f_Pseudonoca laceae | 0.000 6 | 0.002 1 | 0.000 2 | 0.001 1 | 0.002 8 | 0.34 | 0.66 | 0.96 | 0.14 | 0.58 | 0.94 | 0.07 |
| f_Caulobacter aceae | 0.004 2 | 0.007 2 | 0.001 5 | 0.003 0 | 0.000 0 | 0.36 | 0.72 | 0.90 | 0.24 | 0.59 | 0.92 | 0.18 |
| f_[Weeksella ceae] | 0.003 6 | 0.006 3 | 0.001 3 | 0.006 1 | 0.000 0 | 0.36 | 0.70 | 0.96 | 0.14 | 0.66 | 0.95 | 0.07 |
| f_Dietziaceae | 0.000 7 | 0.002 2 | 0.000 3 | 0.000 8 | 0.000 2 | 0.38 | 0.66 | 0.96 | 0.14 | 0.56 | 0.93 | 0.07 |
| f_Aerococcac eae | 0.004 4 | 0.006 9 | 0.001 7 | 0.004 2 | 0.000 0 | 0.38 | 0.71 | 0.94 | 0.18 | 0.64 | 0.95 | 0.13 |
| f_Porphyrom onadaceae | 0.007 2 | 0.016 3 | 0.014 5 | 0.009 0 | 0.000 0 | 2.02 | 0.75 | 0.96 | 0.20 | 0.73 | 0.98 | 0.18 |
| f_Veillonella ceae | 0.018 2 | 0.023 0 | 0.037 5 | 0.028 8 | 0.000 0 | 2.06 | 0.75 | 0.92 | 0.25 | 0.75 | 0.90 | 0.22 |
| f_Enterobacte riaceae | 0.100 6 | 0.078 3 | 0.209 1 | 0.087 8 | 0.000 0 | 2.08 | 0.84 | 0.91 | 0.54 | 0.85 | 0.93 | 0.50 |
| f_[Bamesiella ceae] | 0.001 0 | 0.003 0 | 0.002 4 | 0.005 0 | 0.000 1 | 2.49 | 0.68 | 0.96 | 0.13 | 0.59 | 0.94 | 0.10 |
| f_Rikenellace ae | 0.002 7 | 0.005 3 | 0.007 0 | 0.008 7 | 0.000 0 | 2.60 | 0.73 | 0.93 | 0.28 | 0.68 | 0.91 | 0.19 |
| f_Pseudonoca eae | 0.037 9 | 0.037 7 | 0.114 0 | 0.046 9 | 0.000 0 | 3.01 | 0.91 | 0.92 | 0.70 | 0.88 | 0.88 | 0.64 |
| f_Bifidobacte | 0.019 | 0.022 | 0.062 | 0.033 | 0.000 | 3.19 | 0.87 | 0.93 | 0.61 | 0.91 | 0.97 | 0.54 |
| riaceae | 6 | 0 | 7 | 5 | 0 | | | | | | | |

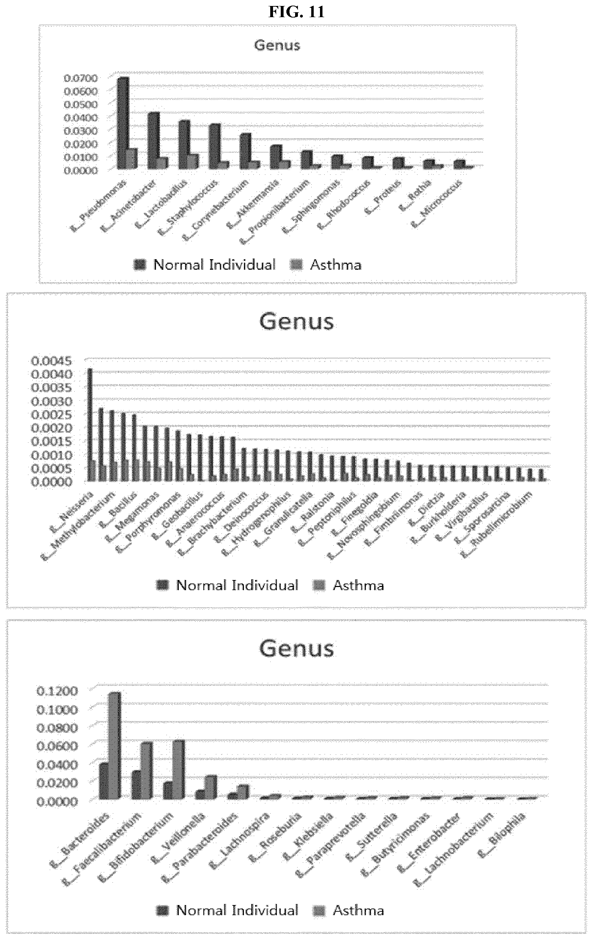

As a result of analyzing bacteria-derived extracellular vesicles in blood at a genus level, a diagnostic model developed using, as a biomarker, one or more bacteria from the genus *Geobacillus,* the genus *Rubrobacter,* the genus *Exiguobacterium,* the genus *Ralstonia,* the genus *Sporosarcina,* the genus *Hydrogenophilus,* the genus *Rhodococcus,* the genus *Proteus,* the genus *Leptotrichia,* the genus *Brevibacterium,* the genus *Brachybacterium,* the genus *Staphylococcus,* the genus *Peptoniphilus,* the genus *Lautropia,* the genus *Finegoldia,* the genus *Anaerococcus,* the genus *Sphingobacterium,* the genus *Propionibacterium,* the genus *Micrococcus,* the genus *Fimbriimonas,* the genus *Dermacoccus,* the genus *Campylobacter,* the genus *Agrobacterium,* the genus *Neisseria,* the genus *Acinetobacter,* the genus *Thermus,* the genus *Corynebacterium,* the genus *Fusobacterium,* the genus *Pseudomonas,* the genus *Jeotgalicoccus,* the genus *Dietzia,* the genus *Rubellimicrobium,* the genus *Flavobacterium,* the genus *Megamonas,* the genus *Porphyromonas,* the genus *Granulicatella,* the genus *Novosphingobium,* the genus *Sphingomonas,* the genus *Mycobacterium,* the genus *Methylobacterium,* the genus *Gordonia,* the genus *Burkholderia,* the genus *Kocuria,* the genus *Lactobacillus,* the genus *Deinococcus,* the genus *Kaistobacter,* the genus *Akkermansia,* the genus *Actinomyces,* the genus *Brevundimonas,* the genus *Virgibacillus,* the genus *Bacillus,* the genus *Eubacterium,* the genus *Rothia,* the genus *Chryseobacterium,* the genus *Faecalibacterium,* the genus *Roseburia,* the genus *Klebsiella,* the genus *Sutterella,* the genus *Paraprevotella,* the genus *Parabacteroides,* the genus *Butyricimonas,* the genus *Lachnobacterium,* the genus *Veillonella,* the genus *Bacteroides,* the genus *Lachnospira,* the genus *Bifidobacterium,* the genus *Bilophila,* and the genus *Enterobacter* exhibited significant diagnostic performance for asthma (see Table 11 and FIG. 11).

**[Table 11]**

| | normal individual | | asthma | | t-test | | Training Set | | | Test Set | | |
|---|---|---|---|---|---|---|---|---|---|---|---|---|
| Taxon | Mean | SD | Mean | SD | p-value | Ratio | AUC | sensit ivity | speci ficity | AUC | sensit ivity | speci ficity |
| g_ Geobacill us | 0.001 7 | 0.006 1 | 0.000 0 | 0.000 0 | 0.000 0 | 0.00 | 0.70 | 0.92 | 0.23 | 0.65 | 0.92 | 0.17 |
| g_Rubrobac ter | 0.000 6 | 0.002 5 | 0.000 0 | 0.000 0 | 0.000 0 | 0.00 | 0.67 | 0.95 | 0.16 | 0.62 | 0.93 | 0.10 |
| g_Exiguoba cterium | 0.000 7 | 0.003 4 | 0.000 0 | 0.000 2 | 0.000 1 | 0.04 | 0.67 | 0.97 | 0.12 | 0.59 | 0.95 | 0.04 |
| g_Ralstonia | 0.000 9 | 0.003 1 | 0.000 0 | 0.000 1 | 0.000 0 | 0.04 | 0.70 | 0.94 | 0.18 | 0.61 | 0.90 | 0.13 |
| g_Sporosar cina | 0.000 5 | 0.002 1 | 0.000 0 | 0.000 2 | 0.000 0 | 0.05 | 0.68 | 0.96 | 0.14 | 0.62 | 0.94 | 0.10 |
| g_Hydroge nophilus | 1 | 0.007 0 | 0.000 1 | 0.000 7 | 0.001 7 | 0.06 | 0.67 | 0.97 | 0.12 | 0.62 | 0.95 | 0.06 |
| g_Rhodoco ccus | 0.008 2 | 0.035 5 | 0.000 8 | 0.001 9 | 0.000 0 | 0.09 | 0.73 | 0.91 | 0.27 | 0.62 | 0.88 | 0.21 |
| g_Proteus | 0.007 5 | 0.022 5 | 0.000 8 | 0.003 6 | 0.000 0 | 0.11 | 0.65 | 0.99 | 0.06 | 0.58 | 0.97 | 0.01 |
| g_Leptotric | 0.001 | 0.002 | 0.000 | 0.000 | 0.000 | 0.12 | 0.69 | 0.94 | 0.18 | 0.59 | 0.91 | 0.13 |
| hia | 0 | 9 | 1 | 5 | 0 | | | | | | | |
| g_ Brevibact erium | 0.001 7 | 0.006 6 | 0.000 2 | 0.000 6 | 0.000 0 | 0.12 | 0.71 | 0.92 | 0.21 | 0.64 | 0.92 | 0.15 |
| g_Brachyba cterium | 0.001 2 | 0.004 6 | 0.000 1 | 0.000 5 | 0.000 0 | 0.12 | 0.70 | 0.93 | 0.19 | 0.62 | 0.92 | 0.10 |
| g_Staphylo coccus | 0.032 8 | 0.058 8 | 0.004 4 | 0.006 2 | 0.000 0 | 0.13 | 0.84 | 0.82 | 0.59 | 0.75 | 0.81 | 0.39 |
| g_Peptonip hilus | 0.000 9 | 0.003 5 | 0.000 1 | 0.000 5 | 0.000 0 | 0.13 | 0.68 | 0.96 | 0.14 | 0.57 | 0.94 | 0.07 |
| g_Lautropia | 0.001 7 | 0.006 3 | 0.000 2 | 0.000 5 | 0.000 0 | 0.14 | 0.68 | 0.95 | 0.16 | 0.60 | 0.92 | 0.07 |
| g_Finegoldi a | 0.000 8 | 0.002 4 | 0.000 1 | 0.000 6 | 0.000 0 | 0.14 | 0.67 | 0.96 | 0.14 | 0.62 | 0.92 | 0.08 |
| g_Anaeroco ccus | 0.001 6 | 0.005 4 | 0.000 3 | 0.001 3 | 0.000 0 | 0.15 | 0.69 | 0.95 | 0.17 | 0.62 | 0.94 | 0.14 |
| g_Sphingob acterium | 0.000 5 | 0.002 8 | 0.000 1 | 0.000 6 | 0.000 9 | 0.16 | 0.65 | 0.96 | 0.11 | 0.58 | 0.93 | 0.08 |
| g_Propionib acterium | 0.012 7 | 0.015 3 | 0.002 1 | 0.003 1 | 0.000 0 | 0.17 | 0.83 | 0.84 | 0.56 | 0.77 | 0.83 | 0.43 |
| g_Micrococ cus | 0.005 7 | 0.010 0 | 0.001 0 | 0.001 7 | 0.000 0 | 0.17 | 0.74 | 0.87 | 0.30 | 0.70 | 0.88 | 0.28 |
| g_Fimbriim onas | 0.000 6 | 0.002 3 | 0.000 1 | 0.000 4 | 0.000 0 | 0.17 | 0.66 | 0.96 | 0.13 | 0.55 | 0.95 | 0.07 |
| g_Dermaco ccus | 0.001 2 | 0.003 6 | 0.000 2 | 0.000 5 | 0.000 0 | 0.18 | 0.68 | 0.94 | 0.18 | 0.58 | 0.90 | 0.10 |
| g_Campylo bacter | 0.000 4 | 0.001 5 | 0.000 1 | 0.000 4 | 0.000 0 | 0.18 | 0.66 | 0.96 | 0.13 | 0.60 | 0.92 | 0.08 |
| g_Agrobact erium | 0.001 1 | 0.003 3 | 0.000 2 | 0.000 7 | 0.000 0 | 0.18 | 0.67 | 0.96 | 0.12 | 0.58 | 0.92 | 0.04 |
| g_Neisseria | 0.004 1 | 0.010 7 | 0.000 8 | 0.002 6 | 0.000 0 | 0.18 | 0.72 | 0.90 | 0.23 | 0.62 | 0.90 | 0.17 |
| g_Acinetob acter | 0.041 5 | 0.056 9 | 0.007 6 | 0.012 8 | 0.000 0 | 0.18 | 0.84 | 0.84 | 0.64 | 0.79 | 0.83 | 0.49 |
| g_Thermus | 0.000 5 | 0.002 1 | 0.000 1 | 0.000 7 | 0.000 1 | 0.19 | 0.67 | 0.96 | 0.12 | 0.58 | 0.92 | 0.07 |
| g_Coryneba cterium | 0.025 7 | 0.045 6 | 0.004 8 | 0.005 5 | 0.000 0 | 0.19 | 0.83 | 0.85 | 0.55 | 0.73 | 0.79 | 0.42 |
| g_Fusobact erium | 0.002 7 | 0.006 2 | 0.000 6 | 0.002 6 | 0.000 0 | 0.21 | 0.72 | 0.92 | 0.23 | 0.66 | 0.90 | 0.15 |
| g_Pseudom onas | 0.067 7 | 0.069 6 | 0.014 4 | 0.014 5 | 0.000 0 | 0.21 | 0.84 | 0.82 | 0.65 | 0.75 | 0.76 | 0.56 |
| g_Jeotgalic occus | 0.001 1 | 0.004 2 | 0.000 2 | 0.001 3 | 0.000 0 | 0.21 | 0.67 | 0.96 | 0.13 | 0.59 | 0.92 | 0.10 |
| g_Dietzia | 0.000 6 | 0.001 8 | 0.000 1 | 0.000 6 | 0.000 0 | 0.22 | 0.68 | 0.95 | 0.16 | 0.60 | 0.93 | 0.08 |
| g_Rubellim icrobium | 0.000 4 | 0.001 6 | 0.000 1 | 0.000 7 | 0.000 2 | 0.23 | 0.65 | 0.96 | 0.12 | 0.59 | 0.92 | 0.08 |
| g_Flavobact erium | 0.000 6 | 0.002 1 | 0.000 1 | 0.000 5 | 0.000 0 | 0.23 | 0.65 | 0.96 | 0.14 | 0.57 | 0.92 | 0.08 |
| g_Megamo nas | 0.002 0 | 0.009 4 | 0.000 5 | 0.001 1 | 0.000 7 | 0.24 | 0.65 | 0.96 | 0.12 | 0.56 | 0.93 | 0.07 |
| g_Porphyro monas | 0.001 9 | 0.007 2 | 0.000 5 | 0.003 6 | 0.000 9 | 0.25 | 0.69 | 0.94 | 0.18 | 0.62 | 0.93 | 0.14 |
| g_Granulica tella | 0.001 1 | 0.002 9 | 0.000 3 | 0.001 1 | 0.000 0 | 0.26 | 0.67 | 0.95 | 0.14 | 0.62 | 0.95 | 0.08 |
| g_Novosphi ngobium | 0.000 7 | 0.002 7 | 0.000 2 | 0.000 9 | 0.000 1 | 0.26 | 0.65 | 0.97 | 0.12 | 0.59 | 0.97 | 0.06 |
| g_Sphingo monas | 0.009 4 | 0.011 7 | 0.002 5 | 0.004 0 | 0.000 0 | 0.27 | 0.77 | 0.87 | 0.38 | 0.67 | 0.86 | 0.28 |
| g_Mycobac terium | 0.000 8 | 0.002 8 | 0.000 2 | 0.000 9 | 0.000 1 | 0.27 | 0.67 | 0.97 | 0.14 | 0.57 | 0.92 | 0.08 |
| g_Methylob acterium | 0.002 6 | 0.007 8 | 0.000 7 | 0.002 0 | 0.000 0 | 0.27 | 0.66 | 0.96 | 0.11 | 0.62 | 0.95 | 0.06 |
| g_Gordonia | 0.000 5 | 0.002 3 | 0.000 1 | 0.000 5 | 0.001 7 | 0.27 | 0.66 | 0.96 | 0.14 | 0.55 | 0.92 | 0.08 |
| g_Burkhold eria | 0.000 6 | 0.001 9 | 0.000 2 | 0.000 8 | 0.000 2 | 0.27 | 0.66 | 0.96 | 0.14 | 0.58 | 0.92 | 0.10 |
| g_Kocuria | 0.001 6 | 0.004 7 | 0.000 4 | 0.001 4 | 0.000 0 | 0.27 | 0.68 | 0.94 | 0.13 | 0.60 | 0.93 | 0.06 |
| g__Lactobaci llus | 0.035 5 | 0.056 0 | 0.010 1 | 0.014 0 | 0.000 0 | 0.29 | 0.81 | 0.86 | 0.52 | 0.73 | 0.85 | 0.38 |
| g _ Deinococ cus | 0.001 2 | 0.003 3 | 0.000 3 | 0.001 3 | 0.000 0 | 0.30 | 0.67 | 0.96 | 0.14 | 0.59 | 0.92 | 0.08 |
| g_Kaistoba cter | 0.000 8 | 0.002 7 | 0.000 2 | 0.001 0 | 0.000 1 | 0.30 | 0.67 | 0.96 | 0.14 | 0.55 | 0.94 | 0.04 |
| g_Akkerma nsia | 0.016 9 | 0.026 5 | 0.005 2 | 0.006 0 | 0.000 0 | 0.31 | 0.78 | 0.88 | 0.39 | 0.72 | 0.82 | 0.38 |
| g_Actinom yces | 0.002 5 | 0.004 6 | 0.000 8 | 0.003 8 | 0.000 0 | 0.31 | 0.75 | 0.89 | 0.32 | 0.69 | 0.89 | 0.26 |
| g_Brevundi monas | 0.000 9 | 0.003 6 | 0.000 3 | 0.000 8 | 0.000 5 | 0.32 | 0.67 | 0.97 | 0.13 | 0.56 | 0.92 | 0.07 |
| g_Virgibaci llus | 0.000 5 | 0.001 9 | 0.000 2 | 0.000 6 | 0.000 2 | 0.32 | 0.65 | 0.96 | 0.12 | 0.58 | 0.92 | 0.06 |
| g_Bacillus | 0.002 5 | 0.004 4 | 0.000 8 | 0.001 6 | 0.000 0 | 0.32 | 0.69 | 0.95 | 0.20 | 0.61 | 0.92 | 0.11 |
| g_[Eubacter ium] | 0.002 0 | 0.004 3 | 0.000 7 | 0.001 3 | 0.000 0 | 0.35 | 0.68 | 0.94 | 0.19 | 0.59 | 0.91 | 0.11 |
| g_Rothia | 0.005 9 | 0.013 7 | 0.002 1 | 0.013 9 | 0.001 0 | 0.36 | 0.74 | 0.88 | 0.30 | 0.66 | 0.88 | 0.26 |
| g_Chryseob acterium | 0.002 0 | 0.004 5 | 0.000 7 | 0.002 5 | 0.000 0 | 0.36 | 0.67 | 0.97 | 0.12 | 0.61 | 0.95 | 0.06 |
| g_Faecaliba cterium | 0.029 2 | 0.029 0 | 0.060 2 | 0.023 6 | 0.000 0 | 2.06 | 0.85 | 0.90 | 0.52 | 0.82 | 0.91 | 0.35 |
| g_Roseburi a | 0.000 9 | 0.002 0 | 0.002 1 | 0.002 9 | 0.000 0 | 2.24 | 0.72 | 0.94 | 0.20 | 0.64 | 0.92 | 0.14 |
| g_Klebsiell a | 0.000 7 | 0.001 7 | 0.001 8 | 0.001 2 | 0.000 0 | 2.48 | 0.84 | 0.94 | 0.32 | 0.76 | 0.90 | 0.28 |
| g_Sutterella | 0.000 5 | 0.001 7 | 0.001 4 | 0.002 9 | 0.000 1 | 2.53 | 0.66 | 0.96 | 0.12 | 0.59 | 0.97 | 0.15 |
| g_Paraprev otella | 0.000 6 | 0.002 1 | 0.001 4 | 0.002 2 | 0.000 0 | 2.54 | 0.68 | 0.98 | 0.11 | 0.61 | 0.96 | 0.11 |
| g_Parabacte roides | 0.005 2 | 0.014 9 | 0.013 8 | 0.008 6 | 0.000 0 | 2.66 | 0.83 | 0.93 | 0.37 | 0.83 | 0.95 | 0.32 |
| g_Butyrici monas | 0.000 4 | 0.001 4 | 0.001 2 | 0.001 9 | 0.000 0 | 2.78 | 0.72 | 0.95 | 0.18 | 0.61 | 0.92 | 0.14 |
| g_Lachnoba cterium | 0.000 1 | 0.000 5 | 0.000 4 | 0.000 7 | 0.000 0 | 2.86 | 0.68 | 0.95 | 0.12 | 0.62 | 0.95 | 0.17 |
| g_Veillonel la | 0.008 4 | 0.015 2 | 0.024 3 | 0.024 8 | 0.000 0 | 2.89 | 0.78 | 0.93 | 0.30 | 0.79 | 0.92 | 0.29 |
| g_Bacteroid es | 0.037 8 | 0.037 7 | 0.114 0 | 0.046 9 | 0.000 0 | 3.02 | 0.91 | 0.92 | 0.70 | 0.88 | 0.88 | 0.64 |
| g_Lachnosp ira | 0.001 2 | 0.002 9 | 0.003 7 | 0.013 0 | 0.004 1 | 3.23 | 0.73 | 0.95 | 0.18 | 0.69 | 0.95 | 0.19 |
| g_Bifidobac terium | 0.017 4 | 0.020 9 | 0.062 3 | 0.033 6 | 0.000 0 | 3.59 | 0.89 | 0.93 | 0.61 | 0.92 | 0.97 | 0.58 |
| g_Bilophila | 0.000 1 | 0.000 4 | 0.000 5 | 0.000 8 | 0.000 0 | 4.25 | 0.74 | 0.94 | 0.29 | 0.69 | 0.95 | 0.21 |
| g_Enteroba cter | 0.000 2 | 0.000 7 | 0.001 6 | 0.001 3 | 0.000 0 | 6.56 | 0.87 | 0.94 | 0.53 | 0.88 | 0.95 | 0.54 |

### Example 6. Model for Differential Diagnosis of COPD and Asthma Based on Metagenomic Analysis of Bacteria-Derived Extracellular Vesicles Isolated from COPD Patient-Derived Blood and Asthma Patient-Derived Blood

Extracellular vesicles were isolated from blood samples of 205 COPD patients and 219 asthma patients, and then metagenomic sequencing was performed thereon using the method of Example 3. For the development of a diagnostic model, first, a strain exhibiting a p value of less than 0.05 between two groups in a t-test and a difference of two-fold or more between two groups was selected, and then an area under curve (AUC), sensitivity, and specificity, which are diagnostic performance indexes, were calculated by logistic regression analysis.

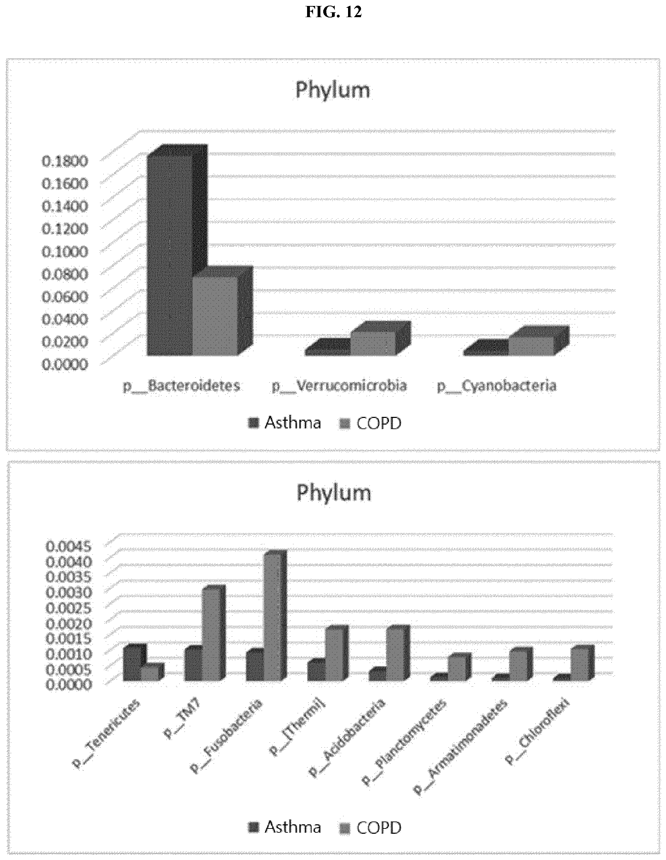

As a result of analyzing bacteria-derived extracellular vesicles in blood at a phylum level, a diagnostic model developed using, as a biomarker, one or more bacteria from the phylum *Bacteroidetes,* the phylum *Tenericutes,* the phylum *Thermi,* the phylum TM7, the phylum *Cyanobacteria,* the phylum *Verrucomicrobia,* the phylum *Fusobacteria,* the phylum *Acidobacteria,* the phylum *Planctomycetes,* the phylum *Armatimonadetes,* and the phylum *Chloroflexi* exhibited significant differential diagnostic performance for asthma and COPD (see Table 12 and FIG. 12).

**[Table 12]**

| | asthma | | COPD | | t-test | | Training Set | | | Test Set | | |
|---|---|---|---|---|---|---|---|---|---|---|---|---|
| Taxon | Mean | SD | Mean | SD | p-value | Ratio | AUC | sensit ivity | speci ficity | Auc | sensit ivity | speci ficity |
| p_Bacteroid etes | 0.176 2 | 0.049 9 | 0.069 4 | 0.026 5 | 0.000 0 | 0.39 | 0.98 | 0.95 | 0.96 | 0.99 | 0.94 | 0.95 |
| p_Tenericut es | 0.001 1 | 0.001 8 | 0.000 4 | 0.001 2 | 0.000 0 | 0.42 | 0.85 | 0.72 | 0.90 | 0.82 | 0.75 | 0.85 |
| p_[Thermi] | 0.000 6 | 0.001 9 | 0.001 7 | 0.003 3 | 0.000 0 | 2.80 | 0.85 | 0.68 | 0.92 | 0.84 | 0.76 | 0.87 |
| p_TM7 | 0.001 0 | 0.002 9 | 0.003 0 | 0.004 4 | 0.000 0 | 2.91 | 0.86 | 0.68 | 0.91 | 0.86 | 0.75 | 0.87 |
| p_Cyanobac teria | 0.004 3 | 0.011 9 | 0.016 2 | 0.020 0 | 0.000 0 | 3.75 | 0.88 | 0.70 | 0.91 | 0.89 | 0.75 | 0.85 |
| p _V errucom icrobia | 0.005 3 | 0.006 0 | 0.020 6 | 0.014 0 | 0.000 0 | 3.92 | 0.93 | 0.87 | 0.84 | 0.94 | 0.90 | 0.78 |
| p_Fusobacte ria | 0.000 9 | 0.002 9 | 0.004 1 | 0.005 5 | 0.000 0 | 4.42 | 0.88 | 0.68 | 0.90 | 0.88 | 0.76 | 0.85 |
| p_Acidobact eria | 0.000 3 | 0.001 1 | 0.001 7 | 0.004 2 | 0.000 0 | 5.13 | 0.85 | 0.67 | 0.89 | 0.86 | 0.76 | 0.85 |
| p_Planctom ycetes | 0.000 1 | 0.000 8 | 0.000 8 | 0.003 1 | 0.004 9 | 5.81 | 0.85 | 0.68 | 0.90 | 0.82 | 0.75 | 0.85 |
| p_Armatimo nadetes | 0.000 1 | 0.000 4 | 0.001 0 | 0.002 7 | 0.000 0 | 9.14 | 0.85 | 0.68 | 0.90 | 0.86 | 0.75 | 0.83 |
| p_Chlorofle xi | 0.000 1 | 0.000 3 | 0.001 0 | 0.002 9 | 0.000 0 | 10.5 6 | 0.86 | 0.68 | 0.90 | 0.85 | 0.75 | 0.85 |

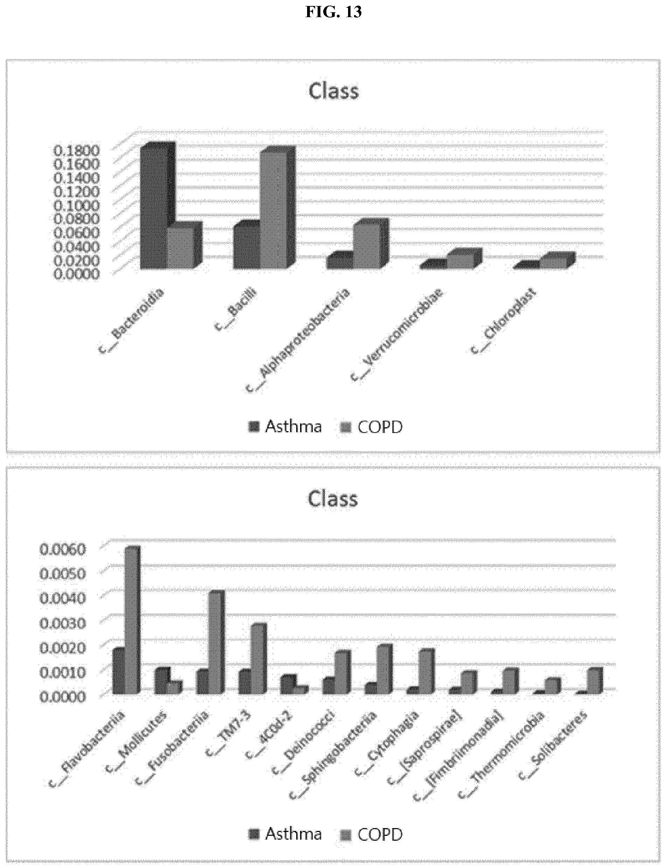

As a result of analyzing bacteria-derived extracellular vesicles in blood at a class level, a diagnostic model developed using, as a biomarker, one or more bacteria from the class *Bacteroidia,* the class 4C0d-2, the class *Mollicutes,* the class *Bacilli,* the class *Deinococci,* the class TM7-3, the class *Flavobacteriia,* the class *Alphaproteobacteria,* the class *Verrucomicrobiae,* the class *Fusobacteriia,* the class *Saprospirae,* the class *Sphingobacteriia,* the class *Chloroplast,* the class *Cytophagia,* the class *Fimbriimonadia,* the class *Thermomicrobia,* and the class *Solibacteres* exhibited significant differential diagnostic performance for asthma and COPD (see Table 13 and FIG. 13).

**[Table 13]**

| | asthma | | COPD | | t-test | | Training Set | | | Test Set | | |
|---|---|---|---|---|---|---|---|---|---|---|---|---|
| Taxon | Mean | SD | Mean | SD | p-value | Ratio | AUC | sensit ivity | speci ficity | AUC | sensit ivity | speci ficity |
| c_Bacteroidi a | 0.173 6 | 0.051 4 | 0.058 5 | 0.026 1 | 0.000 0 | 0.34 | 0.98 | 0.94 | 0.97 | 0.99 | 0.99 | 0.93 |
| c_4C0d-2 | 0.000 7 | 0.001 4 | 0.000 3 | 0.001 0 | 0.000 3 | 0.36 | 0.84 | 0.64 | 0.88 | 0.82 | 0.72 | 0.87 |
| c_Mollicutes | 0.001 0 | 0.001 6 | 0.000 4 | 0.001 2 | 0.000 1 | 0.45 | 0.84 | 0.70 | 0.89 | 0.82 | 0.75 | 0.80 |
| c_Bacilli | 0.061 1 | 0.040 8 | 0.167 8 | 0.074 5 | 0.000 0 | 2.75 | 0.96 | 0.91 | 0.91 | 0.98 | 0.97 | 0.87 |
| c_Deinococc i | 0.000 6 | 0.001 9 | 0.001 7 | 0.003 3 | 0.000 0 | 2.80 | 0.85 | 0.68 | 0.92 | 0.84 | 0.76 | 0.87 |
| c_TM7-3 | 0.000 9 | 0.002 7 | 0.002 8 | 0.004 4 | 0.000 0 | 3.02 | 0.85 | 0.68 | 0.91 | 0.85 | 0.75 | 0.87 |
| c_Flavobacte riia | 0.001 8 | 0.006 6 | 0.005 9 | 0.007 5 | 0.000 0 | 3.28 | 0.85 | 0.68 | 0.93 | 0.86 | 0.75 | 0.87 |
| c_Alphaprot | 0.016 | 0.032 | 0.063 | 0.034 | 0.000 | 3.87 | 0.95 | 0.88 | 0.88 | 0.96 | 0.93 | 0.88 |
| eobacteria | 3 | 3 | 2 | 3 | 0 | | | | | | | |
| c_Verrucomi crobiae | 0.005 2 | 0.006 0 | 0.020 5 | 0.014 0 | 0.000 0 | 3.95 | 0.93 | 0.87 | 0.83 | 0.94 | 0.90 | 0.78 |
| c_Fusobacter iia | 0.000 9 | 0.002 9 | 0.004 1 | 0.005 5 | 0.000 0 | 4.42 | 0.88 | 0.68 | 0.90 | 0.88 | 0.76 | 0.85 |
| c_[Saprospir ae] | 0.000 2 | 0.000 6 | 0.000 9 | 0.002 7 | 0.000 9 | 4.46 | 0.84 | 0.68 | 0.92 | 0.85 | 0.74 | 0.83 |
| c_Sphingoba cteriia | 0.000 4 | 0.001 1 | 0.001 9 | 0.004 2 | 0.000 0 | 4.98 | 0.86 | 0.68 | 0.90 | 0.86 | 0.74 | 0.82 |
| c_Chloroplas t | 0.002 7 | 0.004 9 | 0.015 1 | 0.019 6 | 0.000 0 | 5.54 | 0.91 | 0.79 | 0.87 | 0.91 | 0.81 | 0.80 |
| c_Cytophagi a | 0.000 2 | 0.000 8 | 0.001 7 | 0.005 0 | 0.000 0 | 8.49 | 0.85 | 0.68 | 0.88 | 0.86 | 0.75 | 0.85 |
| c_[Fimbriim onadia] | 0.000 1 | 0.000 4 | 0.001 0 | 0.002 7 | 0.000 0 | 9.14 | 0.85 | 0.68 | 0.90 | 0.86 | 0.75 | 0.83 |
| c_Thermomi crobia | 0.000 0 | 0.000 2 | 0.000 6 | 0.001 8 | 0.000 0 | 12.13 | 0.85 | 0.67 | 0.90 | 0.85 | 0.74 | 0.83 |
| c_Solibacter es | 0.000 0 | 0.000 2 | 0.001 0 | 0.002 8 | 0.000 0 | 31.46 | 0.85 | 0.67 | 0.90 | 0.84 | 0.72 | 0.85 |

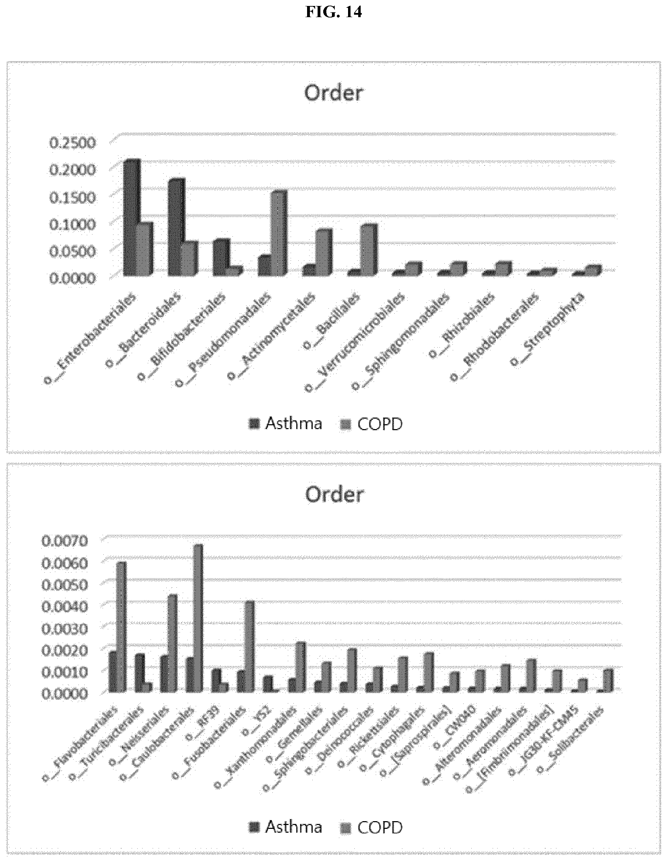

As a result of analyzing bacteria-derived extracellular vesicles in blood at an order level, a diagnostic model developed using, as a biomarker, one or more bacteria from the order YS2, the order *Bifidobacteriales,* the order *Turicibacterales,* the order *Bacteroidales,* the order RF39, the order *Enterobacteriales,* the order *Rhodobacterales,* the order *Neisseriales,* the order *Gemellales,* the order *Deinococcales,* the order *Flavobacteriales,* the order *Xanthomonadales,* the order *Verrucomicrobiales,* the order *Sphingomonadales,* the order *Caulobacterales,* the order *Fusobacteriales,* the order *Saprospirales,* the order *Pseudomonadales,* the order *Sphingobacteriales,* the order *Rhizobiales,* the order *Actinomycetales,* the order CW040, the order *Streptophyta,* the order *Rickettsiales,* the order *Alteromonadales,* the order *Cytophagales,* the order *Aeromonadales,* the order *Fimbriimonadales,* the order JG30-KF-CM45, the order *Bacillales,* and the order *Solibacterales* exhibited significant differential diagnostic performance for asthma and COPD (see Table 14 and FIG. 14).

**[Table 14]**

| | asthma | | COPD | | t-test | | Training Set | | | Test Set | | |
|---|---|---|---|---|---|---|---|---|---|---|---|---|
| Taxon | Mean | SD | Mean | SD | p-value | Ratio | AUC | sensit ivity | speci ficity | AUC | sensit ivity | speci ficity |
| o_YS2 | 0.000 7 | 0.001 4 | 0.000 0 | 0.000 2 | 0.000 0 | 0.04 | 0.88 | 0.74 | 0.93 | 0.87 | 0.78 | 0.92 |
| ο_Bifidobact eriales | 0.062 7 | 0.033 5 | 0.012 7 | 0.010 1 | 0.000 0 | 0.20 | 0.97 | 0.89 | 0.95 | 1.00 | 0.99 | 0.93 |
| o_Turicibact erales | 0.001 7 | 0.005 3 | 0.000 4 | 0.001 4 | 0.000 5 | 0.22 | 0.87 | 0.70 | 0.90 | 0.81 | 0.75 | 0.80 |
| o_Bacteroida les | 0.173 6 | 0.051 4 | 0.058 5 | 0.026 1 | 0.000 0 | 0.34 | 0.98 | 0.94 | 0.97 | 0.99 | 0.99 | 0.93 |
| o_RF39 | 0.001 0 | 0.001 6 | 0.000 4 | 0.001 1 | 0.000 0 | 0.36 | 0.85 | 0.72 | 0.88 | 0.82 | 0.75 | 0.77 |
| o_Enterobact | 0.209 | 0.087 | 0.093 | 0.039 | 0.000 | 0.45 | 0.94 | 0.83 | 0.93 | 0.97 | 0.94 | 0.85 |
| eriales | 1 | 8 | 2 | 8 | 0 | | | | | | | |
| o_Rhodobact erales | 0.003 4 | 0.023 7 | 0.009 0 | 0.009 8 | 0.001 4 | 2.66 | 0.84 | 0.66 | 0.90 | 0.84 | 0.74 | 0.85 |
| o_Neisseriale s | 0.001 6 | 0.006 4 | 0.004 4 | 0.005 2 | 0.000 0 | 2.72 | 0.87 | 0.68 | 0.90 | 0.85 | 0.74 | 0.82 |
| o_Gemellale s | 0.000 4 | 0.003 2 | 0.001 3 | 0.002 8 | 0.003 4 | 2.92 | 0.83 | 0.66 | 0.91 | 0.84 | 0.74 | 0.85 |
| o_Deinococc ales | 0.000 4 | 0.001 3 | 0.001 1 | 0.002 6 | 0.000 4 | 2.99 | 0.83 | 0.66 | 0.90 | 0.84 | 0.74 | 0.87 |
| o_Flavobacte riales | 0.001 8 | 0.006 6 | 0.005 9 | 0.007 5 | 0.000 0 | 3.28 | 0.85 | 0.68 | 0.93 | 0.86 | 0.75 | 0.87 |
| o_Xanthomo nadales | 0.000 6 | 0.001 2 | 0.002 2 | 0.003 0 | 0.000 0 | 3.90 | 0.86 | 0.68 | 0.90 | 0.87 | 0.75 | 0.82 |
| o_Verrucomi crobiales | 0.005 2 | 0.006 0 | 0.020 5 | 0.014 0 | 0.000 0 | 3.95 | 0.93 | 0.87 | 0.83 | 0.94 | 0.90 | 0.78 |
| o_Sphingom onadales | 0.004 9 | 0.008 2 | 0.021 0 | 0.020 8 | 0.000 0 | 4.25 | 0.93 | 0.85 | 0.86 | 0.93 | 0.90 | 0.80 |
| o_Caulobact erales | 0.001 5 | 0.003 0 | 0.006 7 | 0.006 8 | 0.000 0 | 4.41 | 0.91 | 0.80 | 0.88 | 0.91 | 0.79 | 0.80 |
| o_Fusobacter iales | 0.000 9 | 0.002 9 | 0.004 1 | 0.005 5 | 0.000 0 | 4.42 | 0.88 | 0.68 | 0.90 | 0.88 | 0.76 | 0.85 |
| o_[Saprospir ales] | 0.000 2 | 0.000 6 | 0.000 9 | 0.002 7 | 0.000 9 | 4.46 | 0.84 | 0.68 | 0.92 | 0.85 | 0.74 | 0.83 |
| o_Pseudomo | 0.033 | 0.049 | 0.152 | 0.071 | 0.000 | 4.53 | 0.97 | 0.93 | 0.94 | 0.98 | 0.97 | 0.90 |
| nadales | 5 | 6 | 0 | 0 | 0 | | | | | | | |
| o_Sphingoba cteriales | 0.000 4 | 0.001 1 | 0.001 9 | 0.004 2 | 0.000 0 | 4.98 | 0.86 | 0.68 | 0.90 | 0.86 | 0.74 | 0.82 |
| o_Rhizobiale s | 0.004 3 | 0.005 7 | 0.021 5 | 0.014 1 | 0.000 0 | 5.02 | 0.95 | 0.90 | 0.88 | 0.96 | 0.93 | 0.82 |
| o_Actinomyc etales | 0.016 0 | 0.021 8 | 0.081 2 | 0.040 5 | 0.000 0 | 5.07 | 0.98 | 0.95 | 0.92 | 0.99 | 0.99 | 0.88 |
| o_CW040 | 0.000 2 | 0.000 9 | 0.001 0 | 0.002 5 | 0.000 0 | 5.45 | 0.85 | 0.68 | 0.90 | 0.85 | 0.74 | 0.85 |
| o_Streptophy ta | 0.002 7 | 0.004 8 | 0.014 9 | 0.019 5 | 0.000 0 | 5.61 | 0.91 | 0.79 | 0.87 | 0.92 | 0.82 | 0.80 |
| o_Rickettsial es | 0.000 3 | 0.001 1 | 0.001 5 | 0.002 7 | 0.000 0 | 5.86 | 0.86 | 0.70 | 0.86 | 0.85 | 0.75 | 0.82 |
| o_Alteromon adales | 0.000 2 | 0.000 7 | 0.001 2 | 0.003 0 | 0.000 0 | 7.38 | 0.86 | 0.68 | 0.90 | 0.86 | 0.74 | 0.85 |
| ο_Cytophaga les | 0.000 2 | 0.000 8 | 0.001 7 | 0.005 0 | 0.000 0 | 8.49 | 0.85 | 0.68 | 0.88 | 0.86 | 0.75 | 0.85 |
| o_Aeromona dales | 0.000 2 | 0.000 7 | 0.001 4 | 0.004 2 | 0.000 0 | 8.93 | 0.86 | 0.70 | 0.90 | 0.85 | 0.74 | 0.75 |
| o_[Fimbriim onadales] | 0.000 1 | 0.000 4 | 0.001 0 | 0.002 7 | 0.000 0 | 9.14 | 0.85 | 0.68 | 0.90 | 0.86 | 0.75 | 0.83 |
| o_JG30-KF-CM45 | 0.000 0 | 0.000 2 | 0.000 5 | 0.001 7 | 0.000 1 | 11.57 | 0.85 | 0.67 | 0.90 | 0.85 | 0.74 | 0.83 |
| o_Bacillales | 0.007 | 0.008 | 0.090 | 0.074 | 0.000 | 12.76 | 0.99 | 0.99 | 0.98 | 0.99 | 0.97 | 0.97 |
| | 1 | 5 | 3 | 0 | 0 | | | | | | | |
| o_Solibacter ales | 0.000 0 | 0.000 2 | 0.001 0 | 0.002 8 | 0.000 0 | 31.46 | 0.85 | 0.67 | 0.90 | 0.84 | 0.72 | 0.85 |

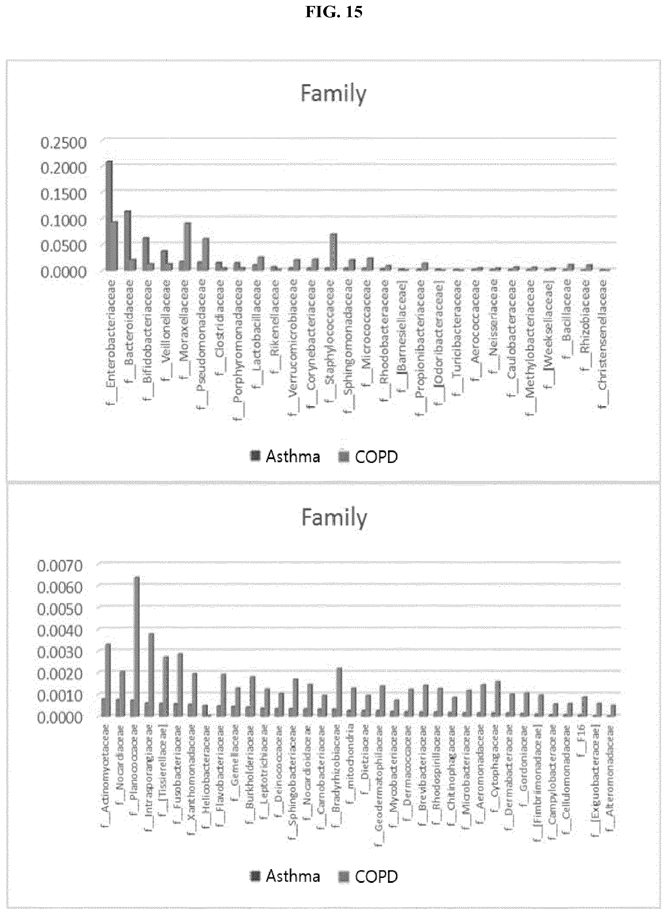

As a result of analyzing bacteria-derived extracellular vesicles in blood at a family level, a diagnostic model developed using, as a biomarker, one or more bacteria from the family *Helicobacteraceae,* the family *Bacteroidaceae,* the family *Bifidobacteriaceae,* the family *Turicibacteraceae,* the family *Rikenellaceae,* the family *Odoribacteraceae,* the family *Clostridiaceae,* the family *Barnesiellaceae,* the family *Veillonellaceae,* the family *Porphyromonadaceae,* the family *Enterobacteriaceae,* the family *Christensenellaceae,* the family *Lactobacillaceae,* the family *Rhodobacteraceae,* the family *Nocardiaceae,* the family *Neisseriaceae,* the family *Gemellaceae,* the family *Carnobacteriaceae,* the family *Aerococcaceae,* the family *Weeksellaceae,* the family *Deinococcaceae,* the family *Leptotrichiaceae,* the family *Alycobacteriaceae,* the family *Dietziaceae,* the family *Xanthomonadaceae,* the family *Pseudomonadaceae,* the family *Verrucomicrobiaceae,* the family *Methylobacteriaceae,* the family *Flavobacteriaceae,* the family *Actinomycetaceae,* the family *Burkholderiaceae,* the family *Nocardioidaceae,* the family *Caulobacteraceae,* the family *Sphingomonadaceae,* the family *Corynebacteriaceae,* the family *Tissierellaceae,* the family *Chitinophagaceae,* the family *mitochondria,* the family *Sphingobacteriaceae,* the family *Fusobacteriaceae,* the family *Moraxellaceae,* the family *Micrococcaceae,* the family *Geodermatophilaceae,* the family *Dermacoccaceae,* the family *Intrasporangiaceae,* the family *Dermabacteraceae,* the family *Propionibacteriaceae,* the family *Rhodospirillaceae,* the family *Bradyrhizobiaceae,* the family *Campylobacteraceae,* the family *Brevibacteriaceae,* the family *Microbacteriaceae,* the family *Cellulomonadaceae,* the family *Gordoniaceae,* the family *Bacillaceae,* the family *Planococcaceae,* the family *Rhizobiaceae,* the family *Aeromonadaceae,* the family *Fimbriimonadaceae,* the family *Cytophagaceae,* the family F16, the family *Staphylococcaceae,* the family *Exiguobacteraceae,* and the family *Alteromonadaceae* exhibited significant differential diagnostic performance for asthma and COPD (see Table 15 and FIG. 15).

**[Table 15]**

| | asthma | | COPD | | t-test | | Training Set | | | Test Set | | |
|---|---|---|---|---|---|---|---|---|---|---|---|---|
| Taxon | Mean | SD | Mean | SD | p-value | Rati o | AUC | sensit ivity | speci ficity | AUC | sensit ivity | speci ficity |
| f_Helicobacteraceae | 0.000 5 | 0.002 2 | 0.000 0 | 0.000 4 | 0.004 0 | 0.09 | 0.84 | 0.67 | 0.90 | 0.82 | 0.74 | 0.85 |
| f_Bacteroidaceae | 0.114 0 | 0.046 9 | 0.020 9 | 0.014 3 | 0.000 0 | 0.18 | 0.98 | 0.93 | 0.96 | 0.99 | 0.97 | 0.95 |
| f_Bifidobacteriaceae | 0.062 7 | 0.033 5 | 0.012 7 | 0.010 1 | 0.000 0 | 0.20 | 0.97 | 0.89 | 0.95 | 1.00 | 0.99 | 0.93 |
| f_Turicibacteraceae | 0.001 7 | 0.005 3 | 0.000 4 | 0.001 4 | 0.000 5 | 0.22 | 0.87 | 0.70 | 0.90 | 0.81 | 0.75 | 0.80 |
| f_Rikenellaceae | 0.007 0 | 0.008 7 | 0.001 7 | 0.002 9 | 0.000 0 | 0.24 | 0.90 | 0.75 | 0.89 | 0.88 | 0.78 | 0.90 |
| f_[Odoribacteraceae] | 0.002 1 | 0.002 9 | 0.000 7 | 0.001 9 | 0.000 0 | 0.31 | 0.86 | 0.70 | 0.92 | 0.86 | 0.75 | 0.92 |
| f_Clostridiace | 0.015 | 0.014 | 0.004 | 0.006 | 0.000 | 0.32 | 0.90 | 0.75 | 0.90 | 0.83 | 0.75 | 0.85 |
| ae | 1 | 3 | 8 | 2 | 0 | | | | | | | |
| f_[Barnesiella ceae] | 0.002 4 | 0.005 0 | 0.000 8 | 0.002 2 | 0.000 0 | 0.34 | 0.85 | 0.66 | 0.89 | 0.84 | 0.74 | 0.85 |
| f_Veillonellac eae | 0.037 5 | 0.028 8 | 0.012 8 | 0.015 4 | 0.000 0 | 0.34 | 0.92 | 0.79 | 0.88 | 0.94 | 0.87 | 0.92 |
| f_Porphyromo nadaceae | 0.014 5 | 0.009 0 | 0.005 2 | 0.004 8 | 0.000 0 | 0.36 | 0.93 | 0.82 | 0.89 | 0.92 | 0.90 | 0.83 |
| f_Enterobacte riaceae | 0.209 1 | 0.087 8 | 0.093 2 | 0.039 8 | 0.000 0 | 0.45 | 0.94 | 0.83 | 0.93 | 0.97 | 0.94 | 0.85 |
| f_Christensen ellaceae | 0.001 0 | 0.001 8 | 0.000 5 | 0.001 9 | 0.003 8 | 0.50 | 0.83 | 0.65 | 0.87 | 0.83 | 0.72 | 0.88 |
| f_Lactobacilla ceae | 0.010 8 | 0.014 1 | 0.025 6 | 0.026 4 | 0.000 0 | 2.38 | 0.89 | 0.75 | 0.90 | 0.92 | 0.79 | 0.87 |
| f_Rhodobacte raceae | 0.003 4 | 0.023 7 | 0.008 9 | 0.009 8 | 0.001 6 | 2.64 | 0.84 | 0.66 | 0.90 | 0.84 | 0.74 | 0.85 |
| f_Nocardiacea e | 0.000 8 | 0.001 9 | 0.002 0 | 0.002 8 | 0.000 0 | 2.69 | 0.87 | 0.70 | 0.90 | 0.86 | 0.75 | 0.78 |
| f_Neisseriacea e | 0.001 6 | 0.006 4 | 0.004 4 | 0.005 2 | 0.000 0 | 2.72 | 0.87 | 0.68 | 0.90 | 0.85 | 0.74 | 0.82 |
| f_Gemellacea e | 0.000 4 | 0.003 2 | 0.001 3 | 0.002 8 | 0.004 1 | 2.90 | 0.83 | 0.66 | 0.91 | 0.83 | 0.72 | 0.85 |
| f_Camobacter iaceae | 0.000 3 | 0.001 2 | 0.000 9 | 0.001 9 | 0.000 1 | 2.90 | 0.84 | 0.66 | 0.91 | 0.83 | 0.74 | 0.83 |
| f_Aerococcac | 0.001 | 0.004 | 0.004 | 0.005 | 0.000 | 2.95 | 0.88 | 0.73 | 0.87 | 0.88 | 0.76 | 0.80 |
| eae | 7 | 2 | 9 | 7 | 0 | | | | | | | |
| f_[Weeksellac eae] | 0.001 3 | 0.006 1 | 0.004 0 | 0.006 9 | 0.000 0 | 2.98 | 0.83 | 0.66 | 0.92 | 0.84 | 0.74 | 0.87 |
| f_Deinococca ceae | 0.000 3 | 0.001 3 | 0.001 0 | 0.002 6 | 0.000 6 | 3.04 | 0.83 | 0.66 | 0.90 | 0.84 | 0.74 | 0.87 |
| f_Leptotrichia ceae | 0.000 4 | 0.001 1 | 0.001 2 | 0.002 9 | 0.000 1 | 3.41 | 0.85 | 0.67 | 0.90 | 0.84 | 0.75 | 0.83 |
| f_Mycobacteri aceae | 0.000 2 | 0.000 9 | 0.000 7 | 0.001 8 | 0.000 3 | 3.45 | 0.83 | 0.66 | 0.90 | 0.84 | 0.74 | 0.87 |
| f_Dietziaceae | 0.000 3 | 0.000 8 | 0.000 9 | 0.002 1 | 0.000 0 | 3.62 | 0.84 | 0.66 | 0.92 | 0.84 | 0.74 | 0.82 |
| f_Xanthomon adaceae | 0.000 5 | 0.001 2 | 0.001 9 | 0.002 8 | 0.000 0 | 3.63 | 0.85 | 0.67 | 0.91 | 0.87 | 0.75 | 0.85 |
| f_Pseudomon adaceae | 0.016 0 | 0.015 4 | 0.061 2 | 0.035 3 | 0.000 0 | 3.82 | 0.97 | 0.93 | 0.90 | 0.97 | 0.91 | 0.88 |
| f_Verrucomic robiaceae | 0.005 2 | 0.006 0 | 0.020 5 | 0.014 0 | 0.000 0 | 3.95 | 0.93 | 0.87 | 0.83 | 0.94 | 0.90 | 0.78 |
| f_Methylobact eriaceae | 5 | 0.003 4 | 0.006 0 | 0.006 7 | 0.000 0 | 4.06 | 0.90 | 0.74 | 0.88 | 0.88 | 0.78 | 0.78 |
| f_Flavobacteri aceae | 0.000 5 | 0.002 2 | 0.001 9 | 0.003 3 | 0.000 0 | 4.10 | 0.84 | 0.66 | 0.92 | 0.85 | 0.74 | 0.90 |
| f_Actinomyce taceae | 0.000 8 | 0.003 8 | 0.003 3 | 0.004 4 | 0.000 0 | 4.18 | 0.87 | 0.68 | 0.92 | 0.88 | 0.76 | 0.85 |
| f_Burkholderi | 0.000 | 0.001 | 0.001 | 0.003 | 0.000 | 4.35 | 0.85 | 0.67 | 0.90 | 0.84 | 0.74 | 0.85 |
| aceae | 4 | 0 | 8 | 5 | 0 | | | | | | | |
| f_Nocardioidaceae | 0.000 3 | 0.000 8 | 0.001 5 | 0.003 0 | 0.000 0 | 4.36 | 0.86 | 0.67 | 0.87 | 0.84 | 0.75 | 0.77 |
| f_Caulobacteraceae | 0.001 5 | 0.003 0 | 0.006 7 | 0.006 8 | 0.000 0 | 4.41 | 0.91 | 0.80 | 0.88 | 0.91 | 0.79 | 0.80 |
| f_Sphingomonadaceae | 0.004 6 | 0.007 0 | 0.020 4 | 0.020 6 | 0.000 0 | 4.41 | 0.94 | 0.87 | 0.83 | 0.94 | 0.90 | 0.82 |
| f_Corynebacteriaceae | 0.004 8 | 0.005 5 | 0.021 6 | 0.023 4 | 0.000 0 | 4.50 | 0.92 | 0.84 | 0.86 | 0.94 | 0.91 | 0.80 |
| f_[Tissierellaceae] | 0.000 6 | 0.001 6 | 0.002 7 | 0.004 3 | 0.000 0 | 4.62 | 0.88 | 0.72 | 0.85 | 0.87 | 0.78 | 0.82 |
| f_Chitinophagaceae | 0.000 2 | 0.000 6 | 0.000 9 | 0.002 7 | 0.000 7 | 4.87 | 0.84 | 0.68 | 0.92 | 0.85 | 0.76 | 0.83 |
| f_mitochondria | 0.000 3 | 0.001 1 | 0.001 3 | 0.002 5 | 0.000 0 | 4.91 | 0.85 | 0.68 | 0.89 | 0.84 | 0.75 | 0.83 |
| f_Sphingobacteriaceae | 0.000 3 | 0.001 0 | 0.001 7 | 0.004 1 | 0.000 0 | 4.94 | 0.85 | 0.68 | 0.91 | 0.86 | 0.74 | 0.85 |
| f_Fusobacteriaceae | 0.000 6 | 0.002 6 | 0.002 9 | 0.004 2 | 0.000 0 | 5.08 | 0.87 | 0.68 | 0.90 | 0.87 | 0.76 | 0.87 |
| f_Moraxellaceae | 0.017 5 | 0.045 1 | 0.090 8 | 0.062 8 | 0.000 0 | 5.20 | 0.96 | 0.91 | 0.92 | 0.97 | 0.96 | 0.88 |
| f_Micrococcaceae | 0.004 5 | 0.014 3 | 0.023 6 | 0.016 5 | 0.000 0 | 5.22 | 0.96 | 0.91 | 0.88 | 0.97 | 0.94 | 0.87 |
| f_Geodermato | 0.000 | 0.001 | 0.001 | 0.002 | 0.000 | 5.32 | 0.86 | 0.68 | 0.89 | 0.86 | 0.75 | 0.80 |
| philaceae | 3 | 2 | 4 | 8 | 0 | | | | | | | |
| f_Dermacocca ceae | 2 | 0.000 5 | 0.001 2 | 0.002 5 | 0.000 0 | 5.92 | 0.85 | 0.68 | 0.91 | 0.86 | 0.76 | 0.87 |
| f_Intrasporang iaceae | 0.000 6 | 0.001 3 | 0.003 8 | 0.004 2 | 0.000 0 | 6.29 | 0.91 | 0.79 | 0.83 | 0.90 | 0.82 | 0.73 |
| f_Dermabacte raceae | 0.000 2 | 0.000 5 | 0.001 0 | 0.001 9 | 0.000 0 | 6.51 | 0.87 | 0.68 | 0.90 | 0.86 | 0.76 | 0.83 |
| f_Propionibac teriaceae | 0.002 1 | 0.003 1 | 0.014 0 | 0.010 9 | 0.000 0 | 6.52 | 0.97 | 0.95 | 0.90 | 0.94 | 0.87 | 0.83 |
| f_Rhodospirill, aceae | 0.000 2 | 0.001 1 | 0.001 3 | 0.005 8 | 0.009 7 | 6.56 | 0.83 | 0.66 | 0.91 | 0.84 | 0.74 | 0.85 |
| f_Bradyrhizob iaceae | 0.000 3 | 0.000 8 | 0.002 2 | 0.003 9 | 0.000 0 | 6.80 | 0.87 | 0.70 | 0.90 | 0.86 | 0.76 | 0.77 |
| f_Campylobac, teraceae | 0.000 1 | 0.000 4 | 0.000 6 | 0.002 4 | 0.005 5 | 6.84 | 0.84 | 0.66 | 0.90 | 0.83 | 0.74 | 0.83 |
| f_Brevibacteri aceae | 0.000 2 | 0.000 6 | 0.001 4 | 0.003 2 | 0.000 0 | 7.13 | 0.86 | 0.69 | 0.88 | 0.85 | 0.76 | 0.80 |
| f_Microbacter iaceae | 0.000 2 | 0.000 8 | 0.001 2 | 0.003 2 | 0.000 0 | 7.18 | 0.89 | 0.75 | 0.86 | 0.86 | 0.75 | 0.83 |
| f_Cellulomon adaceae | 0.000 1 | 0.000 4 | 0.000 6 | 0.001 8 | 0.000 2 | 7.19 | 0.84 | 0.67 | 0.91 | 0.83 | 0.74 | 0.83 |
| f_Gordoniace ae | 0.000 1 | 0.000 5 | 0.001 1 | 0.002 6 | 0.000 0 | 8.05 | 0.84 | 0.66 | 0.89 | 0.85 | 0.74 | 0.83 |
| f_Bacillaceae | 0.001 | 0.002 | 0.011 | 0.014 | 0.000 | 8.42 | 0.95 | 0.89 | 0.83 | 0.94 | 0.91 | 0.77 |
| | 3 | 0 | 2 | 6 | 0 | | | | | | | |
| f_Planococcac eae | 7 | 0.001 3 | 0.006 4 | 0.009 8 | 0.000 0 | 8.73 | 0.95 | 0.91 | 0.87 | 0.94 | 0.94 | 0.78 |
| f_Rhizobiacea e | 0.001 2 | 0.002 5 | 0.010 5 | 0.009 5 | 0.000 0 | 8.90 | 0.95 | 0.89 | 0.83 | 0.96 | 0.96 | 0.85 |
| f_Aeromonad aceae | 0.000 2 | 0.000 7 | 0.001 4 | 0.004 2 | 0.000 0 | 8.92 | 0.86 | 0.70 | 0.90 | 0.84 | 0.74 | 0.75 |
| f_[Fimbriimo nadaceae] | 0.000 1 | 0.000 4 | 0.001 0 | 0.002 7 | 0.000 0 | 9.14 | 0.85 | 0.68 | 0.90 | 0.86 | 0.75 | 0.83 |
| f_Cytophagac eae | 0.000 2 | 0.000 5 | 0.001 6 | 0.004 3 | 0.000 0 | 10.1 0 | 0.85 | 0.68 | 0.88 | 0.87 | 0.76 | 0.85 |
| f_F16 | 0.000 1 | 0.000 5 | 0.000 9 | 0.002 3 | 0.000 0 | 13.4 6 | 0.85 | 0.68 | 0.90 | 0.86 | 0.76 | 0.85 |
| f_Staphylococ caceae | 0.004 7 | 0.006 8 | 0.070 0 | 0.071 1 | 0.000 0 | 14.9 0 | 0.99 | 0.97 | 0.96 | 0.99 | 0.97 | 0.95 |
| f_[Exiguobact eraceae] | 0.000 0 | 0.000 2 | 0.000 6 | 0.003 0 | 0.008 5 | 24.4 7 | 0.85 | 0.67 | 0.91 | 0.86 | 0.76 | 0.87 |
| f_Alteromona daceae | 0.000 0 | 0.000 1 | 0.000 5 | 0.001 8 | 0.000 1 | 50.6 9 | 0.85 | 0.67 | 0.92 | 0.86 | 0.74 | 0.88 |

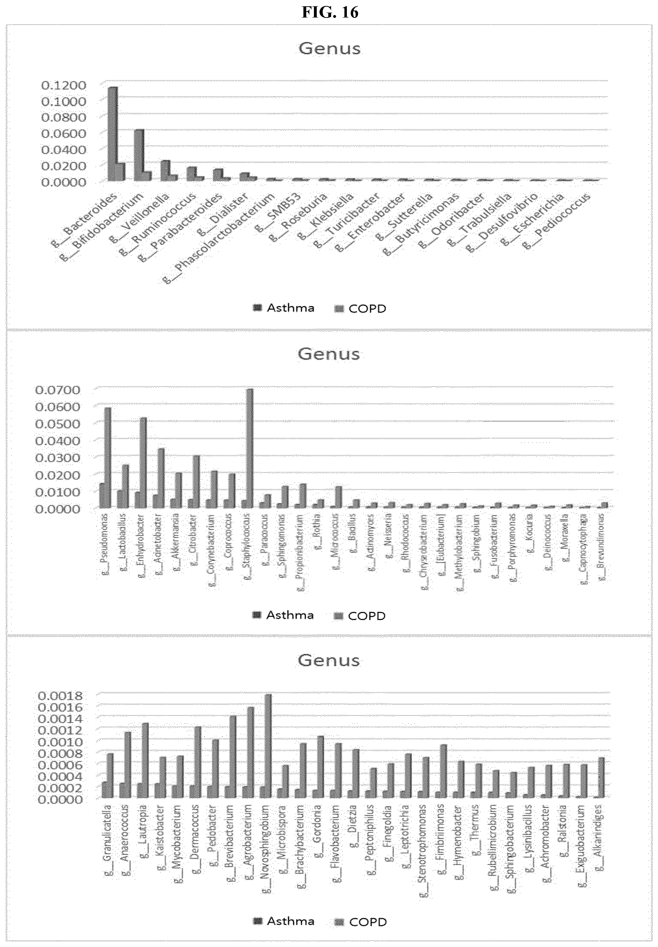

As a result of analyzing bacteria-derived extracellular vesicles in blood at a genus level, a diagnostic model developed using, as a biomarker, one or more bacteria from the genus *Enterobacter,* the genus *Trabulsiella,* the genus *Phascolarctobacterium,* the genus *Klebsiella,* the genus *Bifidobacterium,* the genus *Bacteroides,* the genus *Turicibacter,* the genus *Sutterella,* the genus *Butyricimonas,* the genus *Parabacteroides,* the genus *Ruminococcus,* the genus *Veillonella,* the genus *Pediococcus,* the genus *Desulfovibrio,* the genus SMB53, the genus *Roseburia,* the genus *Odoribacter,* the genus *Dialister,* the genus *Escherichia,* the genus *Sphingobium,* the genus *Rothia,* the genus *Paracoccus,* the genus *Lactobacillus,* the genus *Rhodococcus,* the genus *Eubacterium,* the genus *Granulicatella,* the genus *Kaistobacter,* the genus *Capnocytophaga,* the genus *Deinococcus,* the genus *Mycobacterium,* the genus *Microbispora,* the genus *Methylobacterium,* the genus *Chryseobacterium,* the genus *Actinomyces,* the genus *Porphyromonas,* the genus *Kocuria,* the genus *Akkermansia,* the genus *Pseudomonas,* the genus *Coprococcus,* the genus *Peptoniphilus,* the genus *Neisseria,* the genus *Corynebacterium,* the genus *Anaerococcus,* the genus *Acinetobacter,* the genus *Rubellimicrobium,* the genus *Sphingobacterium,* the genus *Sphingomonas,* the genus *Pedobacter,* the genus *Finegoldia,* the genus *Fusobacterium,* the genus *Lautropia,* the genus *Moraxella,* the genus *Enhydrobacter,* the genus *Dermacoccus,* the genus *Thermus,* the genus *Citrobacter,* the genus *Bacillus,* the genus *Stenotrophomonas,* the genus *Hymenobacter,* the genus *Brachybacterium,* the genus *Propionibacterium,* the genus *Leptotrichia,* the genus *Dietzia,* the genus *Brevibacterium,* the genus *Flavobacterium,* the genus *Gordonia,* the genus *Agrobacterium,* the genus *Fimbriimonas,* the genus *Novosphingobium,* the genus *Lysinibacillus,* the genus *Brevundimonas,* the genus *Achromobacter,* the genus *Micrococcus,* the genus *Staphylococcus,* the genus *Ralstonia,* the genus *Exiguobacterium,* and the genus *Alkanindiges* exhibited significant differential diagnostic performance for asthma and COPD (see Table 16 and FIG. 16).

**[Table 16]**

| | asthma | | COPD | | t-test | | Training Set | | | Test Set | | |
|---|---|---|---|---|---|---|---|---|---|---|---|---|
| Taxon | Mean | SD | Mean | SD | p-value | Ratio | AUC | sensit ivity | speci ficity | AUC | sensit ivity | speci ficity |
| g_Enterobact er | 0.001 6 | 0.001 3 | 0.000 1 | 0.000 5 | 0.000 0 | 0.08 | 0.96 | 0.91 | 0.94 | 0.95 | 0.94 | 0.85 |
| g_Trabulsiell a | 0.000 7 | 0.001 2 | 0.000 1 | 0.000 4 | 0.000 0 | 0.08 | 0.93 | 0.82 | 0.93 | 0.95 | 0.96 | 0.87 |
| g_Phascolarct obacterium | 0.002 4 | 0.010 1 | 0.000 3 | 0.001 0 | 0.002 6 | 0.12 | 0.88 | 0.72 | 0.90 | 0.89 | 0.78 | 0.90 |
| g_Klebsiella | 0.001 8 | 0.001 2 | 0.000 3 | 0.000 6 | 0.000 0 | 0.16 | 0.96 | 0.87 | 0.92 | 0.97 | 0.94 | 0.90 |
| g_Bifidobact erium | 0.062 3 | 0.033 6 | 0.010 5 | 0.008 3 | 0.000 0 | 0.17 | 0.97 | 0.89 | 0.96 | 1.00 | 0.99 | 0.95 |
| g_Bacteroide s | 0.114 0 | 0.046 9 | 0.020 9 | 0.014 3 | 0.000 0 | 0.18 | 0.98 | 0.93 | 0.96 | 0.99 | 0.97 | 0.95 |
| g_Turicibacte r | 0.0011 7 | 0.005 3 | 0.000 4 | 0.001 4 | 0.000 5 | 0.22 | 0.87 | 0.70 | 0.90 | 0.81 | 0.75 | 0.80 |
| g_Sutterella | 0.001 4 | 0.002 9 | 0.000 3 | 0.001 8 | 0.000 0 | 0.23 | 0.85 | 0.70 | 0.92 | 0.83 | 0.75 | 0.87 |
| g_ Butyricimo nas | 0.001 2 | 0.001 9 | 0.000 3 | 0.000 9 | 0.000 0 | 0.23 | 0.88 | 0.73 | 0.90 | 0.86 | 0.75 | 0.87 |
| g_Parabacter oides | 0.013 8 | 0.008 6 | 0.003 2 | 0.003 8 | 0.000 0 | 0.23 | 0.95 | 0.85 | 0.88 | 0.96 | 0.90 | 0.87 |
| g_Ruminococ | 0.016 | 0.011 | 0.004 | 0.005 | 0.000 | 0.26 | 0.94 | 0.84 | 0.88 | 0.94 | 0.87 | 0.82 |
| cus | 3 | 2 | 2 | 8 | 0 | | | | | | | |
| g_Veillonella | 0.024 3 | 0.024 8 | 0.006 4 | 0.012 7 | 0.000 0 | 0.26 | 0.91 | 0.77 | 0.90 | 0.94 | 0.84 | 0.93 |
| g_Pediococcu s | 0.000 4 | 0.001 0 | 0.000 1 | 0.000 8 | 0.000 6 | 0.28 | 0.83 | 0.64 | 0.90 | 0.83 | 0.72 | 0.88 |
| g_Desulfovib rio | 0.000 5 | 0.001 6 | 0.000 1 | 0.000 6 | 0.001 6 | 0.28 | 0.82 | 0.65 | 0.90 | 0.82 | 0.74 | 0.88 |
| g_SMB53 | 0.002 3 | 0.003 3 | 0.000 7 | 0.001 6 | 0.000 0 | 0.32 | 0.86 | 0.72 | 0.90 | 0.81 | 0.75 | 0.85 |
| g_Roseburia | 0.002 1 | 0.002 9 | 0.000 7 | 0.001 4 | 0.000 0 | 0.33 | 0.86 | 0.69 | 0.88 | 0.86 | 0.76 | 0.83 |
| g_Odoribacte r | 0.000 9 | 0.001 7 | 0.000 4 | 0.001 7 | 0.002 8 | 0.44 | 0.83 | 0.68 | 0.90 | 0.84 | 0.74 | 0.87 |
| g_Dialister | 0.009 0 | 0.007 7 | 0.004 1 | 0.006 6 | 0.000 0 | 0.46 | 0.86 | 0.69 | 0.89 | 0.87 | 0.76 | 0.85 |
| g_Escherichi a | 0.000 5 | 0.000 7 | 0.000 3 | 0.000 3 | 0.000 0 | 0.50 | 0.84 | 0.66 | 0.91 | 0.86 | 0.76 | 0.85 |
| g_Sphingobiu m | 0.000 6 | 0.002 2 | 0.001 2 | 0.002 4 | 0.003 7 | 2.15 | 0.83 | 0.66 | 0.90 | 0.84 | 0.74 | 0.87 |
| g_Rothia | 0.002 1 | 0.013 9 | 0.004 8 | 0.005 8 | 0.009 8 | 2.26 | 0.83 | 0.65 | 0.90 | 0.83 | 0.72 | 0.87 |
| g_Paracoccus | 0.003 1 | 0.023 5 | 0.007 8 | 0.009 5 | 0.007 6 | 2.47 | 0.84 | 0.66 | 0.90 | 0.83 | 0.74 | 0.85 |
| g_Lactobacill | 0.010 | 0.014 | 0.025 | 0.026 | 0.000 | 2.49 | 0.89 | 0.75 | 0.90 | 0.92 | 0.79 | 0.87 |
| us | 1 | 0 | 2 | 4 | 0 | | | | | | | |
| g_Rhodococc us | 0.000 8 | 0.001 9 | 0.002 0 | 0.002 8 | 0.000 0 | 2.71 | 0.87 | 0.70 | 0.90 | 0.86 | 0.75 | 0.78 |
| g_[Eubacteri um] | 0.000 7 | 0.001 3 | 0.002 0 | 0.003 4 | 0.000 0 | 2.75 | 0.86 | 0.70 | 0.89 | 0.85 | 0.78 | 0.82 |
| g_Granulicat ella | 0.000 3 | 0.001 1 | 0.000 8 | 0.001 9 | 0.001 0 | 2.80 | 0.84 | 0.66 | 0.90 | 0.83 | 0.74 | 0.83 |
| g_Kaistobact er | 0.000 2 | 0.001 0 | 0.000 7 | 0.001 8 | 0.001 7 | 2.86 | 0.84 | 0.66 | 0.90 | 0.84 | 0.74 | 0.87 |
| g_Capnocyto phaga | 0.000 3 | 0.002 1 | 0.000 9 | 0.002 1 | 0.006 0 | 2.91 | 0.82 | 0.65 | 0.90 | 0.82 | 0.72 | 0.87 |
| g_Deinococc us | 0.000 3 | 0.001 3 | 0.001 0 | 0.002 6 | 0.000 7 | 3.01 | 0.83 | 0.66 | 0.90 | 0.84 | 0.74 | 0.87 |
| g _ Mycobacte rium | 0.000 2 | 0.000 9 | 0.000 7 | 0.001 8 | 0.000 3 | 3.45 | 0.83 | 0.66 | 0.90 | 0.84 | 0.74 | 0.87 |
| g_Microbispo ra | 0.000 2 | 0.000 6 | 0.000 6 | 0.001 8 | 0.002 6 | 3.58 | 0.83 | 0.65 | 0.91 | 0.83 | 0.72 | 0.88 |
| g_Methyloba cterium | 0.000 7 | 0.002 0 | 0.002 6 | 0.004 4 | 0.000 0 | 3.71 | 0.86 | 0.67 | 0.88 | 0.85 | 0.74 | 0.80 |
| g_Chryseoba cterium | 0.000 7 | 0.002 5 | 0.002 7 | 0.006 3 | 0.000 0 | 3.81 | 0.85 | 0.67 | 0.91 | 0.85 | 0.74 | 0.82 |
| g _Actinomyc es | 0.000 8 | 0.003 8 | 0.003 0 | 0.004 1 | 0.000 0 | 3.85 | 0.87 | 0.68 | 0.91 | 0.87 | 0.76 | 0.85 |
| g_ Porphyrom | 0.000 | 0.003 | 0.001 | 0.003 | 0.000 | 3.86 | 0.84 | 0.65 | 0.90 | 0.84 | 0.74 | 0.88 |
| onas | 5 | 6 | 8 | 2 | 1 | | | | | | | |
| g_Kocuria | 0.000 4 | 0.001 4 | 0.001 7 | 0.003 2 | 0.000 0 | 3.88 | 0.85 | 0.67 | 0.89 | 0.87 | 0.75 | 0.83 |
| g_Akkennans^{,} ia | 0.005 2 | 0.006 0 | 0.020 5 | 0.014 0 | 0.000 0 | 3.95 | 0.93 | 0.87 | 0.83 | 0.94 | 0.90 | 0.78 |
| g_Pseudomo nas | 0.014 4 | 0.014 5 | 0.058 4 | 0.035 2 | 0.000 0 | 4.06 | 0.97 | 0.93 | 0.90 | 0.97 | 0.91 | 0.87 |
| g_Coprococc us | 0.004 7 | 0.005 0 | 0.019 9 | 0.015 9 | 0.000 0 | 4.19 | 0.91 | 0.80 | 0.81 | 0.95 | 0.84 | 0.87 |
| g_Peptoniphil us | 0.000 1 | 0.000 5 | 0.000 5 | 0.001 5 | 0.000 3 | 4.20 | 0.84 | 0.67 | 0.90 | 0.84 | 0.74 | 0.83 |
| g_Neisseria | 0.000 8 | 0.002 6 | 0.003 2 | 0.004 8 | 0.000 0 | 4.27 | 0.87 | 0.70 | 0.90 | 0.86 | 0.76 | 0.80 |
| g_Corynebact, erium | 0.004 8 | 0.005 5 | 0.021 6 | 0.023 4 | 0.000 0 | 4.50 | 0.92 | 0.84 | 0.86 | 0.94 | 0.91 | 0.80 |
| g_Anaerococ cus | 0.000 3 | 0.001 3 | 0.001 1 | 0.002 8 | 0.000 0 | 4.50 | 0.85 | 0.67 | 0.90 | 0.84 | 0.75 | 0.87 |
| g _Acinetobac ter | 0.007 6 | 0.012 8 | 0.034 7 | 0.029 2 | 0.000 0 | 4.58 | 0.95 | 0.91 | 0.92 | 0.96 | 0.91 | 0.88 |
| g_Rubellimic robium | 0.000 1 | 0.000 7 | 0.000 5 | 0.001 6 | 0.002 1 | 4.81 | 0.84 | 0.68 | 0.90 | 0.83 | 0.72 | 0.85 |
| g_Sphingoba cterium | 0.000 1 | 0.000 6 | 0.000 4 | 0.001 6 | 0.003 2 | 5.00 | 0.84 | 0.67 | 0.90 | 0.82 | 0.71 | 0.85 |
| g_Sphingomo | 0.002 | 0.004 | 0.012 | 0.017 | 0.000 | 5.01 | 0.93 | 0.84 | 0.82 | 0.93 | 0.90 | 0.83 |
| nas | 5 | 0 | 7 | 6 | 0 | | | | | | | |
| g_Pedobacter | 0.000 2 | 0.000 5 | 0.001 0 | 0.003 5 | 0.001 5 | 5.03 | 0.84 | 0.67 | 0.90 | 0.84 | 0.74 | 0.85 |
| g_Finegoldia | 0.000 1 | 0.000 6 | 0.000 6 | 0.001 8 | 0.000 4 | 5.06 | 0.84 | 0.67 | 0.90 | 0.83 | 0.74 | 0.82 |
| g_Fusobacter ium | 0.000 6 | 0.002 6 | 0.002 9 | 0.004 2 | 0.000 0 | 5.08 | 0.87 | 0.68 | 0.90 | 0.87 | 0.76 | 0.87 |
| g_Lautropia | 0.000 2 | 0.000 5 | 0.001 3 | 0.003 0 | 0.000 0 | 5.21 | 0.84 | 0.66 | 0.90 | 0.83 | 0.75 | 0.88 |
| g_Moraxella | 0.000 3 | 0.000 8 | 0.001 9 | 0.005 4 | 0.000 1 | 5.50 | 0.83 | 0.66 | 0.90 | 0.84 | 0.74 | 0.87 |
| g_Enhydroba cter | 0.009 3 | 0.042 5 | 0.052 5 | 0.047 8 | 0.000 0 | 5.66 | 0.94 | 0.81 | 0.92 | 0.95 | 0.87 | 0.92 |
| g_Dermacocc us | 0.000 2 | 0.000 5 | 0.001 2 | 0.002 5 | 0.000 0 | 5.92 | 0.85 | 0.68 | 0.91 | 0.86 | 0.76 | 0.87 |
| g_Thermus | 0.000 1 | 0.000 7 | 0.000 6 | 0.002 0 | 0.001 0 | 5.94 | 0.85 | 0.66 | 0.90 | 0.81 | 0.71 | 0.83 |
| g_Citrobacter | 0.005 0 | 0.004 1 | 0.030 5 | 0.021 4 | 0.000 0 | 6.09 | 0.95 | 0.91 | 0.85 | 0.97 | 0.93 | 0.88 |
| g_Bacillus | 0.000 8 | 0.001 6 | 0.004 8 | 0.007 0 | 0.000 0 | 6.15 | 0.90 | 0.78 | 0.83 | 0.88 | 0.79 | 0.73 |
| g_Stenotroph omonas | 0.000 1 | 0.000 5 | 0.000 7 | 0.001 6 | 0.000 0 | 6.26 | 0.85 | 0.68 | 0.91 | 0.85 | 0.76 | 0.88 |
| g_Hymenoba | 0.000 | 0.000 | 0.000 | 0.001 | 0.000 | 6.35 | 0.84 | 0.66 | 0.90 | 0.83 | 0.74 | 0.85 |
| cter | 1 | 4 | 6 | 9 | 2 | | | | | | | |
| g_Brachybact, erium | 1 | 0.000 5 | 0.000 9 | 0.001 8 | 0.000 0 | 6.46 | 0.86 | 0.68 | 0.90 | 0.86 | 0.76 | 0.83 |
| g_Propioniba cterium | 0.002 1 | 0.003 1 | 0.014 0 | 0.010 9 | 0.000 0 | 6.51 | 0.97 | 0.95 | 0.90 | 0.94 | 0.87 | 0.83 |
| g_Leptotrichi a | 0.000 1 | 0.000 5 | 0.000 8 | 0.002 2 | 0.000 1 | 6.65 | 0.85 | 0.67 | 0.90 | 0.84 | 0.75 | 0.83 |
| g_Dietzia | 0.000 1 | 0.000 6 | 0.000 8 | 0.002 0 | 0.000 0 | 6.84 | 0.84 | 0.66 | 0.92 | 0.84 | 0.76 | 0.82 |
| g_Brevibacte rium | 0.000 2 | 0.000 6 | 0.001 4 | 0.003 2 | 0.000 0 | 7.13 | 0.86 | 0.69 | 0.88 | 0.85 | 0.76 | 0.80 |
| g_Flavobacte rium | 0.000 1 | 0.000 5 | 0.000 9 | 0.002 1 | 0.000 0 | 7.17 | 0.86 | 0.68 | 0.90 | 0.85 | 0.75 | 0.83 |
| g_Gordonia | 0.000 1 | 0.000 5 | 0.001 1 | 0.002 6 | 0.000 0 | 8.05 | 0.84 | 0.66 | 0.89 | 0.85 | 0.74 | 0.83 |
| g_Agrobacter ium | 0.000 2 | 0.000 7 | 0.001 6 | 0.003 9 | 0.000 0 | 8.07 | 0.86 | 0.67 | 0.90 | 0.85 | 0.75 | 0.83 |
| g_Fimbriimo nas | 0.000 1 | 0.000 4 | 0.000 9 | 0.002 5 | 0.000 0 | 9.15 | 0.85 | 0.68 | 0.90 | 0.86 | 0.75 | 0.83 |
| g_Novosphin gobium | 0.000 2 | 0.000 9 | 0.001 8 | 0.004 4 | 0.000 0 | 9.30 | 0.86 | 0.68 | 0.88 | 0.86 | 0.76 | 0.83 |
| g_Lysinibacil lus | 0.000 1 | 0.000 3 | 0.000 5 | 0.001 7 | 0.000 1 | 9.37 | 0.84 | 0.65 | 0.90 | 0.84 | 0.74 | 0.85 |
| g_Brevundim | 0.000 | 0.000 | 0.003 | 0.005 | 0.000 | 10.67 | 0.89 | 0.75 | 0.88 | 0.88 | 0.76 | 0.80 |
| onas | 3 | 8 | 1 | 0 | 0 | | | | | | | |
| g_Achromob acter | 0.000 1 | 0.000 4 | 0.000 6 | 0.001 8 | 0.000 1 | 10.92 | 0.85 | 0.64 | 0.90 | 0.84 | 0.74 | 0.85 |
| g_Micrococc us | 0.001 0 | 0.001 7 | 0.012 5 | 0.012 1 | 0.000 0 | 12.87 | 0.96 | 0.95 | 0.86 | 0.96 | 0.94 | 0.87 |
| g_Staphyloco ccus | 0.004 4 | 0.006 2 | 0.069 4 | 0.071 1 | 0.000 0 | 15.73 | 0.99 | 0.97 | 0.96 | 0.99 | 0.99 | 0.95 |
| g_Ralstonia | 0.000 0 | 0.000 1 | 0.000 6 | 0.001 4 | 0.000 0 | 16.54 | 0.86 | 0.68 | 0.91 | 0.85 | 0.76 | 0.85 |
| g_Exiguobact erium | 0 | 0.000 2 | 0.000 6 | 0.003 0 | 0.008 3 | 24.69 | 0.85 | 0.67 | 0.91 | 0.86 | 0.76 | 0.87 |
| g_Alkamndig es | 0.000 0 | 0.000 1 | 0.000 7 | 0.003 2 | 0.002 7 | 55.07 | 0.85 | 0.68 | 0.91 | 0.85 | 0.76 | 0.85 |

The embodiments described herein should be considered in an illustrative sense only and not for the purpose of limitation.

### [Industrial Applicability]

A method of diagnosing chronic obstructive airway diseases through bacterial metagenomic analysis, can be used to predict and diagnose a risk of developing chronic obstructive airway diseases such as asthma, COPD, and the like by analyzing an increase or decrease in content of extracellular vesicles derived from specific bacteria through bacterial metagenomic analysis using a subject-derived sample. Extracellular vesicles secreted from bacteria present in the environment are absorbed into the human body, and thus may directly affect the occurrence of inflammation, and it is difficult to diagnose chronic obstructive airway diseases such as asthma, COPD, and the like early before symptoms occur, and thus efficient treatment therefor is difficult. Thus, a risk of developing chronic obstructive airway diseases such as asthma, COPD, and the like can be predicted through metagenomic analysis of bacteria or bacteria-derived extracellular vesicles by using a human body-derived sample, and thus the onset of chronic obstructive airway diseases can be delayed or prevented through appropriate management by early diagnosis and prediction of a risk group for chronic obstructive airway diseases, and, even after chronic obstructive airway diseases occur, early diagnosis therefor can be implemented, thereby lowering the incidence rate of chronic obstructive airway diseases and increasing therapeutic effects. In addition, patients diagnosed with asthma or COPD are able to avoid exposure to causative factors predicted by metagenomic analysis, whereby the progression of asthma and COPD can be ameliorated, or recurrence thereof can be prevented.

## Claims

1. A method of diagnosing chronic obstructive pulmonary disease (COPD) or asthma, the method comprising:
(a) extracting DNA from extracellular vesicles isolated from a subject sample;
(b) performing polymerase chain reaction (PCR) on the extracted DNA using a pair of primers having SEQ ID NO: 1 and SEQ ID NO: 2; and
(c) i) diagnosing COPD by comparing an increase or decrease in content of bacteria-derived extracellular vesicles between the subject sample and a normal individual-derived sample through sequencing of a product of the PCR, wherein said extracellular vesicles are derived from
bacteria belonging to the phylum *Tenericutes;*
one or more bacteria selected from the group consisting of the class *Mollicutes* and the class *Solibacteres;*
one or more bacteria selected from the group consisting of the order *Stramenopiles,* the order *Rubrobacterales,* the order *Turicibacterales,* and the order *Rhodocyclales;*
one or more bacteria selected from the group consisting of the family *Rubrobacteraceae,* the family *Turicibacteraceae,* and the family *Rhodocyclaceae;* or
one or more bacteria selected from the group consisting of the genus *Proteus,* the genus *Rubrobacter,* the genus *Turicibacter,* the genus *Faecalibacterium,* the genus *Coprococcus,* the genus *Enhydrobacter,* the genus *Citrobacter,* and the genus *Brevundimonas;* or
ii) diagnosing asthma by comparing an increase or decrease in content of bacteria-derived extracellular vesicles between the subject sample and a normal individual-derived sample through sequencing of a product of the PCR, wherein said extracellular vesicles are derived from
one or more bacteria selected from the group consisting of the phylum *Chloroflexi,* the phylum *Armatimonadetes,* the phylum *Fusobacteria,* the phylum *Cyanobacteria,* the phylum *Thermi,* and the phylum *Verrucomicrobia;*
one or more bacteria selected from the group consisting of the class *Fimbriimonadia,* the class *Cytophagia,* the class *Chloroplast,* the class *Fusobacteriia,* the class *Deinococci,* the class *Alphaproteobacteria,* the class *Flavobacteriia,* and the class *Bacilli;*
one or more bacteria selected from the group consisting of the order *Bacillales,* the order *Rhodocyclales,* the order *Cytophagales,* the order *Actinomycetales,* the order *Streptophyta,* the order *Fusobacteriales,* the order *Aeromonadales,* the order *Neisseriales,* the order *Rhizobiales,* the order *Pseudomonadales,* the order *Deinococcales,* the order *Xanthomonadales,* the order *Sphingomonadales,* the order *Verrucomicrobiales,* the order *Flavobacteriales,* and the order *Caulobacterales;*
one or more bacteria selected from the group consisting of the family *Nocardiaceae,* the family *Brevibacteriaceae,* the family *Staphylococcaceae,* the family *Planococcaceae,* the family *Tissierellaceae,* the family *Propionibacteriaceae,* the family *Dermacoccaceae,* the family *Rhizobiaceae,* the family *Bacillaceae,* the family *Corynebacteriaceae,* the family *Fusobacteriaceae,* the family *Pseudomonadaceae,* the family *Neisseriaceae,* the family *Methylobacteriaceae,* the family *Xanthomonadaceae,* the family *Micrococcaceae,* the family *Moraxellaceae,* the family *Sphingomonadaceae,* the family *Actinomycetaceae,* the family *Lactobacillaceae,* the family *Caulobacteraceae,* the family *Weeksellaceae,* the family *Aerococcaceae,* the family *Porphyromonadaceae,* the family *Veillonellaceae,* the family *Enterobacteriaceae,* the family *Barnesiellaceae,* the family *Rikenellaceae,* the family *Bacteroidaceae,* and the family *Bifidobacteriaceae;* or
one or more bacteria selected from the group consisting of the genus *Rhodococcus,* the genus *Proteus,* the genus *Staphylococcus,* the genus *Micrococcus,* the genus *Acinetobacter,* the genus *Corynebacterium,* the genus *Pseudomonas,* the genus *Sphingomonas,* the genus *Lactobacillus,* the genus *Akkermansia,* the genus *Rothia,* the genus *Faecalibacterium,* the genus *Roseburia,* the genus *Klebsiella,* the genus *Sutterella,* the genus *Paraprevotella,* the genus *Parabacteroides,* the genus *Butyricimonas,* the genus *Lachnobacterium,* the genus *Veillonella,* the genus *Bacteroides,* the genus *Lachnospira,* the genus *Bifidobacterium,* the genus *Bilophila,* and the genus *Enterobacter;* and
wherein the subject sample is blood.

2. The method of claim 1, wherein the blood is whole blood, serum, plasma, or blood mononuclear cells.

## Patentansprüche

1. Verfahren zum Diagnostizieren einer chronisch obstruktiven Lungenerkrankung (COPD) oder von Asthma, wobei das Verfahren Folgendes umfasstm
(a) Extrahieren von DNA aus extrazellulären Vesikeln, die aus einer Probandenprobe isoliert wurden;
(b) Durchführen einer Polymerase-Kettenreaktion (PCR) an der extrahierten DNA unter Verwendung eines Paars von Primern, die SEQ ID Nr.: 1 und SEQ ID Nr.: 2 aufweisen; und
(c) i) Diagnostizieren von COPD, indem ein Anstieg oder eine Abnahme des Gehalts an von Bakterien stammenden extrazellulären Vesikeln zwischen der Probandenprobe und einer von einer normalen Person stammenden Probe durch Sequenzieren eines Produkts der PCR verglichen wird, wobei die extrazellulären Vesikel stammen von
Bakterien, die zum Stamm *Tenericutes* gehören;
einem oder mehreren Bakterien, ausgewählt aus der Gruppe, die aus der Klasse *Mollicutes* und der Klasse *Solibacteres* besteht;
einem oder mehreren Bakterien, ausgewählt aus der Gruppe, die aus der Ordnung *Stramenopiles,* der Ordnung *Rubrobacterales,* der Ordnung *Turicibacterales* und der Ordnung *Rhodocyclales* besteht;
einem oder mehreren Bakterien, ausgewählt aus der Gruppe, die aus der Familie *Rubrobacteraceae,* der Familie *Turicibacteraceae* und der Familie *Rhodocyclaceae* besteht; oder
einem oder mehreren Bakterien, ausgewählt aus der Gruppe, die aus der Gattung *Proteus,* der Gattung *Rubrobacter,* der Gattung *Turicibacter,* der Gattung *Faecalibacterium,* der Gattung *Coprococcus,* der Gattung *Enhydrobacter,* der Gattung *Citrobacter* und der Gattung *Brevundimonas* besteht; oder
(ii) Diagnostizieren von Asthma, indem ein Anstieg oder eine Abnahme des Gehalts an von Bakterien stammenden extrazellulären Vesikeln zwischen der Probandenprobe und einer von einer normalen Person stammenden Probe durch Sequenzieren eines Produkts der PCR verglichen wird, wobei die extrazellulären Vesikel stammen von
einem oder mehreren Bakterien, ausgewählt aus der Gruppe, die aus der Abteilung *Chloroflexi,* der Abteilung *Armatimonadetes,* der Abteilung *Fusobacteria,* der Abteilung *Cyanobacteria,* der Abteilung *Thermi* und der Abteilung *Verrucomicrobia* besteht;
einem oder mehreren Bakterien, ausgewählt aus der Gruppe, die aus der Klasse *Fimbriimonadia,* der Klasse *Cytophagia,* der Klasse *Chloroplast,* der Klasse *Fusobacteriia,* der Klasse *Deinococci,* der Klasse *Alphaproteobacteria,* der Klasse *Flavobacteriia* und der Klasse *Bacilli* besteht;
einem oder mehreren Bakterien, ausgewählt aus der Gruppe, die aus der Ordnung *Bacillales,* der Ordnung *Rhodocyclales,* der Ordnung *Cytophagales,* der Ordnung *Actinomycetales,* der Ordnung *Streptophyta,* der Ordnung *Fusobacteriales,* der Ordnung *Aeromonadales,* der Ordnung *Neisseriales,* der Ordnung *Rhizobiales,* der Ordnung *Pseudomonadales,* der Ordnung *Deinococcales,* der Ordnung *Xanthomonadales,* der Ordnung *Sphingomonadales,* der Ordnung *Verrucomicrobiales,* der Ordnung *Flavobacteriales* und der Ordnung *Caulobacterales* besteht;
einem oder mehreren Bakterien, ausgewählt aus der Gruppe, die aus der Familie *Nocardiaceae,* der Familie *Brevibacteriaceae,* der Familie *Staphylococcaceae,* der Familie *Planococcaceae,* der Familie *Tissierellaceae,* der Familie *Propionibacteriaceae,* der Familie *Dermacoccaceae,* der Familie *Rhizobiaceae,* der Familie *Bacillaceae,* der Familie *Corynebacteriaceae,* der Familie *Fusobacteriaceae,* der Familie *Pseudomonadaceae,* der Familie *Neisseriaceae,* der Familie *Methylobacteriaceae,* der Familie *Xanthomonadaceae,* der Familie *Micrococcaceae,* der Familie *Moraxellaceae,* der Familie *Sphingomonadaceae,* der Familie *Actinomycetaceae,* der Familie *Lactobacillaceae,* der Familie *Caulobacteraceae,* der Familie *Weeksellaceae,* der Familie *Aerococcaceae,* der Familie *Porphyromonadaceae,* der Familie *Veillonellaceae,* der Familie *Enterobacteriaceae,* der Familie *Barnesiellaceae,* der Familie *Rikenellaceae,* der Familie *Bacteroidaceae* und der Familie *Bifidobacteriaceac* besteht, oder
einem oder mehreren Bakterien, ausgewählt aus der Gruppe, die aus der Gattung *Rhodococcus,* der Gattung *Proteus,* der Gattung *Staphylococcus,* der Gattung *Micrococcus,* der Gattung *Acinetohacter,* der Gattung *Corynehacterium,* der Gattung *Pseudomonas,* der Gattung *Sphingomonas,* der Gattung *Lactobacillus,* der Gattung *Akkermansia,* die Gattung *Rothia,* der Gattung *Faecalibacterium,* der Gattung *Roseburia,* der Gattung *Klebsiella,* der Gattung *Sutterella,* der Gattung *Paraprevotella,* der Gattung *Parabacteroides,* der Gattung *Butyricimonas,* der Gattung *Lachnobacterium,* der Gattung *Veillonella,* der Gattung *Bacteroides,* der Gattung *Lachnospira,* der Gattung *Bifidobacterium,* der Gattung *Bilophila* und der Gattung *Enterobacter* besteht, und
wobei es sich bei der Probandenprobe um Blut handelt.

2. Verfahren nach Anspruch 1, wobei das Blut Vollblut, Serum, Plasma oder mononukleäre Blutzellen ist.

## Revendications

1. Procédé de diagnostic de bronchopneumopathie chronique obstructive (BPCO) ou d'asthme, le procédé comprenant :
(a) l'extraction de l'ADN de vésicules extracellulaires isolées d'un échantillon de sujet ;
(b) la réalisation d'une réaction en chaîne par polymérase (PCR) sur l'ADN extrait en utilisant une paire d'amorces présentant la SEQ ID NO : 1 et la SEQ ID NO : 2 ; et
(c) i) le diagnostic d'une BPCO en comparant une augmentation ou une diminution de la teneur en vésicules extracellulaires issues de bactéries entre l'échantillon de sujet et un échantillon issu d'un individu normal par l'intermédiaire d'un séquençage d'un produit de la PCR, dans lequel lesdites vésicules extracellulaires sont issues de
bactéries appartenant au phylum Tenericutes ;
une ou plusieurs bactéries sélectionnés parmi le groupe consistant en la classe des Mollicutes et la classe des Solibacteres ;
une ou plusieurs bactéries sélectionnées parmi le groupe consistant en l'ordre Stramenopiles, l'ordre Rubrobacterales, l'ordre Turicibacterales et l'ordre Rhodocyclales ;
une ou plusieurs bactéries sélectionnées parmi le groupe consistant en la famille des Rubrobacteraceae, la famille des Turicibacteraceae et la famille des Rhodocyclaceae ; ou
une ou plusieurs bactéries sélectionnées parmi le groupe consistant en le genre Proteus, le genre Rubrobacter, le genre Turicibacter, le genre Faecalibacterium, le genre Coprococcus, le genre Enhydrobacter, le genre Citrobacter et le genre Brevundimonas ; ou
ii) le diagnostic d'un asthme en comparant une augmentation ou une diminution de la teneur en vésicules extracellulaires issues de bactéries entre l'échantillon de sujet et un échantillon issu d'un individu normal par l'intermédiaire d'un séquençage d'un produit de la PCR, dans lequel lesdites vésicules extracellulaires sont issues de
une ou plusieurs bactéries sélectionnées parmi le groupe consistant en le phylum Chloroflexi, le phylum Armatimonadetes, le phylum Fusobacteria, le phylum Cyanobacteria, le phylum Thermi et le phylum Verrucomicrobia ;
une ou plusieurs bactéries sélectionnées parmi le groupe consistant en la classe Fimbriimonadia, la classe Cytophagia, la classe des chloroplastes, la classe Fusobacteriia, la classe Deinococci, la classe Alphaproteobacteria, la classe Flavobacteriia et la classe Bacilli ;
une ou plusieurs bactéries sélectionnées parmi le groupe consistant en l'ordre Bacillales, l'ordre Rhodocyclales, l'ordre Cytophagales, l'ordre Actinomycetales, l'ordre Streptophyta, l'ordre Fusobacteriales, l'ordre Aeromonadales, l'ordre Neisseriales, l'ordre Rhizobiales, l'ordre Pseudomonadales, l'ordre Deinococcales, l'ordre Xanthomonadales, l'ordre Sphingomonadales, l'ordre Verrucomicrobiales, l'ordre Flavobacteriales et l'ordre Caulobacterales ;
une ou plusieurs bactéries sélectionnées parmi le groupe consistant en la famille Nocardiaceae, la famille Brevibacteriaceae, la famille Staphylococcaceae, la famille Planococcaceae, la famille Tissierellaceae, la famille Propionibacteriaceae, la famille Dermacoccaceae, la famille Rhizobiaceae, la famille Bacillaceae, la famille Corynebacteriaceae, la famille Fusobacteriaceae, la famille Pseudomonadaceae, la famille Neisseriaceae, la famille Methylobacteriaceae, la famille Xanthomonadaceae, la famille Micrococcaceae, la famille Moraxellaceae, la famille Sphingomonadaceae, la famille Actinomycetaceae, la famille Lactobacillaceae, la famille Caulobacteraceae, la famille Weeksellaceae, la famille Aerococcaceae, la famille Porphyromonadaceae, la famille Veillonellaceae, la famille Enterobacteriaceae, la famille Barnesiellaceae, la famille Rikenellaceae, la famille Bacteroidaceae et la famille Bifidobacteriaceae, ou
une ou plusieurs bactéries sélectionnées parmi le groupe consistant en le genre Rhodococcus, le genre Proteus, le genre Staphylococcus, le genre Micrococcus, le genre Acinetohacter, le genre Corynehacterium, le genre Pseudomonas, le genre Sphingomonas, le genre Lactobacillus, le genre Akkermansia, le genre Rothia, le genre Faecalibacterium, le genre Roseburia, le genre Klebsiella, le genre Sutterella, le genre Paraprevotella, le genre Parabacteroides, le genre Butyricimonas, le genre Lachnobacterium, le genre Veillonella, le genre Bacteroides, le genre Lachnospira, le genre Bifidobacterium, le genre Bilophila et le genre Enterobacter ; et
dans lequel l'échantillon de sujet est du sang.

2. Procédé selon la revendication 1, dans lequel le sang est du sang total, du sérum, du plasma ou des cellules mononucléaires sanguines.
